(19) **Europäisches Patentamt / European Patent Office / Office européen des brevets**

(11) **EP 4 117 756 B1**

(12) **EUROPEAN PATENT SPECIFICATION**

(45) Date of publication and mention of the grant of the patent:
**23.07.2025 Bulletin 2025/30**

(21) Application number: **21715743.7**

(22) Date of filing: **11.03.2021**

(51) International Patent Classification (IPC):
**A61M 16/00** *(2006.01)*    **A61M 16/16** *(2006.01)*

(52) Cooperative Patent Classification (CPC):
(C-Sets available)
**A61M 16/0069; A61M 16/024; A61M 16/109; A61M 16/125; A61M 16/16;** A61M 16/0051; A61M 16/0066; A61M 16/0666; A61M 16/1005; A61M 16/162; A61M 2016/0039; A61M 2016/1025; A61M 2205/123; A61M 2205/3313; A61M 2205/3368;    (Cont.)

(86) International application number:
**PCT/US2021/021989**

(87) International publication number:
**WO 2021/183817 (16.09.2021 Gazette 2021/37)**

(54) **HIGH VELOCITY RESPIRATORY THERAPY UNIT WITH NON-CONTACT SENSING AND CONTROL**

HOCHGESCHWINDIGKEITSATEMTHERAPIEEINHEIT MIT KONTAKTLOSER MESSUNG UND STEUERUNG

UNITÉ DE THÉRAPIE RESPIRATOIRE À VITESSE ÉLEVÉE AVEC DÉTECTION ET COMMANDE SANS CONTACT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2020   US 202062988583 P**
**15.06.2020   US 202016901902**
**14.12.2020   US 202063125005 P**

(43) Date of publication of application:
**18.01.2023   Bulletin 2023/03**

(73) Proprietor: **Vapotherm, Inc.**
**Exeter, NH 03833 (US)**

(72) Inventors:
• **LEONARD, Scott, A.**
**Exeter, NH 03833 (US)**
• **BODWELL, Jesse**
**Exeter, NH 03833 (US)**

• **ADAMS, David**
**Exeter, NH 03833 (US)**
• **WINSTON, David**
**Exeter, NH 03833 (US)**
• **DAVIDSON, Marc**
**Exeter, NH 03833 (US)**
• **JALBERT, Joseph**
**Exeter, NH 03833 (US)**
• **LAUDER, Nick**
**Exeter, NH 03833 (US)**

(74) Representative: **Larsen, Charles et al**
**McDermott Will Emery LLC**
**22 Bishopsgate**
**London EC2N 4BQ (GB)**

(56) References cited:
**WO-A1-2015/033288     WO-A1-2018/071812**

(52) Cooperative Patent Classification (CPC): (Cont.)
A61M 2205/502; A61M 2205/6018;
A61M 2205/6054; A61M 2205/6072;
A61M 2230/205

C-Sets
A61M 2230/205, A61M 2230/005

**Description**

**BACKGROUND**

Respiratory distress can impact patients in a wide variety of ways, from a number of causes, and with indications that can range from mild, to moderate and severe. In some situations respiratory distress arises from progression of typical asthma or temporary airway impairment, in other cases from hereditary sources, or the result of ongoing environmental exposure, or the consequence of non-pulmonary diseases. Chronic obstructive pulmonary disease (COPD) is a major contributor to respiratory distress as it can cause chronic airflow limitations through inflammation, changes in the small airways or parenchymal destruction. The elasticity of the lung can be decreased, which can inhibit the ability of the lungs to extend and distend during breathing. The actual portion of the lung responsible for gas transport (of oxygen and carbon dioxide) can also be damaged, with substantial reduction in the efficacy of breathing. As a result, a patient can suffer numerous harmful indications, which can increase in severity as the condition worsens. Hypercapnia and hypoxemia are common clinical signs of acute or chronic respiratory conditions, and shortness of breath (dyspnea) is a common symptom of these conditions. Given the rising costs of healthcare, the patients with severe conditions can have a particularly high burden on the healthcare system, particularly if there is a long-term care need.

Another important clinical cause of respiratory distress is Acute Respiratory Distress (ARDS) which is the severe impairment of lung oxygenation. The level of severity of ARDS depends on the stages of the severity of hypoxemia experienced by the patient (measured by the index of blood oxygen level over the amount of inspired oxygen required to achieve that blood oxygen measurement) and can range from *mild* (200 mm Hg $\leq$ PaO2/FIO2 $\leq$ 300 mm Hg), to *moderate* (100 mm Hg $\leq$ PaO2/FIO2 $\leq$ 200 mm Hg), and *severe* (PaO2/FIO2 $\leq$ 100 mm Hg). ARDS is often caused by the body's immune response to infection or injury in the lungs. In the case of a COVID infection, the host immune system produces an inflammatory response in an attempt to kill the virus, leading to cytokine storm and organ damage, with particular effect on the lungs.

Common causes of ARDS include sepsis (serious infection in the lungs or other organs), pneumonia, pancreatitis, side effects from blood transfusions, major trauma and burns (especially inhalation injury due to exposure to high concentration of fumes or smoke), and drug overdose. A common pathology of ARDS is leakage in the tissue barrier between the lung alveoli and the lung capillaries, causing fluid from the capillaries and interstitial fluid to leak into the lungs, preventing the affected area of the lung from performing proper gas exchange. In the case of ARDS, the diffusion barrier can break down and permit fluid to leak into the lung alveoli. The consequences of that barrier breaking down can be severe, including pulmonary edema across the broken diffusion barrier, pressurization and collapse of the affected alveoli, fibrosis, and further inflammation (the cytokine storm) as the body rushes cytokines to the affected area to try to contain the injury, exacerbating the condition even further. Patients can progress very rapidly after an incident and can die of multiple organ failures (lung, heart, renal). The body responds to these challenges by increasing the work of breathing, which eventually will result in fatigue and failure.

Patients suffering from COPD are also prone to development of ARDS, brought on by increased work of breathing due to symptoms of shortness of breath or chronic coughing, hyperventilation and inflammation of the lungs and airways. Individuals with end-stage or Stage 4 COPD, who are more likely to develop a lung infection, are at high risk of ARDS. ARDS is one of the most severe complications caused by pneumonia related to COVID-19 due to infection by the SARS-CoV-2 virus. Early symptoms can include increased breathing rate, blushing and other color changes around the mouth and airways, difficulty exhaling, nose flaring, sweating, wheezing and other indications. This constellation of presenting signs and symptoms are common among respiratory distress patients and represent the underlying requirement to relieve work of breathing.

Patients with respiratory distress or other respiratory ailments, whether mild, moderate, or severe, are often treated with respiratory assist devices that deliver supplemental breathing gas to the patient in order to support oxygenation or ventilation, or provide medication. Such devices may deliver gas to the patient using high flow therapy (HFT). HFT devices deliver breathing gas at a high flow rate (generally supraphysiologic) via an interface such as an open nasal cannula to increase the patient's fraction of inspired oxygen (FiO$_2$), decrease the patient's work of breathing, or do both. Increasing FiO$_2$ or decreasing the work of breathing helps the patient recover from respiratory ailments, such as respiratory distress or bronchospasms, or provide support to non-pulmonary etiologies of respiratory distress, such as acute decompensated heart failure. Some HFT devices heat and humidify the delivered breathing gas for medical reasons (e.g., to maintain the pliability of the tissues of surfactant-deficient patients, or to preserve mucosal integrity) or to reduce patient discomfort.

HFT systems typically operate at a high volumetric flow rate but with wide-bore cannula designs, such that the therapeutic breathing gas is delivered at a low velocity. Some HFT systems use membrane humidification to humidify the breathing gasses. The use of a membrane humidifier increases the pressure requirements of the system because membrane humidifiers resist the flow of air more than non-membrane humidifiers. Furthermore, some HFT systems use nasal cannulas with small bore nasal prongs to increase the velocity of the breathing gasses entering a patient's airway.

However, such cannulas further increase the required pressure compared to a system using large bore nasal prongs or a face mask. These increased pressure requirements of such HFT systems necessitate the use of wall air or a large compressor which may limit the HFT from being delivered at high enough velocity to address moderate or severe respiratory conditions.

Some respiratory therapy devices use a blower and a non-membrane humidifier to create humidified high flow therapy, typically at low velocities for the treatment of patients with mild conditions. Such non-membrane humidifiers are essentially heated water vessels through which gasses are channeled. These blower-based systems use larger bore cannulas to reduce pressure requirements. However, large bore cannulas do not flush $CO_2$ from a patient's airway as effectively as do small bore cannulas and are often unsuitable for use in treating patients with moderate to severe respiratory conditions. Additionally, HFT devices typically include a display which shows the current flow rate of breathing gas but does not make other system parameters available to the user. Accordingly, the user is able to adjust the flow rate of the breathing gas, but cannot control other system parameters.

These respiratory therapy devices may be configured to allow the gasses to be humidified prior to delivery to the patient to avoid drying the airway. Conventional humidifiers for breathing gas suffer from several drawbacks. The water that is used to humidify the gas must be heated. Traditionally this has required a resistive heater in a capital unit that is in contact with a conductive plate on the outside surface of an auxiliary unit that is also in contact with the water within the auxiliary unit. Either the heater in the capital unit or the heater plate on the auxiliary unit can be hot enough to injure a caregiver if the auxiliary unit is removed without sufficient time to cool. In such configurations, the heat is generated in the capital unit and conducted to the water. By definition, the heater must be hotter than the desired water temperature and the heater may not only be heating the water. Conduction into the capital unit and convection with the surrounding air are both sources of heat loss to the environment. Proper function of this type of system also typically relies on a good conduction path for the heat from the resistive heater to the heater plate and into the water. The conduction path typically works best when both plates are flat to achieve intimate contact. Any surface anomalies or surfaces that are not flat and pressed together with sufficient force may increase the temperature that the heater must reach to achieve the desired heat transfer rate. This also increases the heat loss of the system and may pose danger to users and caregivers.

Humidifier systems require a reservoir of water that can fill the device as the water is consumed. In most devices, the flow of water is controlled by a float that allows water to enter when the device has depleted the water. These are prone to failure, which can let in too much water. Additionally, when the device is idle for long periods, the floats tend to leak water into the system. This water can end up in the gas passages and then be delivered to the patient through the patient interface when the device is started up, which can be uncomfortable or dangerous. The water chamber in the device should be vented to allow air out as water enters. The vent is typically a membrane material that allows air to pass while not allowing water to pass. A failure of the float can cause the membrane material to be submerged in water which over time may deteriorate the membrane and prevent air from escaping. This may eventually prevent water from entering the system and to humidify the gas, or may cause overheating of the machine or the gas.

These devices may be designed to monitor the presence of water in the system. Running out of water can be dangerous to the patient or damage the device. Typically, a sensor that detects the position of a float provides this detection. Sensor systems often only signal when the water is depleted, at which point the operator must immediately replenish the water supply to continue therapy.

In some devices, water from the water reservoir could get into the capital unit, particularly if the device is on standby for long periods while connected to a water source. In some cases, the user must clamp off the water source manually to avoid this occurring. Failure to do so may allow water to flood the humidification chamber and push water into the air path. Requiring the user to monitor and control the water in the system leads to increased risk of damage or misuse.

Conventional systems detect the presence of an auxiliary unit in the capital unit using sensors. Position switches and optical sensors are often used. In some cases, these may be adequate, but they provide only limited information about the auxiliary unit installed. Some devices may have different versions of an auxiliary unit that require the capital unit to know what auxiliary unit is installed so that the correct settings or functions can be enabled. Conventional systems rely on user input to ensure that the correct settings are implemented, but users can make mistakes or become confused. In some devices, the auxiliary unit must be fully seated to ensure correct function, but conventional systems do not have a way to detect if the device is slightly out of position.

Most conventional humidifiers do not measure the humidity of the breathing gas delivered to the patient. Humidity measurement at near-saturated conditions is known to be very difficult and requires special sensors. Capacitive humidity sensors operate on the principal of hygroscopic materials absorbing moisture from the air, and are sometimes used in conventional systems. However, these sensors may fail if they contact liquid water, which is common in humidifier flow paths. Chilled mirror systems, used in other conventional humidifiers or laboratory tests, can be bulky and not cost effective. They are also prone to false readings in the presence of liquid water.

Use of a blower as a gas source for HFT has limitations, as suggested above, particularly the limited ability to generate pressure, so its use in delivering breathing gas is limited by the flow resistance of the system. In particular, blowers have been considered to be unsuitable for use with high velocity nasal insufflation (HVNI) systems, which utilize restrictive small bore cannulas to create a high velocity flow of gasses and therefore have high pressure requirements. Accordingly, known low velocity, blower-based HFT systems are particularly unsuitable for use in treating severe conditions. Many systems use a large diameter delivery tube with heated wire to reduce rainout, typically at least 15 or 22 mm in diameter. The flow velocity in these systems is very low, so the pressure drop is minimal and therefore more conducive to use with a blower. The low velocity flow in the delivery tube can allow condensation to accumulate in the delivery tube. This could be sent to the patient if the delivery tube is moved to allow the water to run downhill.

In WO 2018/071812 A1, systems, methods, and devices for humidifying a breathing gas are presented. The system includes a base unit, a vapor transfer unit, a nasal cannula, and a liquid container. The base unit includes a blower. The vapor transfer unit is external to the base unit and includes a gas passage, a liquid passage, a gas outlet, and a membrane separating the gas passage and the liquid passage. The membrane permits transfer of vapor into the gas passage from liquid in the liquid passage. The nasal cannula is coupled to the gas outlet. The liquid container is configured to reversibly mate with the base unit. WO 2015/033288 A1 describes a method of estimating a parameter indicative of respiratory flow of a patient being administered flow therapy, comprising: optionally administering a gas at a flow rate to the patient using a flow therapy apparatus with a patient interface, determining a terminal pressure in, at or proximate the outlet of the patient interface or in, at or proximate the nares of the patient, determining nasal RTF, determining a nasal flow parameter being or indicative of nasal flow based on the pressure and a nasal RTF, and optionally outputting the nasal flow parameter or parameter derived therefrom.

## SUMMARY

To address the foregoing and other issues, systems and methods for high velocity respiratory therapy are provided herein. According to one aspect, there is provided a system according to claim 1 and/or a method according to claim 11. The system includes a blower, a conduit, and an open nasal cannula. The blower outputs breathing gas to the conduit which transmits a flow of breathing gas to the nasal cannula at a high flow rate and high velocity. The nasal cannula has at least one nasal prong which provides the breathing gas to a nare of a patient. The system is configured such that breathing gas exits the at least one nasal prong at a velocity of at least about 40 m/s and less than about 75 m/s, and in particular at least 50 m/s, at least 55 m/s, or at least 60 m/s. The system may also provide breathing gas at a high flowrate, e.g. at least 5 L/min, at least 10 L/min, at least 20 L/min, at least 30 L/min, at least 40 L/min, at least 50 L/min, or at least 60 L/min.

High flow and high velocity respiratory therapy, using the systems and methods provided herein, offers many advantages. Firstly, heated and humidified gas can be provided to a patient allowing for secretion clearance and decreased development of bronchial hyper-response symptoms and minimizing the impact of dry air on lung tissue. Another advantage is that the high-flow respiratory therapy systems and methods can better meet elevated peak inspiratory flow demands compared to conventional systems. These high flow systems and methods can increase functional residual capacity of a patient's lungs via delivery of positive end-expiratory pressure. High flow therapy via open nasal cannula provides more comfort and is more tolerable to a patient relative to conventional systems. Oxygen dilution of the patient's upper airway can be minimized and carbon dioxide can be removed by meeting flow demands and flushing out dead space in the respiratory tract. The systems and methods herein may be configured to treat moderate to severe respiratory diseases and conditions such as COPD and ARDS. Another advantage provided herein is portability of these systems by providing the option for altogether eliminating the need for external sources of power and materials, providing an at-home or on-the-go device for continuity of care. The systems can therefore be used in providing in-home or longer term care for patients, particularly patients with moderate to severe respiratory conditions.

The systems and methods presented herein are configured with a system architecture which permits the system to be operated by a lower pressure source (*e.g,* < 270 hPa, < 200 hPa, < 150 hPa, < 100 hPa, < 50 hPa, < 30 hPa, < 20 hPa, < 10 hPa, or any other suitable gauge pressure). By using a blower or similar low pressure source, the system optionally does not require an external source of high pressure gas. Instead, the system can accept gas (ambient air or gas from another source) at ambient pressure and then pressurize the gas (e.g ., internally). This allows the system to function in environments in which pressurized gas sources are not available (e.g., at home, in an ambulance, and/or an outpatient care center). In some implementations, the blower is a centrifugal blower.

The nasal cannula includes one or two nasal prongs with distal openings positionable within the patient's nares for delivering high velocity breathing gas. A non-sealing cannula is used, and in particular the cannula may be open to the atmosphere at the nasal prong outlet. In some implementations, the gas provided at the prong outlet has a non-zero dynamic pressure.

The breathing gas is humidified within the system. The flow path of gas may have a connector between the nasal cannula the delivery tube and the prong outlet, which can be opened or closed to permit or close the flow of breathing gas existing the prong outlet.

In some implementations, the system is configured with a target setpoint for the flowrate of the breathing gas. The target setpoint may be configured and coordinated with the inner diameter of the cannula nasal prong so as to deliver high velocity breathing gas via the blower. In some implementations, the exit velocity of breathing gas from the nasal prong is a function of the inner diameter of the at least one nasal prong and the target flow setpoint. In order to achieve the desired exit high velocity, the combination of nasal prong inner diameter and target flow setpoint are selected so as to convert the output energy of the breathing gas from the blower (after heating and humidification, as disclosed herein) to high velocity breathing gas exiting the nasal prong. For example, to achieve high velocity breathing gas, the nasal cannula may be configured with a nasal prong having an inner diameter greater than or equal to about 1.4 mm and less than about 1.8 mm, and the blower set with a maximum flow setpoint greater than or equal to about 9 L/min and less than or equal to about 28 L/min. In another example, the nasal prong inner diameter may be greater than or equal to about 1.8 mm and less than about 1.9 mm, and the maximum flow setpoint is greater than or equal to about 13 L/min and less than about 31 L/min, and thereby achieve high velocity breathing gas. For another example of generating high velocity breathing gas, the nasal prong inner diameter may be greater than or equal to about 1.9 mm and less than about 3 mm, and the maximum flow setpoint is greater than or equal to about 21 L/min and less than about 60 L/min. For another example, the nasal prong inner diameter may be greater than or equal to about 3 mm and less than about 4 mm, and the maximum flow setpoint is greater than or equal to about 34 L/min and less than about 80 L/min.

The exit velocity is at least about 40 m/s and less than about 70 m/s. In some implementations, the exit velocity is at least about 40 m/s and less than about 65 m/s, at least about 40 m/s and less than about 60 m/s, at least about 40 m/s and less than about 55 m/s, at least about 40 m/s and less than about 50 m/s, at least about 40 m/s and less than about 45 m/s, or about 40 m/s. In some implementations, the exit velocity is at least 50 m/s, at least 55 m/s, at least 60 m/s, at least 65 m/s, or at least 70 m/s. In some implementations, the exit velocity is greater than a velocity measured at any point along a flow path between the blower and the nasal cannula prong. As used throughout this disclosure, any of the velocities referenced above is considered "high velocity."

In some implementations, the cannula used with the system is configured with a diameter (or ratio of diameter to length) that allows the cannula to provide a pressure drop necessary for the system to operate at specified high flow rates with high velocity. For example, if using a cannula with a nasal prong having an inner diameter greater than or equal to about 1.1 mm and less than about 1.6 mm, the pressure drop of the cannula is less than 80 hPa when operating at a flow setpoint of 8 L/min, while still providing the breathing gas at a high velocity. As another example, if using a cannula with a nasal prong having an inner diameter greater than or equal to about 1.5 mm and less than about 2 mm, the pressure drop of the cannula is less than 100 hPa when operating at a flow setpoint of 20 L/min and a high velocity. As another example, if using a cannula with a nasal prong having an inner diameter greater than or equal to about 1.9 mm and less than about 3.5 mm, the pressure drop of the cannula is less than 80 hPa when operating at a flow setpoint of 40 L/min and a high velocity.

According to one aspect, the system comprises a blower, a conduit, a nasal cannula configured for delivering HFT breathing gas at a high velocity and having at least one nasal prong, a controller, and a processor. The blower is configured to output breathing gas to the conduit which transmits breathing gas to the nasal cannula. From the nasal cannula, the nasal prong provides breathing gas to a nare of a patient. The system is configured such that breathing gas exits the nasal prong at a high velocity, for example at least about 40 m/s and less than about 75 m/s, or in particular at least 50 m/s. The processor is configured to receive first data indicative of one or more dimensions of the nasal cannula, receive second data indicative of a flowrate of the breathing gas, and calculate the exit velocity based on the first data and the second data, which may be further adjusted (e.g., by adjusting flowrate relative to cannula size) to achieve the relevant therapy objectives.

In some implementations, the system further comprises a display, and the processor is further configured to generate for display at least one of: the flowrate of breathing gas, the exit velocity, a target flow setpoint, and a pressure drop of the system. In some instances, both the exit velocity and the flowrate are displayed for the user (e.g., a physician or at-home patient). The user may view the display and decide to change one or more of the parameters. For example, the user may want to change the exit velocity. In some implementations, the system receives a user input indicating a change to at least one of the flowrate of breathing gas and the exit velocity. For example, a user input may indicate a modified flowrate, at which the user may wish to operate the system. For example, a user input may indicate a modified velocity, at which the user may wish to operate the system. In some implementations, after receiving a user input, the controller is configured to change the flowrate to account for the user input indicating a change to the flowrate or exit velocity. This step may require calculating a modified flowrate if the user input is a desired velocity, such that the modified flowrate corresponds to the desired velocity. In some implementations, the processor calculates a modified velocity based on the user input and the first data. For example, the user input may be a desired flowrate, and the processor calculates the modified velocity based on the dimensions of the nasal prong (either one or two prongs) and the desired flowrate.

In some implementations, the blower, controller, and processor are housed in a base unit. The system may comprise an auxiliary unit configured to reversibly connect to the base unit. In some implementations, the conduit is configured to reversibly connect to the auxiliary unit, such that the conduit is in fluid communication with the auxiliary unit which is in fluid communication with the blower when the auxiliary unit is connected to the base unit. The blower may provide the flow of breathing gas to the auxiliary unit while connected to the base unit, and the conduit may receive breathing gas from the auxiliary unit. The nasal cannula may be reversibly connected to the conduit to receive the breathing gas.

According to another aspect, a method for providing high velocity respiratory therapy utilizes the system of the first aspect for treatment of a patient having a respiratory condition. The method includes outputting a flow of breathing gas from a blower through a conduit and into a nasal cannula, and providing the breathing gas to a nare of the patient from the nasal prong of the nasal cannula. The blower may be a low-pressure blower configured to output breathing gas at an output pressure of less than 30 kPa (or less than 25 kPa, less than 20 kPa, less than 15 kPa, or less than 10 kPa). The nasal cannula is in fluid communication with the conduit and configured to receive the breathing gas from the conduit. The nasal cannula has a nasal prong configured with a cross sectional diameter of sized so as to provide breathing gas from its distal end at an exit velocity of at least about 40 m/s and less than about 75 m/s, or in particular at least 50 m/s. The patient may be suffering from a mild, moderate or severe respiratory distress condition. The patient may have COPD or ARDS. In some implementations the method provides therapy for a patient having COPD or ARDS by generating HVNI using a blower, in a mobile respiratory care system that is adapted to intake ambient air, pressurize it to a high pressure, and deliver it through an open nasal cannula at a high velocity.

In some implementations, the method further comprises receiving first data indicative of one or more dimensions of the nasal cannula, receiving second data indicative of a flowrate of breathing gas, and calculating the exit velocity based on the first data and the second data. In some implementations, the method further comprises generating for display at least one parameter selected from the group of: the flowrate, the exit velocity, a target flow setpoint, and a pressure drop. In some implementations, both the flowrate and the exit velocity are displayed. The method may further involve receiving a user input to increase or decrease the flowrate of the breathing gas, changing the flowrate to a modified flowrate of the breathing gas, calculating a modified velocity based on the modified flowrate and the first data, and generating for display at least one selected from the group of: the modified flowrate and the modified velocity.

In another aspect, provided is a system for providing respiratory therapy to a patient, the system comprising a base unit comprising: a blower configured to output breathing gas at a high velocity and a controller; an auxiliary unit configured to be reversibly coupled to the base unit, wherein the auxiliary unit comprises an auxiliary unit outlet; and a delivery tube configured to receive the breathing gas from the auxiliary unit outlet and transmit the breathing gas to the patient. The base unit may further comprise a measurement device comprising a first flow sensor, a second flow sensor, and a device conduit in fluid communication with the blower, wherein the first flow sensor and second flow sensor are positioned in series along the conduit. The controller may be configured to set a breathing gas flowrate of the blower based on at least one of the first measurement or the second measurement. The system may comprise a supplementary gas inlet configured to receive a supplementary gas from an external gas source and add the supplementary gas to the breathing gas, wherein the supplementary gas inlet is in fluid communication with the device conduit and disposed between the first flow sensor and the second flow sensor. At least one advantage is that the dual sensor setup allows for detection of very small changes in flowrate, such that the amount of supplementary gas added can be finely tuned.

The base unit may comprise a seat configured to receive the auxiliary unit when the auxiliary unit is coupled to the base unit. The base unit may comprise at least one alignment sensor (*e.g.,* an radio-frequency identification (RFID) reader or a Hall effect sensor), and the auxiliary unit comprises at least one alignment marker (*e.g.,* an RFID or other tag, or a magnet). The system may include an occluding valve operatively configured to simultaneously control the outputted breathing gas and liquid flow entering the auxiliary unit, for example, using three valve positions. The auxiliary unit may comprise a liquid container and a vapor transfer cartridge (VTC) configured to humidify the breathing gas. The base unit may comprise a level sensor configured to output at least one liquid level measurement indicating a liquid level in the liquid container. The auxiliary unit may include a pump configured to pump the liquid in the auxiliary unit, where the pump may be magnetically coupled to the base unit. The auxiliary unit may comprise a heating plate in a heating section, and the base unit may comprise a heat actuator configured to couple to the heating plate in a non-contact manner. One or more temperature sensors may be disposed in the base unit to monitor liquid temperature or heating plate temperature. These various sensors and components allow for non-contact coupling between the auxiliary unit and the base unit. These non-contact couplings may minimize fluid contamination of the base unit or cross-contamination between the base unit and the auxiliary unit.

In some implementations, the delivery tube includes a jacket for insulating or heating the breathing gas conveyed within the delivery tube. The jacket may also be constructed to prevent kinking of the delivery tube. In some implementations, the base unit may comprise a removable battery and a reserve battery and may be configured to run on a low-power or standby mode when the removable battery is removed. One or more external devices may be coupled to the base unit; for example, a pulse oximeter or transcutaneous carbon dioxide sensor may be coupled to provide real-time oxygen or carbon dioxide measurements from a patient. Methods for closed-loop control of patient oxygen or carbon dioxide may be implemented on the system.

In another aspect, provided herein is a method of measuring breathing gas flow in a respiratory therapy system, the method comprising generating a first measurement of the breathing gas flow using a first flow sensor, generating a second measurement of the breathing gas flow using a second flow sensor, and adjusting one or more parameters of the respiratory therapy device based on at least one of the flow measurement or the second measurement. The method may further involve mixing the breathing gas flow with supplementary gas flow to form a mixed flow after measuring the first measurement. The method may further comprise pausing the flow of the supplementary gas; and calibrating the first flow sensor and the second flow sensor to each other while the supplementary gas flow is paused, wherein calibrating reduces an error of the calculated flow difference to an error of the second flow sensor. The method may further involve receiving $SpO_2$ data from a pulse oximeter; from the $SpO_2$ data, determining $PaO_2$ data; calculating an appropriate oxygen concentration of the breathing gas; effecting adaptive feedback control of the breathing gas based on the $SpO_2$ level signals, wherein the adaptive feedback control is provided by a proportional integral derivative (PID) controller; receiving a signal indicating that the breathing gas delivered by the measurement device has been manually changed; and upon receipt of the signal, entering a manual override mode and halting adaptive feedback control.

In another aspect, provided is a method for controlling operation of a respiratory therapy unit, the method comprising receiving a first signal from an alignment sensor in a base unit of the respiratory therapy unit, the first signal being indicative of an alignment of the alignment sensor with an alignment marker of an auxiliary unit of the respiratory therapy unit; initiating operation of the respiratory therapy unit; receiving a second signal from the alignment sensor, the second signal being indicative of a misalignment of the alignment sensor with the alignment marker; and halting operation of the respiratory therapy unit.

In another aspect, provided is a method for controlling operation of a respiratory therapy unit, the method comprising receiving a temperature measurement from a temperature sensor in a heating section of the respiratory therapy unit; comparing the temperature measurement to a reference temperature; and if the temperature measurement is greater than the reference temperature, halting operation of the respiratory therapy unit.

In another aspect, provided is a method for controlling power in a respiratory therapy unit, the method comprising receiving a first signal indicating that a removable battery has been removed from the respiratory therapy unit; switching operation of the respiratory therapy unit from a regular power mode to a low power mode; and operating the respiratory therapy unit using a reserve battery in the respiratory therapy unit.

In another aspect, provided is a method for operating a respiratory therapy unit, the method comprising receiving a liquid level measurement from a level sensor, the liquid level measurement indicating a liquid level in a liquid container of the respiratory therapy unit; receiving a one flow measurement from a flow sensor, the flow measurement indicating flow rate of breathing gas in the respiratory therapy unit; and calculating the humidity of the breathing gas based on the liquid level measurement and the flow measurement. Multiple flow rate and liquid level measurements may be used in the methods.

In another aspect, provided is a measurement device for a respiratory therapy unit, the measurement device comprising a first flow sensor, a second flow sensor, and a conduit in fluid communication with the respiratory therapy unit, wherein the first flow sensor and second flow sensor are positioned in series along the conduit. The measurement device may further comprise a supplementary gas inlet configured to receive a supplementary gas from an external gas source and add the supplementary gas to the breathing gas, wherein the supplementary gas inlet is disposed between the first flow sensor and the second flow sensor.

In another aspect, provided is a respiratory therapy system comprising a base unit configured to output breathing gas; and an auxiliary unit configured to receive the outputted breathing gas, the auxiliary unit comprising a liquid container; and a vapor transfer cartridge (VTC) configured to humidify the breathing gas; wherein the liquid container comprises an outlet conduit in fluid communication with a cartridge inlet of the VTC. The base unit may comprise a level sensor configured to output a liquid level measurement indicating a liquid level in the liquid container. In some implementations, the system comprises a port configured to connect to a nebulizer from which aerosolized medicament is introduced into and becomes entrained in the breathing gas. For example, the port is located along a delivery tube of the system, on a nasal cannula or patient connector at the end of the delivery tube, or at the inlet or outlet of the VTC.

In some aspects, provided herein is a method of treatment of a respiratory disease or a viral disease. For example, the systems and methods described herein are used to treat coronavirus disease 2019 (COVID-19), caused by severe acute respiratory syndrome coronavirus 2 (SARS-CoV-2). These systems configured with a high flow nasal cannula may be used for mechanical ventilation of patients with respiratory failure (e.g., related to COVID-19). Treatment of COVID-19 may involve introducing supplementary oxygen into the breathing gas via a supplementary gas inlet. A pulse oximeter may be attached to the patient during treatment, such that $SpO_2$ is monitored and the flowrate of supplementary oxygen can be adjusted to provide enough oxygen to achieve a therapeutic level of oxygen in the patient.

In some aspects, provided herein is a mobile system for respiratory therapy. For example, a respiratory therapy system is disposed on a rolling cart, in a vehicle, or in a patient's home. The system may be configured to intake ambient air and pressurize by the blower, in addition to or in lieu of an external source of pressurized gas. The system may include one or more sensors at the air intake in order to measure temperature or humidity of the incoming air. The system may also be configured to operate on power from an internal battery, without an external power source.

In another aspect, provided herein is a system for providing high velocity respiratory therapy to the nare of a patient. As mentioned above, known low velocity, blower-based HFT systems are particularly unsuitable for use in treating sever conditions. Because of this, all components of the air path must have minimal pressure drop so that as much of the blower's pressure as possible is reserved to accelerate the flow through the cannula prongs. The system described here optimizes the pressure drop along the air path in order to conserve enough pressure for high velocity output. The system comprises a breathing gas source configured to output a flow of breathing gas at a flowrate of 8-60 L/min (or more than 60 L/min) and an output pressure; a gas passage in fluid communication with the breathing gas source and configured to convey the flow of breathing gas from the breathing gas source; and a nasal cannula in fluid communication with the gas passage and having a nasal prong with an exit orifice that has a prong cross-sectional area, the nasal cannula defining a first flow path length and being configured to receive the flow of breathing gas from the gas passage and transmit the flow of breathing gas through the exit orifice at a high velocity. The gas passage defines a second flow path length between the breathing gas source and the nasal cannula, the gas passage having a minimum passage cross-sectional area at a point along the second flow path length such that a ratio of the minimum passage cross-sectional area to the prong cross-sectional area is about 2.5 to 5. When breathing gas flows through the nasal cannula a pressure drop occurs in an amount corresponding to less than about 35% of the output pressure. The nasal prong exit orifice is configured with an internal diameter sized so that breathing gas exiting from the exit orifice has an exit velocity of at least 40 m/s, or in particular at least 50 m/s.

In some implementations, the breathing gas source is any one of a blower, a compressor, a portable gas tank, or a wall outlet. In some implementations, the system further comprises a humidifier positioned along the gas passage and configured to humidify the flow of breathing gas. The humidifier may be a vapor transfer unit comprising a plurality of permeable fibers, a liquid inlet, and a liquid outlet, wherein the liquid inlet is configured to convey a heated liquid into the vapor transfer unit such that the heated liquid flows around the plurality of permeable fibers. Alternatively, humidifier may be a hotpot humidifier comprising a heating plate and a fluid reservoir, wherein the heating plate heats a liquid in the fluid reservoir.

In some implementations, the prong cross-sectional area is substantially circular. In some implementations, the prong cross-sectional area has an oval shape. In some implementations, the prong cross-sectional area has an inner diameter of 1.2 to 3.8 mm. In some implementations, the output pressure is about 14 kPa. In some implementations, the pressure drop of the breathing gas through the nasal cannula is 1 to 4.5 kPa. In some implementations, the exit velocity is 40 to 80 m/s. In some implementations, the ratio of the minimum passage cross-sectional area to the prong cross-sectional area is about 2.5, and the flowrate is 40-60 L/min. In some implementations, the ratio of the minimum passage cross-sectional area to the prong cross-sectional area is about 3, and the flowrate is about 20 L/min. In some adaptations of HFT, the exit velocity is high velocity and the pressure drop through the nasal cannula is 1 to 4.5 kPa.

In some implementations, the system further comprises an oxygen source configured to provide a flow of oxygen, such that the flow of breathing gas comprises the flow of oxygen. In some implementations, the gas passage comprises a delivery tube having an inlet port coupled to the breathing gas source, an outlet port coupled to the nasal cannula, and a lumen configured to convey the breathing gas from the inlet port to the outlet port. In some implementations, the nasal cannula comprises a facial tubing section. In some implementations, the breathing gas source is a centrifugal blower.

In another aspect, provided herein is a method for providing high velocity respiratory therapy to the nare of a patient, for example a patient suffering from moderate to severe respiratory disease such as COPD or ARDS. The method comprises the steps of: outputting a high velocity flow of breathing gas from a breathing gas source at a flowrate of 8-60 L/min and a first output pressure; conveying the flow of breathing gas from the breathing gas source along a gas passage to a nasal cannula; and delivering the flow of breathing gas to the nare of a patient through an exit orifice at an exit velocity of at least 40 m/s, or in particular at least 50 m/s. The gas passage defines a first flow path length between the breathing gas source and the nasal cannula. The nasal cannula has a nasal prong with the exit orifice that has a prong cross-sectional area, the nasal cannula defining a second flow path length. The gas passage has a minimum passage cross-sectional area along the first flow path length such that the ratio of the minimum passage cross-sectional area to the prong cross-sectional area is 2.5 to 5. When breathing gas flows through the nasal cannula a pressure drop occurs in an amount corresponding to less than about 35% of the first output pressure.

In some implementations, the breathing gas source is any one of a blower, a compressor, a portable gas tank, or a wall outlet. In some implementations, the method further comprises: humidifying the flow of breathing gas with a humidifier positioned along the gas passage. In some implementations, the humidifier is a vapor transfer unit comprising a plurality of permeable fibers, a liquid inlet, and a liquid outlet, wherein the liquid inlet is configured to convey a heated liquid into the vapor transfer unit such that the heated liquid flows around the plurality of permeable fibers. In other implementations, the humidifier is a hotpot humidifier comprising a heating plate and a fluid reservoir, wherein the heating plate heats a liquid in the fluid reservoir.

In some implementations, the prong cross-sectional area is substantially circular. In some implementations, the prong cross-sectional area has an oval shape. In some implementations, the prong cross-sectional area has an inner diameter of 1.2 to 3.8 mm. In some implementations, the output pressure is about 14 kPa. In some implementations, the pressure drop of the breathing gas through the nasal cannula is 1 to 4.5 kPa. In some implementations, the exit velocity is 40 to 80 m/s. In some implementations, the ratio of the minimum passage cross-sectional area to the prong cross-sectional area is about 2.5, and the flowrate is 40-60 L/min. In some implementations, the ratio of the minimum passage cross-sectional area to the prong cross-sectional area is about 3, and the flowrate is about 20 L/min. In some implementations, the breathing gas is delivered at a second output pressure equal to the first output pressure minus a cumulative pressure drop comprising the pressure drop occurring in the nasal cannula.

In some implementations, the method further comprises mixing the flow of breathing gas with a flow of supplemental oxygen provided by an oxygen source configured to provide a flow of oxygen. In some implementations, the first gas passage comprises a delivery tube having an inlet port coupled to the breathing gas source, an outlet port coupled to the nasal cannula, and a lumen configured to convey the breathing gas from the inlet port to the outlet port. In some implementations, the nasal cannula comprises a facial tubing section. In some implementations, the breathing gas source is a centrifugal blower.

In another aspect, provided herein is a method for providing high velocity respiratory therapy to the nare of a patient. The method comprises the steps of: outputting a flow of breathing gas from a breathing gas source at a flowrate of 8-60 L/min and a first output pressure; conveying the flow of breathing gas from the breathing gas source along a gas passage to a nasal cannula; and delivering the flow of breathing gas to the nare of a patient through an exit orifice of a nasal prong at a high velocity, such as an exit velocity of at least 40 m/s, or in particular at least 50 m/s. When breathing gas flows through the nasal cannula a pressure drop occurs in an amount corresponding to less than about 35% of the first output pressure. In some implementations, the gas passage defines a first flow path length between the breathing gas source and the nasal cannula. In some implementations, the exit orifice of the one nasal prong of the nasal cannula has a prong cross-sectional area, the nasal cannula defining a second flow path length. In some implementations, the gas passage has a minimum passage cross-sectional area along the first flow path length such that the ratio of the minimum passage cross-sectional area to the prong cross-sectional area is 2.5 to 5. The nasal prong used with the cannula may be single prong or dual-prong.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

BRIEF DESCRIPTION OF THE DRAWINGS

The foregoing and other objects and advantages will be apparent upon consideration of the following detailed description, taken in conjunction with the accompanying drawings, in which like reference characters refer to like parts throughout, and in which:

FIG. 1 shows a block diagram of a high-velocity respiratory therapy system, according to an illustrative implementation;

FIG. 2 shows a block diagram of a control system for a respiratory therapy system, according to an illustrative implementation;

FIG. 3 shows a flowchart describing a method for calculating and displaying exit velocity of a respiratory therapy system, according to an illustrative implementation;

FIG. 4 shows a respiratory therapy system including a base unit and an auxiliary unit, according to an illustrative implementation;

FIG. 5 shows a cross-section of a respiratory therapy system including an auxiliary unit seated in a base unit, according to an illustrative implementation;

FIG. 6 shows a cross-section of an occluder valve for linking a base unit and an auxiliary unit, according to an illustrative implementation;

FIGs. 7A-7F show various views and cross-sections of an auxiliary unit, according to an illustrative implementation;

FIGs. 8A and 8B show cross-sections of an occluder valve for linking a base unit and an auxiliary unit, according to an illustrative implementation;

FIG. 9 shows a heating section of an auxiliary unit of a respiratory therapy system, according to an illustrative implementation;

FIG. 10 shows a measurement device, according to an illustrative implementation;

FIG. 11 shows a measurement device configured to receive a supplementary gas, according to an illustrative implementation;

FIG. 12 shows a measurement device configured to receive a supplementary gas and coupled to a PID controller, according to an illustrative implementation;

FIGs. 13A and 13B show a graphical representations of carbon dioxide in a patient's airway during high flow therapy, according to an illustrative implementation;

FIGs. 14A-14C show plots of a computational fluid dynamics (CFD) study performed for high velocity therapy, according to an illustrative implementation; FIG. 14A shows a plot of cannula pressure drop as a function of outlet velocity; FIG. 14B shows a plot of cannula pressure drop as a percentage of total pressure available from a 14 kPa blower as a function of exit velocity; FIG. 14C shows a plot of tubing pressure drop as a function of the ratio of tubing cross-section to prong size of the cannula;

FIG. 15 shows a plot of heater power as a function of gas flow rates for humidification, according to an illustrative implementation; and

FIG. 16 shows a flowchart describing a method for measuring humidity in breathing gas supplied by a respiratory therapy system, according to an illustrative implementation.

## DETAILED DESCRIPTION

To provide an overall understanding of the assemblies and methods described herein, certain illustrative implementations will be described. Although the implementations and features described herein are specifically described for high velocity respiratory therapy, it will be understood that all the components and other features outlined below may be combined with one another in any suitable manner and may be adapted and applied to other respiratory therapy systems and devices, including low flow oxygen therapy, continuous positive airway pressure therapy (CPAP), mechanical ventilation, oxygen masks, Venturi masks, and Tracheostomy masks. The term "about," as used herein, should be understood to mean plus or minus 20%. For example, "about 40 m/s" should be understood to mean 40 m/s $\pm$ 8 m/s.

The systems, devices, and methods described herein may be used for delivering High Flow Therapy (HFT) at a high velocity, for example for the treatment of patients that are suffering from moderate to severe respiratory conditions, such as COPD and ARDS. HFT systems/devices deliver breathing gas at a high flow rate via an interface such as a nasal cannula to increase the patient's fraction of inspired oxygen ($FiO_2$), decrease the patient's work of breathing, or do both. HFT can create a reservoir with high $FiO_2$ in the nasal cavity, the nasopharynx, or the oropharynx. Increasing $FiO_2$ or decreasing the work of breathing helps the patient recover from respiratory ailments, such as respiratory distress or bronchospasms. Some HFT devices heat and humidify the delivered breathing gas for medical reasons (e.g., to maintain the pliability of the tissues of surfactant-deficient patients, or to preserve mucosal integrity) or to reduce patient discomfort (e.g., warmed and humidified nasal oxygen can be better tolerated). HFT may be used to eliminate anatomic dead space, for example, in a patient's upper airway for the reduction in hypercapnia and hypoxemia. The high velocity gas maintains a constant flow of fresh gas into the patient's airway, thereby flushing out upper airway dead space. Patients experience assisted exhalation due to the flushing out of exhaled air by the constant flow of fresh gas, allowing a reservoir of fresh air to be readily inhaled. This flushing of the upper airway creates a reservoir that reduces room-air entrainment so as to provide a more realistic $FiO_2$ as set by the system/device, providing more effective oxygenation. Furthermore, humidification of the flow reduces negative effects of dry air on lung tissue from overcoming the ventilation effects of HFT.

As used herein, the term "processor" or "computing device" refers to one or more computers, microprocessors, logic devices, servers, or other devices configured with hardware, firmware, and software to carry out one or more of the computerized techniques described herein. Processors and processing devices may also include one or more memory devices for storing inputs, outputs, and data that is currently being processed. As used herein, "user interface" includes, without limitation, any suitable combination of one or more input devices (e.g., keypads, touch screens, trackballs, voice recognition systems, etc.) and/or one or more output devices (e.g., visual displays, speakers, tactile displays, printing devices, etc.). Examples of user devices that may implement an interface include, without limitation, personal computers, laptops, and mobile devices (such as smartphones, blackberries, PDAs, tablet computers, etc.). For example, an interface may be implemented over a web browser or a mobile application installed on the user device.

As used herein, the term "liquid" generally refers to water; however, "liquid" may also be inclusive of liquid drugs and mixtures of water and liquid drugs. As used herein, the term "breathing gas" generally refers to air; however, "breathing gas" may be used to refer to a gas having oxygen and/or carbon dioxide in proportions different from ambient air. "Breathing gas" may be used to refer to air, oxygen, carbon dioxide, helium, nitric oxide, gases containing vaporized water, gases containing an aerosol, and anesthetic gases, or a mixture of any or all of the above, if configured for inhalation by a patient. "Breathing gas" may be used to refer to gas mixtures such as mixtures of any of oxygen, carbon dioxide, nitrogen, helium, nitric oxide, gases containing vaporized water, gases containing an aerosol, and anesthetic gases. A "blower" is described as a component of many of the systems described herein; it should be understood that the term "blower" encompasses centrifugal blowers, regenerative blowers, side-channel blowers, vortex blowers, positive-displacement blowers, and any other suitable blowers. It should also be understood that in some implementations a compressor can be used in place of a blower, for example a centrifugal compressor, a side-channel compressor, a ring compressor, or a positive-displacement compressor.

### System Parameters for High Velocity Respiratory Therapy

Several combinations of suitable system parameters for providing high-velocity respiratory therapy are discussed herein. As discussed above, the system is configured with a flowrate of breathing gas controlled by a target flow setpoint, and the cannula is configured to receive the flow of breathing gas at the setpoint and exit the flow of breathing gas to the patient through at least one nasal prong. The nasal prong has an inner diameter suitable to constrict the received flow of breathing gas so that it exits the exit prong at a high velocity. In some implementations, the exit velocity of breathing gas from the nasal prong is a function of the inner diameter of the at least one nasal prong and the target flow setpoint. In order to achieve the desired exit velocity, there may be certain combinations of nasal prong inner diameter and target flow setpoint that are desirable and/or allowable. Table 1 shows exemplary combinations of nasal prong inner diameter and maximum flow setpoint that achieve high velocity respiratory therapy.

**Table 1:** Exemplary combinations of nasal prong inner diameter and maximum flow setpoint configured to provide high velocity respiratory therapy on systems of the present disclosure.

| Nasal Prong Inner Diameter d (mm) | Maximum Flow Setpoint Q (L/min) |
|---|---|
| $1.4 \leq d < 1.8$ | $9 \leq Q < 20$ |
| $1.8 \leq d < 1.9$ | $13 \leq Q < 31$ |
| $1.9 \leq d < 3$ | $21 \leq Q < 60$ |
| $3 \leq d < 4$ | $34 \leq Q < 80$ |

In some implementations, cannula sizes may be used on the high velocity respiratory therapy systems discussed herein in order to achieve a desired pressure drop or other system conditions or properties of the cannula. For example, there may be allowable cannula pressure drops for a given nasal prong inner diameter and flow setpoint. Table 2 shows exemplary ranges of cannula pressure drops for certain pressure drops and flow setpoints.

**Table 2:** Exemplary nasal cannula pressure drops that can allow high velocity respiratory therapy with the described system based on nasal prong inner diameter and flow setpoints.

| Nasal Prong Inner Diameter d (mm) | Cannula Pressure Drop (hPa) | Flow Setpoint (L/min) |
|---|---|---|
| $1.1 \leq d < 1.6$ | 80 | 8 |
| $1.5 \leq d < 2$ | 100 | 20 |
| $1.9 \leq d < 3.5$ | 80 | 40 |

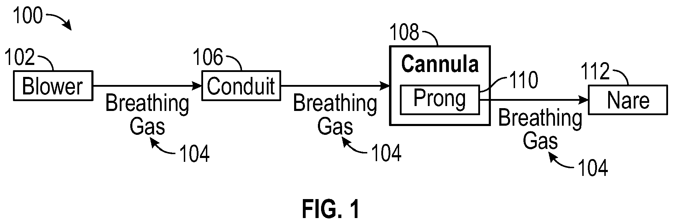

The parameters discussed above may be implemented on a respiratory therapy system, such as that shown in FIG. 1. FIG. 1 is a block diagram describing a system 100 for providing high-velocity respiratory therapy, according to an illustrative implementation. System 100 includes blower 102, conduit (also referred to as delivery tube herein) 106, and nasal cannula 108 having at least one nasal prong 110. Blower 102, delivery tube 106, cannula 108, and prong 110 are positioned sequentially in fluid communication. Accordingly, breathing gas 104 is output from the blower 102, flows through delivery tube 106 to cannula 108, and exits cannula 108 through prong 110 into nare 112 of a patient.

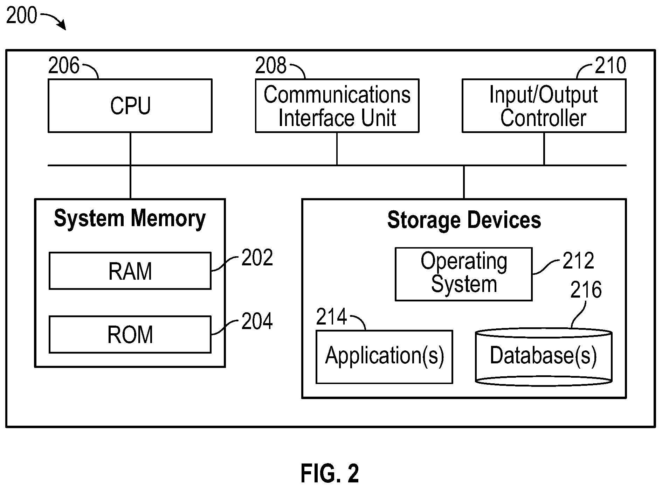

In certain implementations, blower 102 is housed within a base unit, which may additionally include a computing device, such as computing device 200 of FIG. 2. This computing device may include a processor and/or a controller, for example, CPU 206 and input/output controller 210 of FIG. 2. In some implementations, delivery tube 106 does not directly attach to blower 102; rather, system 100 further comprises an auxiliary unit configured to reversibly connect delivery tube 106 to blower 102 or the base unit. Delivery tube 106 may reversibly connect at on end to the auxiliary unit, and cannula 108 may reversibly connect to an opposite end of delivery tube 106. Delivery tube 106 may be any suitable conduit for fluid transmission of air or breathing gas.

In some implementations, information or data about cannula 108 is received by system 100. The information or data may be received when cannula 108 is affixed to delivery tube 106 or when the auxiliary unit is connected to blower 102 or the base unit. For example, system 100 receives data indicative of dimensions of prong 110 of cannula 108. The cannula data may be stored in a chip located in the cannula 108 or in the auxiliary unit. In some implementations, system 100 comprises a transmitter configured to send the data to a receiver or the controller. The transmitter may be an RFID tag or other tag within the auxiliary unit or attached to the cannula 108. The tag may include information relating to the auxiliary unit, the nasal cannula, the at least one nasal prong, or any other relevant information. The base unit may include a receiver configured to receive the information from the tag.

In some implementations, the user provides the data. For example, the user may directly enter the data, upload the data from a separate device, or select an installed cannula from a list *(e.g.,* a drop-down menu on a display), where the selected cannula is associated with certain data in the system. In some implementations, the cannula data may include additional information and may be used to identify the nasal cannula as originating from a specific manufacturer, having certain features, and/or having a use history. Knowing the cannula dimensions, such as an inner diameter of prong 110, may be useful for calculating the exit velocity of the breathing gas 104 from prong 110 into nare 112. Nasal cannula designs compatible with the present disclosure are described in U.S. Patent No. 10,300,236.

For example, exit velocity may be determined by the processor based on prong inner diameter and a measured flowrate of breathing gas 104. Breathing gas flowrate may be measured by one or more sensors in the system. The one or more sensors may be located within blower 102, at an outlet of blower 102, at an inlet or outlet of delivery tube 106, within delivery tube 106, within cannula 108, or at any other suitable location. The one or more sensors may send one or more measurements to the controller or processor. Data or measurements may be stored in a memory, such as memories 202 and 204 in FIG. 2, or in a database, such as database 216 in FIG. 2.

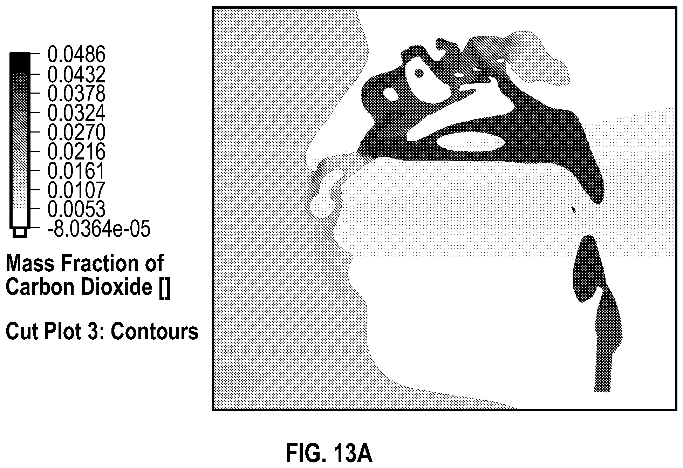

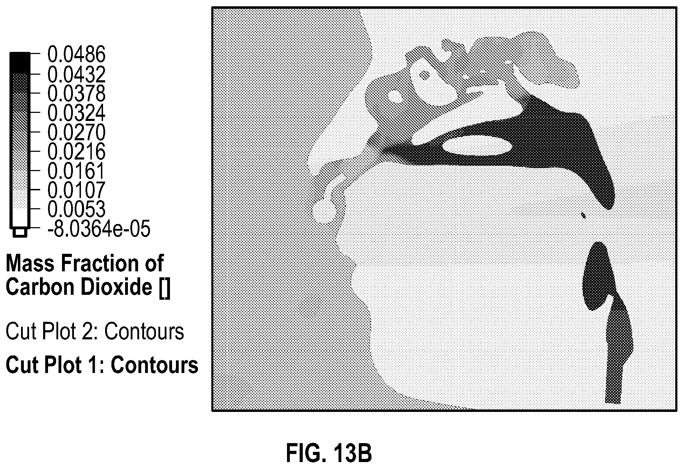

In some implementations, system 100 includes a display, which may be operated by the processor to, for example, display various system parameters or allow for user input. For example, the display may show any of the flowrate of breathing gas, exit velocity (e.g., the velocity calculated by the processor), humidity of the breathing gas, oxygen concentration, target flow setpoint, pressure drop, temperature, therapy duration, FiCh, and/or battery level. This information can have utility to the user, such as a physician, who may evaluate the system parameters and make a decision to change one or more of the parameters. In some implementations, a user may input a change to any of the system parameters, for example, through the display as a touch-screen or an interface (*e.g.,* interface 208 of FIG. 2). The processor may receive the user input, calculate changes to the one or more parameters, and direct the controller to change the system operating conditions accordingly. For example, the input may include a change from a first flowrate to a second flowrate, and the processor directs the controller to operate blower 102 at the second flowrate. The processor may then calculate a modified exit velocity based on cannula dimensions and the second flowrate. In some implementations, the user may choose an installed cannula from a drop-down list on the display as discussed above. Enabling the user to control the flow rate, the system can achieve effective flushing of the dead space in the patient's upper airway to provide better delivery of oxygen to the patient's lungs and provide an easier breathing experience. This effect is shown and described in relation to FIGs. 13A and 13B.

In some implementations, breathing gas 104 is humidified in system 100 before reaching nare 112. For example, the base unit or the auxiliary unit may comprise a vapor transfer cartridge configured to humidify or aerosolize water or medicament or a mixture thereof. Breathing gas 104 may be heated to reach a temperature optimal for humidification. A system which allows for controlling of a gas admixture with dew point and/or humidification management can be used to treat a wider range of patients. Furthermore, the system can be adapted with various gas mixtures and aerosolized medicaments to provide comfortable treatment of a patient. Alternative to a vapor transfer cartridge, in some implementations, a hot pot humidification chamber or evaporator is used to humidify the breathing gas, wherein the breathing gas is flowed over a reservoir of heated water that may be continuously or intermittently refilled. The water may be heated using an induction heating plate disposed in the reservoir, the plate generating heat due to a resistance against an current in the plate induced by a magnetic coil in the base unit.

FIG. 2 is a block diagram of a computing device, such as those described above in relation to FIG. 1, for performing any of the processes described herein, according to an illustrative implementation. Each of the components of these systems may be implemented on one or more computing devices 200. In certain aspects, a plurality of the components of these systems may be included within one computing device 200. In certain implementations, a component and a storage device may be implemented across several computing devices 200. Computing device 200 may be included in a base unit of a respiratory therapy unit, such as base units 402, 502, 602, 802, and 1002 (the computing device sometimes being referred to as a controller herein).

The computing device 200 includes at least one communications interface unit, an input/output controller 210, system memory, and one or more data storage devices. The system memory includes at least one random access memory (RAM 202) and at least one read-only memory (ROM 204). All of these elements are in communication with a central processing unit (CPU 206) to facilitate the operation of the computing device 200. The computing device 200 may be configured in many different ways. For example, the computing device 200 may be a conventional standalone computer or alternatively, the functions of computing device 200 may be distributed across multiple computer systems and architectures. In FIG. 200, the computing device 200 is linked, via network or local network, to other servers or systems.

The computing device 200 may be configured in a distributed architecture, wherein databases and processors are housed in separate units or locations. Some units perform primary processing functions and contain at a minimum a general controller or a processor and a system memory. In distributed architecture implementations, each of these units may be attached via the communications interface unit 208 to a communications hub or port (not shown) that serves as a primary communication link with other servers, client or user computers and other related devices. The communications hub or port may have minimal processing capability itself, serving primarily as a communications router. A variety of communications protocols may be part of the system, including, but not limited to: Ethernet, SAP, SASTM, ATP, BLUETOOTH™, GSM and TCP/IP.

The CPU 206 includes a processor, such as one or more conventional microprocessors and one or more supplementary co-processors such as math co-processors for offloading workload from the CPU 206. The CPU 206 is in communication with the communications interface unit 208 and the input/output controller 210, through which the CPU 206 communicates with other devices such as other servers, user terminals, displays, or devices, such as the components of system 100 of FIG. 1. The communications interface unit 208 and the input/output controller 210 may include multiple communication channels for simultaneous communication with, for example, other processors, servers or client terminals.

The CPU 206 is also in communication with the data storage device. The data storage device may include an appropriate combination of magnetic, optical or semiconductor memory, and may include, for example, RAM 202, ROM 204, flash drive, an optical disc such as a compact disc or a hard disk or drive. The CPU 206 and the data storage device each may be, for example, located entirely within a single computer or other computing device; or connected to each other by a communication medium, such as a USB port, serial port cable, a coaxial cable, an Ethernet cable, a telephone line, a radio frequency transceiver or other similar wireless or wired medium or combination of the foregoing. For example, the CPU 206 may be connected to the data storage device via the communications interface unit 208. The CPU 206 may be configured to perform one or more particular processing functions.

The data storage device may store, for example, (i) an operating system 212 for the computing device 200; (ii) one or more applications 214 (e.g., computer program code or a computer program product) adapted to direct the CPU 206 in accordance with the systems and methods described here, and particularly in accordance with the processes described in detail with regard to the CPU 206; or (iii) database(s) 216 adapted to store information that may be utilized to store information required by the program.

The operating system 212 and applications 214 may be stored, for example, in a compressed, an uncompiled and an encrypted format, and may include computer program code. The instructions of the program may be read into a main memory of the processor from a computer-readable medium other than the data storage device, such as from the ROM 204 or from the RAM 202. While execution of sequences of instructions in the program causes the CPU 206 to perform the process steps described herein, hard-wired circuitry may be used in place of, or in combination with, software instructions for implementation of the processes described herein. Thus, the systems and methods described are not limited to any specific combination of hardware and software.

Suitable computer program code may be provided for performing one or more functions described herein. The program also may include program elements such as an operating system 212, a database management system and "device drivers" that allow the processor to interface with computer peripheral devices (e.g., a video display, a keyboard, a computer mouse, etc.) via the input/output controller 210.

The term "computer-readable medium" as used herein refers to any non-transitory medium that provides or participates in providing instructions to the processor of the computing device 200 (or any other processor of a device described herein) for execution. Such a medium may take many forms, including but not limited to, non-volatile media and volatile media. Non-volatile media include, for example, optical, magnetic, or opto-magnetic disks, or integrated circuit memory, such as flash memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM or EEPROM (electronically erasable programmable read-only memory), a FLASH-EEPROM, any other memory chip or cartridge, or any other non-transitory medium from which a computer can read.

Various forms of computer readable media may be involved in carrying one or more sequences of one or more instructions to the CPU 206 (or any other processor of a device described herein) for execution. For example, the instructions may initially be borne on a magnetic disk of a remote computer (not shown). The remote computer can load the instructions into its dynamic memory and send the instructions over an Ethernet connection, cable line, or even telephone line using a modem. A communications device local to a computing device 200 (e.g., a server) can receive the data on the respective communications line and place the data on a system bus for the processor. The system bus carries the data to main memory, from which the processor retrieves and executes the instructions. The instructions received by main memory may optionally be stored in memory either before or after execution by the processor. In addition, instructions may be received via a communication port as electrical, electromagnetic or optical signals, which are exemplary forms of wireless communications or data streams that carry various types of information.

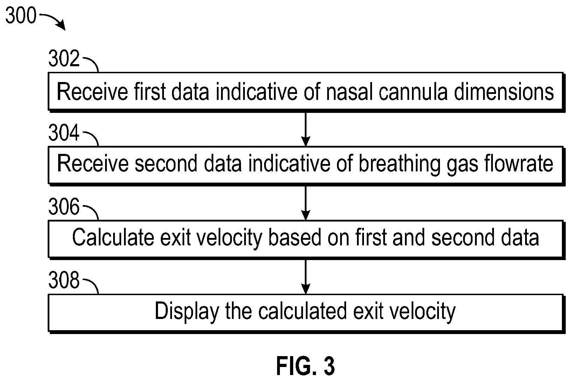

FIG. 3 is a flowchart of a process 300 for processing data relating to high velocity respiratory therapy, according to an illustrative implementation. Process 300 may be implemented on system 100 of FIG. 1, computing device 200 of FIG. 2, or any other suitable system or device. Step 302 involves receiving first data indicative of one or more nasal cannula dimensions. Step 304 involves receiving second data indicative of a breathing gas flowrate. For example, the first data and second data may be received by a processor, such as CPU 206. Step 306 involves calculating exit velocity of the breathing gas based on the first data and second data. Step 308 involves displaying the calculated exit velocity for a user.

The first data may include an inner diameter or cross-sectional area of one or more nasal prongs of a nasal cannula, such as nasal cannula 108 with prong 110. In some implementations, the first data is transmitted to a processor by a transmitter or by an RFID tag or other tag associated with the nasal cannula. In some implementations, the second data is indicative of one or more flowrate measurements taken by one or more sensors.

Process 300 may include additional steps. For example, a physician or other user may view the displayed velocity, other displayed parameters, or a patient's medical records/status and decide to change conditions of the therapy. In some implementations, process 300 further includes a step of receiving one or more user inputs. In some implementations, a user input is received indicating a desired change to at least one of the flowrate of breathing gas and the exit velocity. For example, a user input may indicate a modified flowrate, at which to operate the system or device. Alternatively or in addition, a user input may indicate a modified velocity. In some implementations, after receiving a user input, process 300 includes a step involving changing the flowrate to account for the user input indicating a change to the flowrate or exit velocity. The flowrate may be changed by a controller, such as input/output controller 210. This step may be followed by a step of calculating a modified flowrate if the user input is a desired velocity, such that the modified flowrate corresponds to the desired velocity. In some implementations, an additional step involves calculating a modified velocity based on the user input and the first data. For example, the user input may be a desired flowrate, and the processor calculates the modified velocity based on the dimensions of the at least one nasal prong and the desired flowrate.

In some implementations, steps 304 and 306 are altered such that the second data is indicative of the exit velocity, and the flowrate of breathing gas is calculated based on the first data and second data. In some implementations, step 306 calculates additional system parameters based on additional data. For example, the additional system parameters or additional data may include humidity of the breathing gas, oxygen concentration, target flow setpoint, pressure drop, temperature, therapy duration, FiO2, and/or battery level. Any of these system parameters/data may be displayed in step 308.

### System Design and Architecture

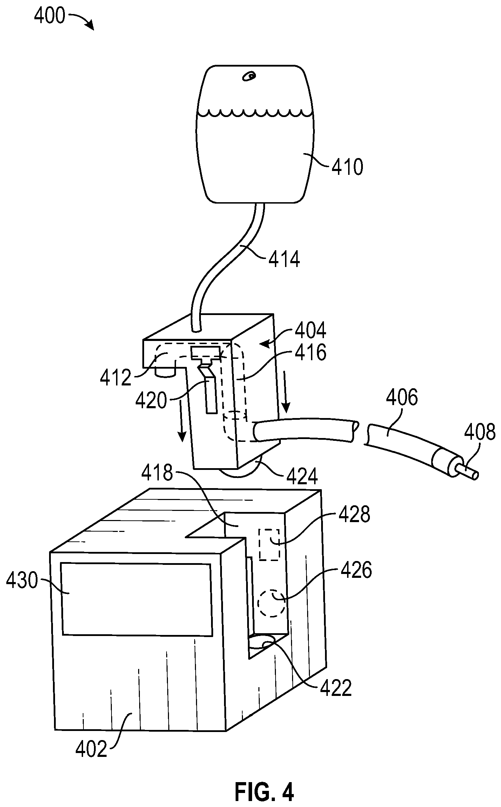

A respiratory therapy unit configured for providing high velocity breathing gas to a patient can include features to improve water management, heating, humidification sensing and automation, among others, resulting in increased patient comfort and safety as well as improved treatment capability. FIGs. 4-10 depict examples of such respiratory therapy units and components thereof. While these units and components are depicted separately, it is to be understood that the units and/or components may be implemented in any combination and subcombination. It may be desirable to combine certain units and/or components in order to establish interoperability between the certain units and/or components which may lead to additional advantages or technical effects beyond those provided by each individual unit or component.

In some implementations, the high velocity breathing gas is provided with a velocity of at least about 40 m/s and less than about 70 m/s, at least about 40 m/s and less than about 65 m/s, at least about 40 m/s and less than about 60 m/s, at least about 40 m/s and less than about 55 m/s, at least about 40 m/s and less than about 50 m/s, at least about 40 m/s and less than about 45 m/s, or about 40 m/s. In some implementations, the velocity is at least 50 m/s, at least 55 m/s, at least 60 m/s, at least 65 m/s, or at least 70 m/s. In some implementations, the velocity is greater than a velocity measured at any point along a flow path between a gas source and a nasal cannula prong configured to deliver the gas to a nare of the patient. The systems, methods, and devices may employ a non-sealing cannula, and in particular the cannula may be open to the atmosphere only at the nasal prong outlet. In some implementations, the gas provided at the prong outlet has a non-zero dynamic pressure. In some implementations, the breathing gas is humidified within the system. The flow path of gas may be closed between a connector for the nasal cannula to the delivery tube and the prong outlet.

FIG. 4 depicts a respiratory therapy system 400 for providing breathing gas to a patient, according to an illustrative implementation. System 400 comprises a base unit 402, an auxiliary unit 404, and a delivery tube 406. Base unit 402 contains a controller (not shown), and base unit 402 is configured to direct a flow of breathing gas into auxiliary unit 404 when auxiliary unit 404 is operatively coupled to base unit 402. Auxiliary unit 404 directs the breathing gas through gas path 412 to vapor transfer cartridge (VTC) 416 disposed within auxiliary unit 404. Auxiliary unit 404 is configured to receive a liquid (e.g., water) from external reservoir 410 through external liquid tube 414. Breathing gas flows from an outlet of VTC 416 into delivery tube 406 for delivery of the breathing gas to the patient. Delivery tube 406 includes a patient connector 408. For the operative coupling of auxiliary unit 404 to base unit 402, base unit 402 includes a recess 418 sized to receive auxiliary unit 404, and auxiliary unit 404 includes a latch 420 for locking auxiliary unit 404 in recess 418. Auxiliary unit 404 includes a pump 424 configured to pump the liquid. Base unit 402 includes various couplings 422, 426, and 428 that are configured to interact with auxiliary unit 404 or components thereof. A display 430 is included on base unit 402.

Base unit 402 may contain a blower configured to produce a flow of breathing gas. In the following, system 400 is described using a blower as an example; however, it is to be understood that base unit 402 (and the other base units described herein) may alternatively use a compressor or be connected to an external gas source such as a wall air outlet, an air tank, or an external blower. Furthermore, a VTC 416 is implemented in system 400; however, it is to be understood that alternatives may be implemented in system 400 (and the other systems described herein), such as a heated wire or hot pot humidifier. The alternatives may operate by disposing water over a heating plate in a chamber and flowing breathing gas into the chamber (either over the water or through the water) to be humidified. A heated wire can be disposed in a conduit to further heat the humidified breathing gas before delivering the breathing gas to a patient.

System 400 may further include a nasal cannula (not shown) coupled to patient connector 408. Alternatively, patient connector 408 may be a nasal cannula or other gas delivery device. In either case, system 400 may be operated with the previously discussed system parameters for high velocity respiratory therapy (i.e., the parameters in Tables 1 and 2). The nasal cannula or patient connector may be sized accordingly, and/or the blower may be operated at certain breathing gas flowrates. By using a blower, system 400 may absolve a need for an external source of pressurized air or breathing gas such as a pressurized tank or a wall air outlet. The controller of base unit 402 may be the computing device 200 of FIG. 2.

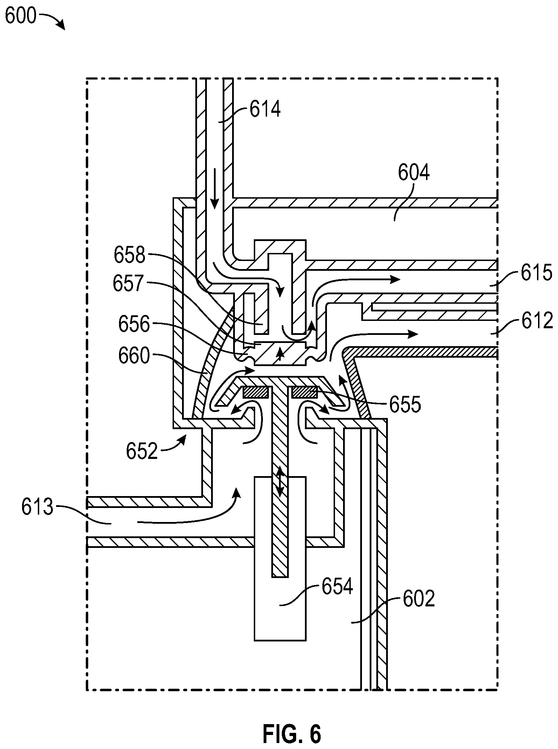

In some implementations, external reservoir 410 is a large container (>1 L, >2 L, >5 L, etc.) such as a bag or bottle, and the connecting tube 414 can be removably connected to auxiliary unit 404. Auxiliary unit 404 may be configured such that the liquid is fully enclosed in auxiliary unit 404 flowing from external reservoir 410. In this case, liquid is kept separate from base unit 402. This may prevent leaks and damage of components within base unit 402. In some implementations, gas path 412 is connected to the blower of base unit 402, but there is no other fluid communication between base unit 402 and auxiliary unit 404. Gas path 412 may be connected to base unit 402 by a valve, such as the occluder valves 652, 752, and 852 of FIGs. 6, 7D, 8A, and 8B. Such valve may be used to control the flow rate of breathing gas entering gas path 412 of auxiliary unit 404. The valve may also interact with liquid flow to simultaneously control breathing gas flowrate and liquid flowrate. The valve may comprise components that are separately attached to base unit 402 and auxiliary unit 404, such that the valve is operable when auxiliary unit 404 is fully seated in recess 418 of base unit 402, and the controller of base unit 402 actuates the valve. A gas seal, such as gas seals 660, 760, and 860 of FIGs. 6, 7A, 7B, 7D, 8A, and 8B, may be attached to auxiliary unit 404, in order to prevent gas leaks out of system 400 and to prevent liquid ingress into base unit 402 during operation. The gas seal may be a flexible portion that surrounds the valve, and the flexible portion is compressed to form a seal when auxiliary unit 404 is fully seated in recess 418 of base unit 402. The flexible portion of the gas seal, throughout this disclosure, may be a film, a gasket, a ring, or a collar, or other suitable mechanism.

While base unit 402 and auxiliary unit 404 are fluidically connected to at least allow breathing gas flow, it can be advantageous to otherwise minimize fluidic connections or contact between base unit 402 and auxiliary unit 404. For example, minimizing fluidic contact with base unit 402 may prevent damage to electronic components. As another example, previous systems have relied on conductive heating of liquid, which requires precise contact between two plates; thus, conductive heating is inefficient if precise contact cannot be established due to misalignment or deformation. Accordingly, couplings 422, 426, and 428 are configured to be contactless or non-contact couplings. Generally, this allows a sealed auxiliary unit without any openings and minimal requirements for tight tolerances. In some implementations, coupling 422 is a stator configured to magnetically couple to pump 424 which includes a rotor. Pump 424 may be shaped like a cup with a convex side protruding from auxiliary unit 404, and the stator may surround a cavity in recess 418, such that pump 424 sits within the cavity when auxiliary unit 404 is seated in recess 418. Pump 424 and coupling 422 may each be hermetically sealed, for example, to prevent fluid ingress into base unit 402. When auxiliary unit 404 is fully seated in base unit 402, the controller can operate the stator to magnetically generate rotation of the rotor of pump 424 to pump liquid through the auxiliary unit or through a jacket of delivery tube 406. As a result, the auxiliary unit 404 pumps liquid without an internalized power source or other electronic components to control the pump 424, and there is no need for liquid to flow through the base unit 402 which separately houses the controller and other components that may be susceptible to, *e.g.,* water damage. Pump designs compatible with the present disclosure are described in U.S. Patent Application Publication No. 2018/0104436.

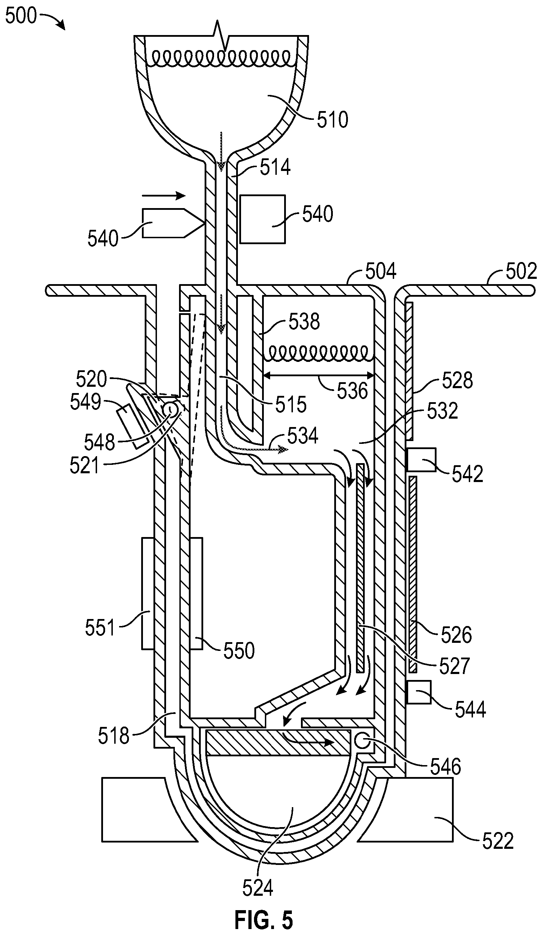

Similarly, coupling 426 may be a contactless or non-contact heating actuator, for example, heat actuator 526 of FIG. 5, used to convey energy to auxiliary unit 404 to heat the liquid. In some implementations, coupling 426 is a coil configured to heat a plate disposed in auxiliary unit 404. The plate may be, for example, the heating plates 527, 727, or 927 of FIGs. 5, 7C, 7F, and 9. The coil may heat the plate via induction. This may involve the coil inducing a current in the plate that is circular in shape and immersed in liquid in auxiliary unit 404. The induced current generates heat in the plate due to a resistance of the plate. Because the heat is generated in the plate that is surrounded by liquid, external surfaces of auxiliary unit 404 and base unit 402 that can be touched by a user/operator never exceed the liquid temperature. This configuration eliminates the need for precisely manufactured flat plates to get intimate contact between a heat source in base unit 402 and a conductive plate in auxiliary unit 404. Inductive heating improves efficiency of heating, because the generated heat is isolated to auxiliary unit 404 and does not heat the base unit 402. If base unit 402 were heated by excess heat, additional cooling would be required to remove the heat; however, additional cooling is unnecessary with the present inductive heating configuration. Improved efficiency also allows longer operation of system 400 if power is supplied by a battery (not shown) in situations where wall power is not available, e.g., during mobile use or power outages.

Heated liquid may be used to heat, in turn, breathing gas. For example, delivery tube 406 may include a jacket configured to receive heated liquid and convey the heated liquid around an inner lumen that conveys the breathing gas. Heated liquid may travel along a full length of delivery tube 406 in a first direction towards patient connector 408, and then return in an opposite direction back to auxiliary unit 404. Heated liquid may also be conveyed into VTC 416 and vaporized into the breathing gas. For example, the liquid may be water used to humidify the breathing gas. Accordingly, the liquid may be heated to a target temperature, where the target temperature is a temperature at which the gas is to be delivered to the patient. The liquid may be circulated through auxiliary unit 404 and delivery tube 406 and back to the internal reservoir. In some implementations, the liquid travels a liquid path from the external reservoir to the internal reservoir (internal to auxiliary unit 404), then to a heating section (e.g., where the heating plate may be disposed), then through pump 424, then into the heating jacket of delivery tube 406, then into VTC 416, and then any remaining, un-vaporized liquid is returned to the internal reservoir. A similar configuration is described in further detail in relation to FIG. 5.

Similar to coupling 426, coupling 428 may be a contactless or non-contact level sensor, for example, level sensor 528 of FIG. 5, configured to detect a liquid level in auxiliary unit 404. For example, auxiliary unit 404 may contain an internal reservoir, such as the one described above and internal reservoirs 532, 732, and 932 of FIGs. 5, 7C, 7D, 7F, and 9. Liquid is stored in the internal reservoir for use, e.g., in VTC 416 and/or heating jacket of delivery tube 406. When the liquid is water that is vaporized into the breathing gas within VTC 416, it may desirable for the system or user to know the humidity of breathing gas provided to the patient. In some implementations, the internal reservoir has a constant diameter or cross-section (for example, cross-section 536 of internal reservoir 532 of FIG. 5), so that the volume of liquid is linearly proportional to the liquid level in the reservoir. In this implementation, level sensor 428 is configured to measure the liquid level when auxiliary unit 404 is fully seated in base unit 402. Level sensor 428 may be a capacitive sensing circuit positioned alongside the internal reservoir. In this implementation, the liquid level may be measured, because the capacitance of the liquid changes as the liquid level changes. Capacitive sensing provides a non-contact means for detecting the liquid level.

Level sensor 428 may output one or more signals indicating the measured liquid level to the controller in base unit 402. The occluder valve or a pinch valve disposed along tube 414 can be used to isolate the volume of liquid in auxiliary unit 404. In implementations where liquid only leaves system 400 when vaporized into the breathing gas in VTC 416, the controller can determine the amount of liquid vaporized (i.e., the vaporization rate) or otherwise consumed (i.e., consumption rate) over time. In some implementations, this information is used to determine the flowrate of liquid needed from external reservoir 410 in order to keep the liquid level at a steady state, where the liquid flowrate from external reservoir 410 equals the vaporization rate, in order to, for example, prevent auxiliary unit 404 and VTC 416 from drying up or ensure the breathing gas is kept at a constant vapor concentration (e.g., humidity). In other implementations, the liquid level is not at a steady state, but the controller determines, based on the vaporization flowrate, an appropriate time interval at which to allow a fixed volume of the liquid to enter the internal reservoir from external reservoir 410 that is approximately equal to the amount of liquid vaporized during that time interval.

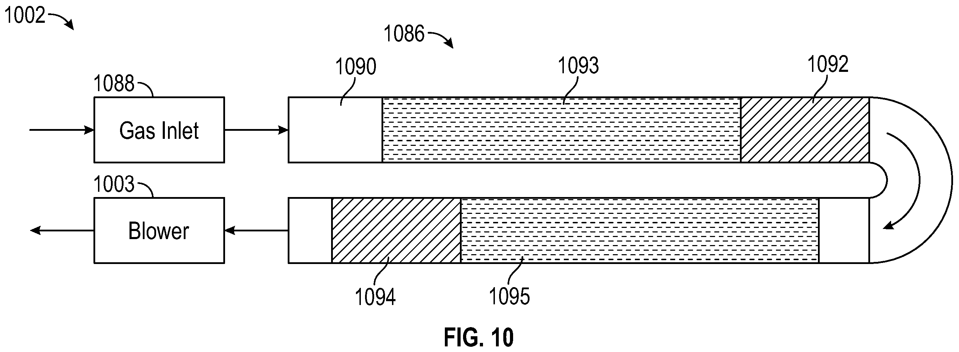

From the calculated vaporization rate, the controller may also compute the humidity of the breathing gas exiting VTC 416 in implementations where the liquid is water. Another device, such as measurement device 1086 of FIG. 10, may be used to determine the flowrate (mass flowrate and/or volumetric flowrate) of breathing gas in system 400. Such a device may be disposed in base unit 402 to measure breathing gas entering or exiting the blower, or the device may be disposed in auxiliary unit 404 to measure breathing gas flowrate entering or exiting auxiliary unit 404 or VTC 416. The device may alternatively be disposed along or within delivery tube 406 or patient connector 408 for a patient-proximate measurement of the breathing gas flowrate. In any of these cases, the device outputs one or more signals indicating the breathing gas flowrate to the controller. Also knowing the consumption rate or vaporization rate, the controller can calculate the humidity of breathing gas based on the liquid consumption/vaporization rate and the breathing gas flowrate, because liquid in the closed auxiliary unit 404 may only exit system 400 as vapor in the breathing gas when liquid flow from external reservoir 410 is stopped.

Knowing the humidity can serve several purposes. For example, an inappropriately low or high humidity may indicate that auxiliary unit 404, VTC 416, or another component of system 400 has become defective and requires replacement, allowing the controller to halt operation of system 400 and notify the operator before the patient is harmed. As another example, the performance of system 400 may be monitored over time, using humidity as a monitored variable, in order to detect deterioration and extend the usable lifetime of system 400. In this case, there is no need to limit all devices to the performance of outliers during lifetime testing. Also, knowing humidity, adjustment of a temperature within VTC 416 relative to a temperature within delivery tube 406 can allow for adjustment of the humidity if the humidity output is monitored for safety. In some implementations, system 400 stores (e.g., on a memory) a range of safe humidity levels for patient breathing gas, and the controller is configured to determine if the calculated humidity is within the safe range. If the calculated humidity falls outside of the safe range, the controller may adjust the power of pump 424 to adjust liquid flowrate and/or adjust the power of the blower to adjust breathing gas flowrate.

In some implementations, VTC 416 comprises a bypass gas passage, configured to allow a portion of the breathing gas flow to flow through the bypass gas passage where it is not humidified, and then it is added back to the humidified gas output by the VTC 416. The flowrate of gas through the bypass gas passage may be controlled in order to control the level of humidity in the combined flow of breathing gas. The systems and methods described herein may utilize any of the methods of humidity control described in U.S. Patent No. 10,596,345 filed December 31, 2014, and titled "SYSTEMS AND METHODS FOR HUMIDITY CONTROL".

Latch 420 is configured to lock auxiliary unit 404 in recess 418 of base unit 402 when auxiliary unit 404 is correctly positioned/seated in recess 418 for proper operation of system 400 and interoperability between auxiliary unit 404 and base unit 402. A user may press against latch 420 to unlock auxiliary unit 404 and remove it from base unit 402. In order to ensure that auxiliary unit 404 is fully seated in recess 418 for proper operation, there may be one or more elements disposed in base unit 402 and auxiliary unit 404 configured to detect the presence, or the precise alignment of auxiliary unit 404 in recess 418. In some implementations, auxiliary unit 404 includes a RFID tag (*e.g.,* RFID tag 550 of FIG. 5) or other tag, and base unit 402 includes an antenna or reader (*e.g.,* RFID reader 551 of FIG. 5). The tag can be detected by the antenna or reader in a non-contact manner when the tag is positioned near the antenna or reader, essentially when auxiliary unit 404 is seated in recess 418.

Accordingly, the tag may also include information that can be read by the antenna or reader and transmitted to the controller or a memory of base unit 402. For example, the tag may include information describing the type of or feature in auxiliary unit 404, such as low flow, high flow, aerosolization, humidification, oxygenation, nitric oxide, helium, and/or closed loop oxygen control. The tag may also include information relating to the state of the specific auxiliary unit 404 and/or its various components. For example, the information may include use history (including the in-use date), a shelf-life or remaining lifetime, embedded licensing, and/or recommended operating parameters (limit flowrates or humidity). The controller may use any of the information described above to determine and execute appropriate adjustments to system 400 and any of its various components. For example, depending on the type or features of auxiliary unit 404, certain operations or features may be enabled or disabled by the controller in accordance with the in-use auxiliary unit 404.

RFID tags or other tags can provide additional information about an auxiliary unit such as the auxiliary unit type, when it was first used, the expiration date, etc. This can overcome the drawbacks of the above-mentioned systems. In some systems the device needs to be aware of a change in auxiliary unit even if the capital unit is in standby. To detect an auxiliary unit change in these systems RFID may require the device to use too much power while in standby. As an example, an active valve can close the air path when the auxiliary unit is removed and close the water fill path when an auxiliary unit is installed. Such features may not function correctly if the auxiliary unit is added or removed when the device is off or in a standby mode. This can occur if the auxiliary unit is set up in advance to be ready for a patient. The device is off when the auxiliary unit is installed and water may flow uncontrolled into the device. It would be beneficial for the device to power on and close the valve.

In some implementations, auxiliary unit 404 incudes one or more magnets (*e.g.,* alignment marker 548 of FIG. 5), and base unit 402 includes a Hall effect sensor (*e.g.,* alignment sensor 549 of FIG. 5) configured to sense the presence of the magnet in order to determine in a non-contact manner if auxiliary unit 404 is fully seated in recess 418. A Hall effect sensor measures a magnitude of a magnetic field and outputs a voltage proportional to the magnetic field magnitude. Accordingly, the Hall effect sensor may detect the magnetic field of the magnet when in close proximity. By using a magnet and Hall effect sensor, the precise position of auxiliary unit 404 may be detected, and a change in state may be detected even when system 400 is in a stand-by or low-power mode, because the Hall effect sensor may operate on a very low power when system 400 is in stand-by, and a change in this Hall effect sensor can be used to wake system 400 to, for example, read the RFID tag or start operation of certain components such as the blower or pump 424. As another example, the controller may be configured to close the occluder valve to block breathing gas flow from base unit 402 to auxiliary unit 404 when auxiliary unit 404 is removed, even if system 400 is in stand-by, to ensure that the gas path is not left open when the disposable is removed. The magnet may be configured to have a narrow range in which it can trigger the Hall effect sensor. The range may be less than about 5 mm, less than about 4 mm, less than about 3 mm, less than about 2 mm, or less than about 1 mm. In some implementations, the magnet is disposed within latch 420, so that the Hall effect sensor is triggered by the magnet when latch 420 is in the locked position. A barcode may be used in auxiliary unit 404 in addition to or in place of an RFID tag or other tag, such that a reader in base unit 402 reads the barcode when auxiliary unit 404 is docked in recess 418. The barcode may include the information about one or more components, as is described in relation to the RFID tag in this disclosure. As another option, base unit 402 includes an infrared (IR) proximity sensor having an IR beam positioned such that latch 420 breaks the beam, triggering the sensor, when auxiliary unit 404 is fully seated in recess 418. Other options for auxiliary unit presence sensing and data storage/transfer include quick response (QR) codes, global positioning system (GPS) chips, Bluetooth®, Bluetooth® low-energy, near-field communication (NFC), and pattern recognition matching.

System 400 may be configured to automatically switch to a stand-by or low-power mode under certain conditions. For example, system 400 may switch to stand-by mode when auxiliary unit 404 is not detected, when the blower is off and a user has not interacted with system 400 for a certain period of time, or when a user inputs a command to switch system 400 to the stand-by mode. When switching to stand-by or low-power mode, the controller may generate an alert to notify the user of the switch. System 400 may be configured to be powered by a standard wall outlet or a main battery. In either case, system 400 may include a reserve battery. The reserve battery may be a rechargeable battery, such as lithium-ion, lead-acid, nickel-cadmium, nickel-metal hydride, lithium-ion polymer, alkaline, or another equivalent. The reserve battery may be configured to provide power to system 400 when the wall outlet becomes unusable (*e.g.,* during a power outage) or when the main battery requires replacement. In some implementations, system 400 can operate on solely the reserve battery for at least about 30 minutes, at least about 1 hour, at least about 2 hours, or at least about 5 hours. The stand-by or low-power mode may limit power usage of system 400 to a percentage of the maximum power usage of system 400 during normal operation (*e.g.,* about 80%, about 70%, about 60%, about 50%, about 40%, about 30%, about 20%). In some implementations, the controller switches system 400 to the stand-by or low-power mode when the reserve battery is in use. When in stand-by or low-power mode, the controller may disable certain features, for example, to reduce power consumption. For example, the controller may disable heating elements or supplementary gas input, allowing operation of the blower and one or more alarms (*e.g.,* auditory alarms, notifications on display 430).

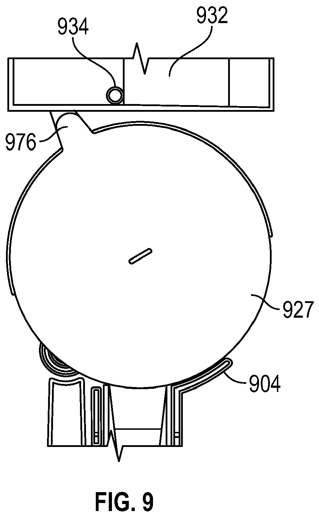

System 400 may also include one or more temperature sensors, such as temperature sensors 542 and 544 of FIG. 5. In some implementations, base unit 402 includes one or more infrared (IR) temperature sensors configured to measure liquid temperatures in auxiliary unit 404 by non-contact means. One or more signals indicating the liquid temperature may be transmitted from the sensor(s) to the controller. The controller may be configured to determine if the measured temperature is within a stored safe temperature range. If the temperature is within the safe range, then the controller may alert the user by one or more alarms (*e.g.,* auditory alarms, notifications on display 430) and/or change one or more controllable operational parameters to effect a change in the temperature. This may involve changing the blower flowrate, In some implementations, base unit 402 includes one or more IR temperature sensors configured to measure a temperature of the heater plate that is immersed in liquid in auxiliary unit 404, for example, to detect overheating of the heater plate. In some implementations, the heater plate includes a tab that protrudes from the top of the heating plate as depicted in FIG. 9. The tab may be disposed such that it is the first part of the heating plate to be exposed (not immersed in liquid) when the liquid is at a low level. When the tab is not immersed, it will heat up quickly if the heating plate and heating element are in operation. A temperature sensor may be disposed to align with the tab when the auxiliary unit is seated, so that the temperature of the tab may be monitored by sending one or more signals indicating the tab temperature to the controller. When the tab heats up quickly during a low liquid level state of auxiliary unit 404, the temperature sensor can detect the rapid rise in temperature and alert the controller. In response to detecting a rapid rise in the tab temperature indicative of a low liquid level, the controller may be configured to turn off the heating element (*e.g.,* the induction coil). In response to detecting a rapid rise in the tab temperature indicative of a low liquid level, the controller may be configured to increase or open the liquid flow from external reservoir 410 by, for example, actuating the occluder valve or pump 424. In response to detecting a rapid rise in the tab temperature indicative of a low liquid level, the controller may be configured to completely shut off system 400 and/or generate an alarm. An alarm may be auditory or visual via display 430.

Delivery tube 406 conducts the breathing gas to the patient. Jacketed delivery tubes are typically smaller in inside diameter than heated wire delivery tubes and have fewer related issues. For example, the weight of liquid needed to properly heat a larger tube can provide a poor user experience if the tube diameter is increased too much, because the user may have difficulty moving a tube substantially weighed down by the liquid. Smaller jacketed delivery tubes can work when the pressure of the pressure source is high enough. In system 400, breathing gas is pressurized and supplied from the blower in base unit 402, without external pressurization in some implementations, so limited pressure may be available. With a blower, the tube diameter needs to be increased to account for a the lower pressure source. It may be advantageous to size a cross-sectional diameter of delivery tube 406 to be as large as possible, in order to accommodate the lower back pressure while maintaining functionality. Generally, an increased diameter increases the weight of the delivery tube due to the larger volume of liquid required to heat the gas. Increasing the diameter can also lead to water accumulation in the delivery tube. Unlike the larger heated wire tubes, which must be tilted to drain liquid to the patient, the smaller jacketed tube may deliver water to the patient by simply increasing the flow above a certain threshold where the water may be conducted along the delivery tube by the flow of breathing gas. Accordingly, an upper limit of the diameter may be set based on, when operating at a minimum breathing gas flowrate, the velocity of breathing gas is sufficient to convey any liquid that has built-up in delivery tube 406 as rain-out to patient connector 408 where it may be collected and removed. In some implementations, the breathing gas flowrate is 5 L/min, and the delivery tube inner diameter is less than or equal to about 0.26 in. Delivery tube 406 may be constructed of a flexible material as to allow a user or operator to manipulate delivery tube 406 without obstructing breathing gas flow.

The increased diameter can also make the delivery tube more prone to kinking. Accordingly, delivery tube 406 may be constructed to be kink resistant. In some implementations, delivery tube 406 includes a jacket. As discussed above, heated liquid may be used to heat, in turn, breathing gas. For example, delivery tube 406 may include a jacket configured to receive heated liquid and convey the heated liquid around an inner lumen that conveys the breathing gas. Heated liquid may travel along a full length of delivery tube 406 in a first direction towards patient connector 408, and then return in an opposite direction back to auxiliary unit 404. In some implementations, the jacket includes a plurality of ribs extending in a radial direction through the jacket from a first conduit defining a breathing gas lumen to a second conduit, wherein the jacket is defined as the annular space between the first conduit and the second conduit. The radial ribs define two or more channels of the jacket through which liquid may flow. The radial ribs allow bending of delivery tube 406 in any direction without kinking by preventing collapse of the inner breathing gas conduit when delivery tube 406 is bent. In some implementations, under flexure of delivery tube 406, one or more of the channels may collapse, but at least some of the channels and the breathing gas lumen remain open to allow for liquid and breathing gas flow, respectively. The jacket having radial ribs may mimic a thick-walled tube to prevent collapse of the breathing gas lumen. In some implementations, the breathing gas lumen wall has a thickness proportional to the breathing gas lumen inside diameter. In some implementations, the outer wall, defining the jacket, has a thickness proportional to the outer diameter of delivery tube 406 minus the inner diameter of the breathing gas lumen. In some implementations, each radial rib has a width proportional to the wall thickness of the breathing gas lumen.

Display 430 may be configured to show one or more parameters of system 400. For example, display 430 shows any of: breathing gas flowrate, liquid vaporization/consumption rate, humidity, liquid level, breathing gas velocity, heating plate temperature, and liquid temperature. In some implementations, display 430 acts as an interface for both alerting the user of system parameters and for accepting user inputs. User inputs, such as those previously described, may include target values for any of these parameters. The user inputs may be transmitted to the controller which is configured to adjust the parameters, for example, by actuating one or more of the components of system 400.

Breathing Gas and Liquid Flow Paths

As described above in relation to FIG. 4, the liquid in systems described herein follows a flow path during operation of said systems. As there are several components that may utilize or act on the liquid, there may be an ordered flow path through which liquid flows and undergoes various state changes or manipulations.

FIG. 5 is a cross-section of a respiratory therapy system 500 configured to output breathing gas to a patient, according to an illustrative implementation. System 500 includes a base unit 502 having a recess 518 in which auxiliary unit 504 is seated. Latch 520 locks auxiliary unit 504 in its current position. Liquid is stored in internal reservoir 532 of auxiliary unit 504. External reservoir 510 supplies liquid through external tube 514 to internal tube 515 of auxiliary unit 504. A valve 540 is disposed along external tube 514. Liquid flowing through internal tube 515 is conveyed into internal reservoir 532 through internal reservoir inlet 534. A vent 538 is disposed on internal reservoir 532. Liquid flows out of internal reservoir 532 and then alongside heating plate 527 immersed in the liquid. After heating, liquid flows into pump 524 and then out of pump outlet 546. Auxiliary unit 504 also includes an RFID tag 550 and an alignment marker 548. Base unit 502 includes a stator 522, a level sensor 528, a heat actuator 526, an RFID reader 551, an alignment sensor 549, and temperature sensors 542 and 544. System 500 and its components may combined with or modified by the systems and devices described in FIGs. 4, 6, 7A-7F, 8A, 8B, 9, and 10.

The various components in base unit 502 may be configured to couple to corresponding components of auxiliary unit 504 in a non-contact manner. These components may be operatively coupled to a controller in base unit 502 for actuation and/or electronic communication. In some implementations, stator 522 is configured to magnetically couple to a rotor of pump 524. By magnetically coupling to the rotor, stator 522 may induce rotational motion in the rotor for non-contact control of pump 524. Heat actuator 526 may convey by non-contact means heat to or generate heat in heating plate 527 immersed in liquid. Heat actuator 526 may be operatively coupled to the controller to allow for indirect actuation of the rate at which heat is transferred to liquid surrounding heating plate 527. In some implementations, heat actuator 526 is a coil configured to generate a current in heating plate 527 via induction, the current generating heat due to a resistance in heating plate 527. The generated heat may transfer from immersed heating plate 527 to liquid on either side. In some implementations, level sensor 528 is a capacitive sensor configured to measure capacitance in internal reservoir 532. Internal reservoir 532 may have a constant cross-section 536, so the measured capacitance is indicative of a liquid level in internal reservoir 532. By utilizing capacitive sensing, the system may provide an early warning of low liquid levels and active control of the water level in the system. Measurements of flow rate and humidification may also be more accurate with the use of capacitive sensing to accurately measure remaining liquid water.

In some implementations, RFID reader 551 detects the presence of RFID tag 550 when auxiliary unit 504 is within recess 518. RFID reader 551 may read information stored on RFID tag 550. In some implementations, alignment marker 548 is disposed in latch 520, and alignment sensor 549 is configured to detect the presence of alignment marker 548 when latch 520 is in a locked position. Latch 520 in an unlocked position 521 is depicted in FIG. 5 by a dashed outline. In some implementations, alignment marker 548 is a magnet, and alignment sensor 549 is a Hall effect sensor configured to detect the magnetic field of the magnet. Alignment sensor 549 may transmit to the controller a signal indicating auxiliary unit 504 is fully seated in recess 418 when alignment marker 548 is detected. A barcode may be used in auxiliary unit 504 in addition to or in place of RFID tag 550, such that reader 551 in the base unit reads the barcode when auxiliary unit 504 is fully docked in recess 518 of base unit 502. The barcode may include the information about one or more components, as is described in relation to the RFID tag in this disclosure. As an alternative to alignment marker 548, the base unit may include an infrared (IR) proximity sensor having an IR beam positioned such that latch 520 breaks the beam, triggering the sensor, when auxiliary unit 504 is fully seated. In some implementations, temperature sensors 542 and 544 are IR temperature sensors configured to measure temperatures in auxiliary unit 504. For example, temperature sensor 542 may be positioned to measure a temperature of heating plate 527, and temperature sensor 544 may be positioned to measure a temperature of the liquid flowing away from heating plate 527.

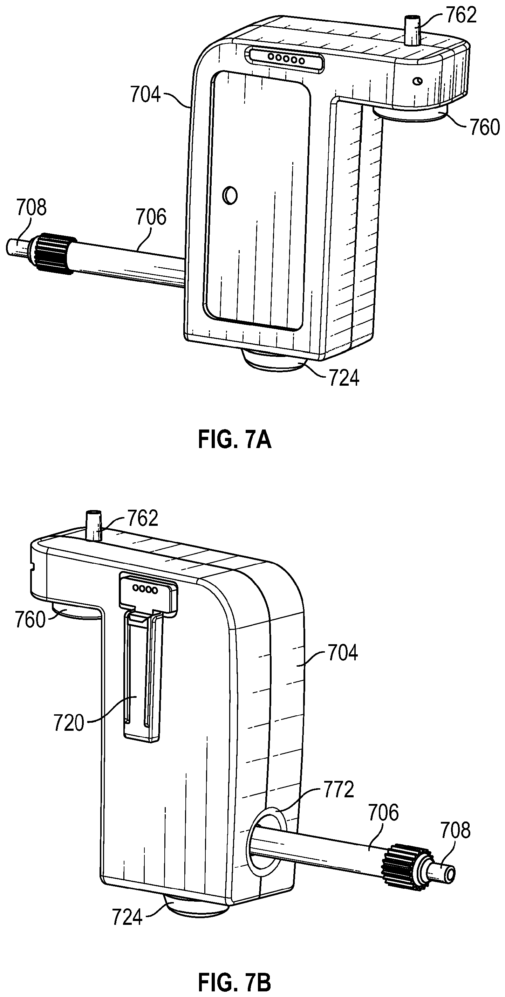

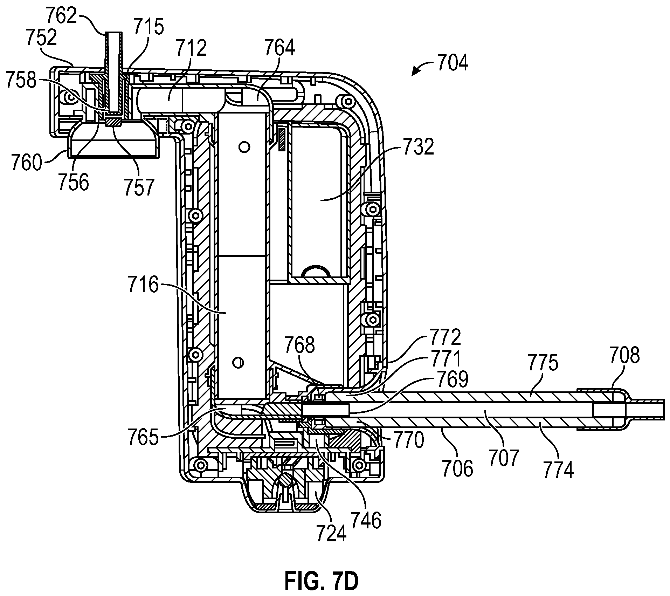

Liquid may flow out of internal reservoir 532 to heating plate 527 and pump 524 due to hydrostatic pressure resulting from the height of internal reservoir 532 positioned above heating plate 527 and pump 524. In some implementations, pump 524 generates a suction pressure that draws liquid from internal reservoir 532, past heating plate 527, and into pump 524. Rotational motion of a rotor (not shown) in pump 524 may be used to convey the liquid along a flow path. The flow path may be partially enclosed in auxiliary unit 504. In some implementations, system 500 further includes a jacketed delivery tube (such as delivery tubes 406 and 706 of FIGs. 4, 7A, 7B, and 7D-7F), and the flow path may extend through the jacket of the delivery tube. In some implementations, pump outlet 546 is in fluid communication with the jacket, such that heated liquid is pumped into the jacket for heating of the breathing gas in an inner lumen of the delivery tube, wherein the inner lumen is concentrically surrounded by the jacket. Pump 524 may generate sufficient pressure such that liquid is conveyed through a first lumen of the jacket away in a direction away from auxiliary unit 504 and through a second lumen of the jacket in an opposite direction back to auxiliary unit 504. In some implementations, auxiliary unit 504 houses a vapor transfer cartridge (VTC), such as VTC's 416, 716, and 816 of FIGs. 4, and liquid is conveyed from the delivery tube jacket into VTC for humidification of the breathing gas.

In some implementations, pump 524 conveys the liquid in a cyclic manner, in which liquid outputted from pump outlet 546 is pumped first through the delivery tube jacket, then through the VTC, into internal reservoir 532 (*e.g.,* through a recycled liquid reservoir inlet not shown), and finally past heating plate 527 before returning into pump 524. In this case, pump 524 generates enough pressure to convey the liquid through an entire cycle of this flow path for the liquid to return to pump 524. This ordered flow path may define a cycle or loop of fluid flow. This loop may be a closed loop, for example, if valve 540 is closed to prevent fluid flow through external tube 514. The loop may be sealed such that, when the loop is closed, liquid only leaves the loop as vapor in the VTC. In some implementations, the volume of liquid in the loop is at a steady state such that the rate of liquid leaving the loop as vapor in the VTC is equal to the rate of liquid entering the loop through tubes 514 and 515 from external reservoir 510. In other implementations, discrete volumes of the liquid are conveyed from external reservoir 510 through tubes 514 and 515 to the internal reservoir 532 at time intervals, such that the discrete volume for a given time interval is equal to the amount of liquid vaporized during that time interval.

Valve 540 may be used to isolate the volume of liquid in the loop, for example, by closing valve 540 to stop all liquid flow through tubes 514 and 515 from external reservoir 510. In some implementations, level sensor 528 outputs one or more signals to the controller in base unit 502 indicative of the liquid level in internal reservoir 532. Internal reservoir 532 may have constant cross-section 536, such that liquid level may be directly proportional to the volume of liquid in internal reservoir 532. When liquid flow is occluded by valve 540, liquid may only exit the loop by vaporization in the VTC. The controller can determine the vaporization rate (or consumption rate of liquid by the VTC) based on a change in liquid level in internal reservoir 532 over a period of time. If the controller also knows the breathing gas flowrate, for example, by receiving one or more signals indicating the flowrate (*e.g.,* from the measurement device 1086 of FIG. 10), then the controller can be configured to determine the vapor content or humidity of the breathing gas using the breathing gas flowrate measurement(s) and the liquid level measurements. The absolute humidity can also be determined by the controller by also taking into account temperature measurements, for example, indicated by one or more signals transmitted to the controller by temperature sensors configured to measure the temperature of the breathing gas in the VTC, at the VTC exit, or in the delivery tube

While external valves such as valve 540 can be used to control liquid flow from an external reservoir, it can be advantageous have a valve built into an auxiliary unit to, for example, allow for optimized control of the liquid flow by a controller. Furthermore, as the use of breathing gas and liquid is intertwined, a built-in valve that provides simultaneous control over breathing gas and liquid flows may enable further optimization and ease of control.

FIG. 6 depicts a portion of a respiratory therapy system 600 having a base unit 602 and auxiliary unit 604 joined by an occluder valve 652, according to an illustrative implementation. Occluder valve 652 is actuated by an actuator 654 which controls the positioning of gas path valve seal 655. Breathing gas enters valve 652 through base unit gas path 613 and exits valve 652 through auxiliary unit gas path 612. Liquid travels through external tube 614 and into valve 652 through liquid inlet 658, and exits valve 652 through internal tube 615. Valve 652 includes a gas seal 660 that is configured to form a closed annular space around gas path valve seal 655 between paths 613 and 612. Valve 652 includes a flexible portion 656 having a liquid path valve seal 657. System 600 and its components may combined with or modified by the systems and devices described in FIGs. 4, 5, 7A-7F, 8A, 8B, 9, and 10.

Actuator 654 is coupled to a controller (not shown) in base unit 602. Actuator 654 may be configured to move in a linear direction, as shown with the up and down arrows on actuator 654. The controller may send one or more signals to actuator 654 in order to adjust the position of actuator 654. Gas path valve seal 655 is coupled to linear actuator 654, such that linear movement of actuator 654 would also move gas path valve seal 655 in a linear direction, allowing gas path valve seal 655 to be positioned in a plurality of positions along the linear direction. Gas path valve seal 655 as shown in FIG. 5 is in a middle position which allows breathing gas flow from gas path 613 into the annular space defined by gas seal 660 in valve 652. As the position of gas path valve seal 655 may be varied, the flowrate of breathing gas from gas path 613 may be varied as a result of actuating gas path valve seal 655 by actuator 654. From the middle position of gas path valve seal 655 as shown in FIG. 5, actuator 654 may be configured to raise gas path valve seal 655 to enlarge the gap and increase the breathing gas flowrate through valve 652. Actuator 654 may be configured to lower gas path valve seal 655 to narrow the gap defined by gas path valve seal 655 and decrease the breathing gas flowrate through valve 652. Actuator 654 may lower gas path valve seal 655 by a certain distance such that gas path valve seal 655 fully occludes breathing gas flow from gas path 613, reducing the breathing gas flowrate to zero.

Flexible portion 656 may similarly have a variable position by virtue of its flexibility. Flexible portion 656 and liquid path valve seal 657 may be moved in a linear direction indicated by the up and down arrows on liquid path valve seal 657. Liquid path valve seal 657 may be formed integrally in flexible portion 656 or disposed in flexible portion 656. Liquid path valve seal 657 may be rigid relative to flexible portion 656, for example, by having a greater thickness than portion 656 or having a reinforced structure. Gas path valve seal 655 may be raised by actuator 654 from its position indicated in FIG. 6 such that gas path valve seal 655 abuts liquid path valve seal 657. Accordingly, controlling actuator 654 may allow system 600 to adjust the position of liquid path valve seal 657 by pushing up against it with gas path valve seal 655. Adjusting the position of liquid path valve seal 657 either narrows or enlarges a gap between liquid inlet 658 and liquid path valve seal 657, wherein liquid from external tube 614 flows through the gap when entering valve 652 on its path into auxiliary unit 604. Narrowing and enlarging the gap may decrease and increase, respectively, the flowrate of liquid out of liquid inlet 658.

Accordingly, valve 652 may provide simultaneous control of both breathing gas and liquid flowrates into auxiliary unit 604. In some implementations, linear actuation of actuator 654 allows for a non-discrete positioning of valve seals 655 and 657. In some implementations, the controller is configured to control actuator 654 between a discrete set of positions. In some implementations, the set includes number of positions > 1, > 2, > 5, > 10, or > 20. In some implementations, three distinct positions of valve 652 are enumerated: (1) a retracted position, wherein the gas path valve seal 655 is fully lowered, and liquid path valve seal 657 is not abutted, such that gas path 613 is fully occluded, and liquid inlet 658 is fully open to allow liquid flow from exterior tube 614 to tube 615; (2) a middle position (shown in FIG. 5), wherein gas path valve seal 655 is partially raised to allow breathing gas flow from gas path 613, and liquid path valve 657 is not abutted to allow for liquid flow from external tube 614 out of liquid inlet 658 to internal tube 615; and (3) an extended position, wherein gas path valve seal 655 is fully extended to allow breathing gas flow from gas path 613 and to abut liquid path valve 657 such that it occludes liquid inlet 658, blocking liquid flow from external tube 614. These three positions are summarized in Table 3.

**Table 3:** Exemplary positions of an occluder valve for controlling gas and liquid flows into an auxiliary unit described herein.

| Valve Position | Gas Path | Liquid Path |
|---|---|---|
| Retracted | Closed | Open |
| Middle | Open | Open |
| Extended | Open | Closed |

The three positions described above may be used for certain purposes. For example, the middle position is used for refilling an internal reservoir of auxiliary unit 604 while breathing gas is delivered. The reservoir may be internal reservoir 532 of FIG. 5. The extended position may be used for isolating the internal reservoir to prevent overfilling or when system 600 is in a standby or low-power mode, as described herein. For example, standby mode may involve only providing breathing gas without vaporizing liquid into the breathing gas through a VTC. Standby mode may be initiated by the controller when a main battery of base unit 602 is removed and system 600 operates on power from a reserve battery in base unit 602. The retracted position may be used when auxiliary unit 604 is removed or detached from base unit 602. In this circumstance, the retracted position may be advantageous in order to protect the gas path 613 of base unit 602 from ingress (*e.g.,* of liquid, dust, particles, contaminants, cleaning solvents).

The controller may be configured to operate actuator 654 to alternate between the valve positions based on received signals. In some implementations, auxiliary unit 604 further comprises an internal reservoir (such as internal reservoir 532 of FIG. 5) for holding the liquid and a level sensor (such as level sensor 538 of FIG. 5) configured to measure the liquid level in the internal reservoir. The level sensor may transmit a signal to the controller indicating that the liquid level is substantially low, and the controller may respond to the signal by actuating valve 652 from the extended position to the middle position, allowing liquid flow into internal tube 615. A low liquid level may also be indicated to the controller by a temperature sensor (such as temperature sensor 542 of FIG. 5) that measures temperature of a heating plate immersed in liquid in auxiliary unit 604, such that a low liquid level is indicated by a rapid increase in the heating temperature when at least a portion of the heating plate is no longer immersed in liquid. In response to one or more signals from the temperature sensor, the controller may actuate valve 652 from the extended position to the middle position to allow liquid flow into internal tube 615 to replenish the internal reservoir and re-immerse the heating plate. In some implementations, auxiliary unit 604 comprises an alignment marker (such as alignment marker 548 of FIG. 5), and base unit 602 includes an alignment sensor (such as alignment sensor 549 of FIG. 5) configured to detect the presence of the alignment marker when auxiliary unit 604 is fully seated in base unit 602. The alignment sensor may transmit a first signal to the controller indicating that it has detected the presence of the alignment marker, indicating auxiliary unit 604 is fully seated, and the controller may respond to the first signal by actuating valve 652 from the retracted position to the middle position, allowing breathing gas and liquid flow into gas path 612 and internal tube 615, respectively, of auxiliary unit 604. The controller may alternatively respond to the first signal by actuating valve 652 from the retracted position to the extended position, for example, if the internal reservoir is full of liquid. The alignment sensor may transmit a second signal to the controller indicating that it no longer detects the presence of the alignment marker, indicating auxiliary unit 604 has been unseated or dislodged, and the controller may respond to the second signal by actuating valve 652 from the extended or middle positions to the retracted position. In some cases, valve 652 remains in the extended position for a fixed period of time or until an internal reservoir of auxiliary unit 604 is depleted of liquid, in order to measure the vaporization rate. Valve 652 may then be moved to the middle position to refill the reservoir via liquid flow path 615.

Flexible portion 656 separates the liquid flow path from the breathing gas flow path, and its flexibility allows for control of the liquid flowrate from the breathing gas side of flexible portion 656. Flexible portion 656 may be constructed from a material that is substantially flexible as to allow upward force from gas path valve seal 655 to vary the position of flexible portion 656 and liquid path valve seal 657. Suitable materials may include ethylene vinyl acetate, polyethylene, polyethylene based polyolefin elastomers, polypropylene, polyurethane, styrene butadiene copolymer, thermoplastic polyester elastomer, polypropylene based elastomers, thermoplastic polyurethane elastomer, polyvinylidene fluoride, fluorinated ethylene propylene, nylon, nylon blends, polystyrene, polyvinyl chloride, polytetrafluorethylene, and thermoplastic vulcanizate. In some implementations, gas seal 660 is formed of a flexible material, such that it may be compressed when auxiliary unit 604 is seated in base unit 602 to form a sealed annular space between gas paths 613 and 612, as shown in FIG. 6 where gas seal 660 forms an annular space around gas path valve seal 655. Gas seal 660 may be formed of the same material as flexible portion 656 or any of the materials listed above.

## Auxiliary Unit Design

In accordance with the implementations described above and in the following, an auxiliary unit for a respiratory therapy system is provided herein. FIGs. 7A-7F show multiple view angle and cross-sections of an auxiliary unit 704 , according to an illustrative implementation. Auxiliary unit 704 may be implemented, for example, in the respiratory therapy systems 400, 500, 600, and 800 of FIGs. 4, 5, 6, 8A, and 8B.

FIG. 7A shows auxiliary unit 704 having a pump 724, a gas seal 760, and an external tube connector 762, according to an illustrative implementation. A delivery tube 706 is connected to auxiliary unit 704, and a patient connector 708 is disposed at a distal end of delivery tube 706. Auxiliary unit 704 is configured to be seated in a base unit (not shown) of a respiratory therapy system which is configured to provide pressurized breathing gas from a blower in the base unit to auxiliary unit 704 during operation. Base unit 402 of FIG. 4 is an example of a base unit that may receive auxiliary unit 704. Gas seal 660, which is part of an occluder valve, forms a sealed flow path for the breathing gas to enter auxiliary unit 704 from the base unit when auxiliary unit 704 is seated in the base unit. External tube connector 762 is configured to connect an external tube (not shown) that provides liquid to auxiliary unit 704 from an external reservoir (not shown). For example, external tube 414 and external reservoir 410 of FIG. 4 may be used in this implementation. Pump 724 is configured to convey the liquid from within auxiliary unit 704.

Delivery tube 706 is configured to receive a flow of breathing gas from auxiliary unit 704 and convey the breathing gas to a patient, through patient connector 708 at the distal end of delivery tube 706. In some implementations, a nasal cannula is connected to patient connector 708 to provide the breathing gas into at least one nare of the patient. The breathing gas may be humidified with the liquid by a VTC in auxiliary unit 704. Pump 724 may convey the liquid along a flow path, including through the VTC in which at least a portion the liquid may be vaporized into the breathing gas which is simultaneously passed through the VTC. When auxiliary unit 704 is seated in the base unit, pump 724 may couple to a pump actuator in the base unit, and the pump actuator may be operatively coupled to a controller which may control the power supplied to pump 724. For example, pump 724 may include a rotor which is configured to magnetically couple to a stator in the base unit. Through the magnetic coupling, rotational motion of the rotor is generated and used to convey fluid along the flow path. In some implementations, the flow path includes a jacket of delivery tube 706.

FIG. 7B shows an alternate view angle of auxiliary unit 704. From this angle, a latch 720 is shown on the side of auxiliary unit 704, according to an illustrative implementation. Latch 720 locks auxiliary unit 704 into the base unit when auxiliary unit 704 is fully seated. Delivery tube 706 is connected to auxiliary unit outlet 772.

Latch 720 allows auxiliary unit 704 to be locked and unlocked from its fully seated position in the base unit. Latch 720 may enable locking of auxiliary unit 704 by having a ridge that resides in a notch in the base unit. A user or operator may press against latch 720 to release latch 720 from the notch and unlock auxiliary unit 704 when removing it from the base unit. Latch 720 may have one or more components embedded in it. For example, a marker, such as alignment marker 548 of FIG. 5, may be embedded in latch 720, and the base unit may include a sensor, such as alignment sensor 549 of FIG. 5, configured to detect the presence of the marker when auxiliary unit 704 is fully seated in the base unit and latch 720 is locked. In some implementations, the marker is a magnet, and the sensor is a Hall effect sensor configured to detect the magnetic field of the magnet. In some implementations, latch 720 includes an RFID tag, such as RFID tag 550 of FIG. 5, and the base unit includes an RFID reader, such as RFID reader 551 of FIG. 5, configured to detect the presence of the RFID tag and read information stored on the RFID tag. The information on the RFID tag may identify auxiliary unit 704 as having certain functionalities, may provide use history of auxiliary unit 704, or may provide recommended system parameters such as flowrates, humidity level, and temperature. A barcode may be used in auxiliary unit 704 in addition to or in place of an RFID tag or other tag, or magnet, such that a reader in the base unit reads the barcode when auxiliary unit 704 is fully docked in the recess of the base unit. The barcode may include the information about one or more components, as is described in relation to the tags in this disclosure. As another option, the base unit includes an infrared (IR) proximity sensor having an IR beam positioned such that latch 720 breaks the beam, triggering the sensor, when auxiliary unit 704 is fully seated. Other options for auxiliary unit presence sensing include quick response (QR) codes, global positioning system (GPS) chips, Bluetooth®, Bluetooth® low-energy, near-field communication (NFC), and pattern recognition matching.

Auxiliary unit outlet 772 functions as a port for connection of delivery tube 706 to auxiliary unit 704 for transmission of the breathing gas. Outlet 772 may have a shape such that delivery tube 706 can bend and rotate at outlet 772 without being kinked by the edges of outlet 772. The shape of outlet 772 may also prevent dislodging of delivery tube 706 from auxiliary unit 704. In some implementations, outlet 772 is cone shaped. In some implementations, outlet 772 is chamfered. In some implementations, outlet 772 is filleted. Outlet 772 may be configured to have no hard edges that may kink or damage delivery tube 706; for example, outlet 772 may be rounded.

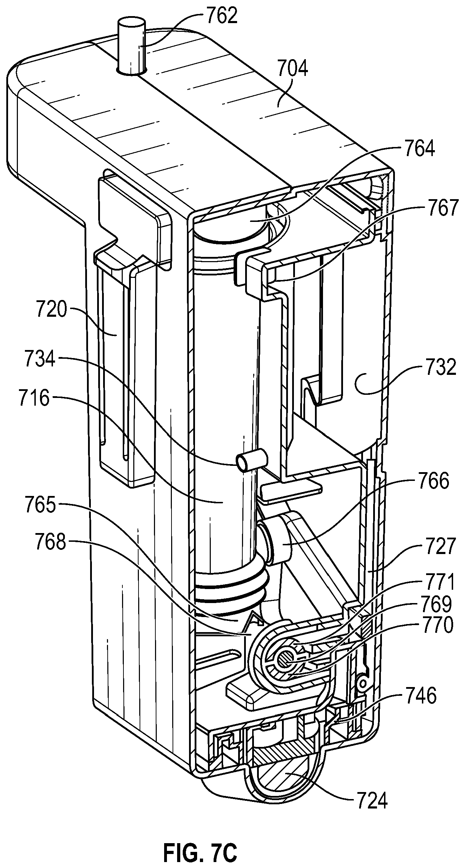

FIG. 7C shows a cross-section of auxiliary unit 704 which includes, in addition to the components previously described, a VTC 716, a heating plate 727, an internal reservoir 732, and a delivery connector 768, according to an illustrative implementation. Liquid is provided to internal reservoir 732 at reservoir inlet 734 which receives liquid from an internal tube (not shown), for example, internal tubes 515 and 615 of FIGs. 5 and 6. Liquid flows out from internal reservoir 732 past heating plate 727 which is immersed in the liquid during operation. Liquid is drawn into pump 724 which expels liquid through pump outlet 746. VTC 716 has a first cap 764 configured to receive breathing gas and a second cap 765 configured to output breathing gas to delivery connector 768. VTC 716 also includes VTC liquid inlet 766 and VTC liquid outlet 767. Delivery connector 768 is configured to convey breathing gas from second cap 765 of VTC 716 to delivery tube 706 through connector gas outlet 769. Delivery connector 768 further includes connector liquid outlet 770 and connector liquid inlet 771.

Liquid entering auxiliary unit 704 through liquid tube connector 762 is directed to reservoir inlet 734 by an internal tube (not shown), such as internal tube 515 of FIG. 5. Liquid stored in internal reservoir 732 may flow out of internal reservoir 732 to a heating section where heating plate 727 is immersed in liquid. In some implementations, liquid flows into the heating section due to hydrostatic pressure and/or gravitational flow, a result of internal reservoir 732 being positioned above the heating section. In some implementations, pump 724 generates a suction pressure that draws liquid out of internal reservoir 732 and into the heating section before being drawn into pump 724. When auxiliary unit 704 is seated in the base unit, heating plate 727 may be coupled to a heat actuator in the base unit. In some implementations, the heat actuator is a coil configured to generate a current in the heating plate via induction, and the current produces heat due to a resistance in the heating plate.

Liquid flows or is drawn into pump 724 from the heating section. The pump expels liquid out of pump outlet 746 which is in fluid communication with connector liquid outlet 770 of delivery connector 768. Delivery connector 768 may be an overmolded rubber piece having one or more lumens. Connector liquid inlet 770 conveys pumped, heated liquid from pump outlet 746 into a jacket of delivery tube 706. The jacket concentrically surrounds an inner gas conduit through which breathing gas is conveyed, such that the heated liquid flowing through the jacket insulates the breathing gas in the inner gas conduit. This feature may be advantageous as to maintain a sufficiently high breathing gas temperature in order to prevent rain-out of vapor in the breathing gas. Rain-out occurs when vapor in the breathing gas condenses into its liquid form during gas delivery. This condensation can cause obstruction of delivery tube 706, patient connector 708, or a nasal cannula connected to patient connector 708. The heated liquid travels in two directions in the jacket, and the jacket accordingly includes at least two lumens or channels, where each lumen or channel connects to either connector liquid outlet 770 or connector liquid inlet 771. For example, liquid is pumped through connector liquid outlet 770 into a first jacket channel which conveys the liquid along delivery tube 706 in a first direction towards patient connector 708. At the distal end of delivery tube 706, where patient connector 708 is disposed, the liquid may change directions and enter a second channel which conveys the liquid along delivery tube 706 in a second direction which is substantially opposite of the first direction. The second channel conveys the liquid into connector liquid inlet 771 of delivery connector 768.

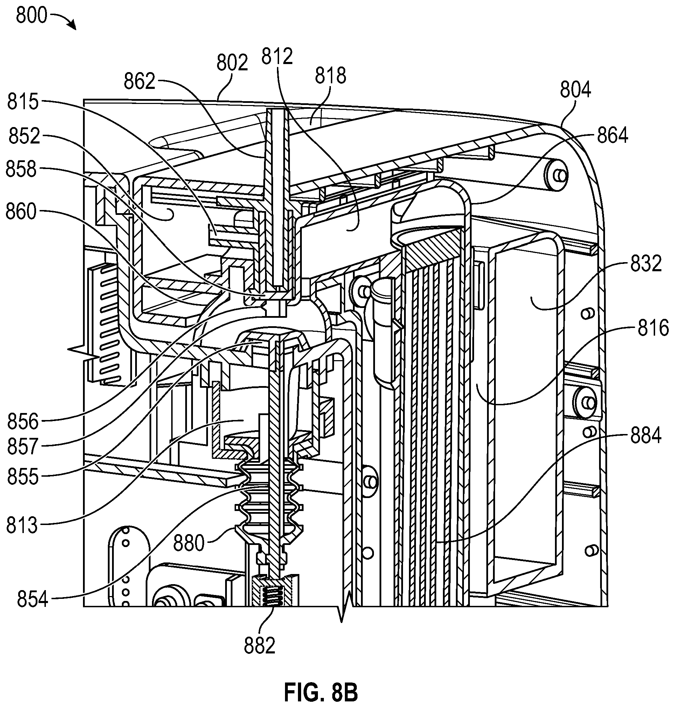

Connector liquid inlet 771 is in fluid communication with VTC liquid inlet 766 in order to convey the fluid from the jacket to VTC 716. Within VTC 716, liquid may be vaporized into the breathing gas conveyed into VTC 716 through first cap 764. For example, the liquid may be water, and VTC 716 may output from second cap 765 a flow of humidified breathing gas containing water vapor. For vaporization to occur, the temperature in VTC 716 may be at or near the gas therapy temperature. Heating plate 727 may heat the liquid to a sufficiently high temperature, e.g., about 100 °F. VTC 716 may contain a plurality of fibers, such as the permeable fibers or semi-permeable fibers 884 of FIG. 8B, which allow vapor to pass from the liquid flow into the breathing gas flow. Liquid that is not vaporized in VTC 716 may flow out of VTC liquid outlet 767 which returns the liquid to internal reservoir 732.

Breathing gas exiting second cap 765 of VTC 716 is directed through connector gas outlet 769 of delivery connector 768 into delivery tube 706. The breathing gas, which may be humidified or contain vapor, flows through the inner gas conduit of delivery tube 706 and is insulated or heated by liquid flowing through the jacket. At patient connector 708, the breathing gas may be directed directly to the patient or into one or more additional devices, such as a face mask or a nasal cannula. Suitable nasal cannulas are described above and specifically in relation to FIG. 1.

FIG. 7D is cross-sectional view of auxiliary unit 704, according to an illustrative implementation. In this view, an occluder valve 752 is shown in auxiliary unit 704. Delivery tube 706 is connected to delivery connector 768 at auxiliary unit outlet 772. Occluder valve 752 receives liquid flow through external tube connector 762. A flexible portion 756 includes a liquid path valve seal 757 disposed below valve liquid inlet 658. Internal tube 715 is in fluid communication with valve 752 and internal reservoir 732. Gas path 712 is in fluid communication with an annular space formed by gas seal 660 and with first cap 764 of VTC 716.

Occluder valve 752 may be occluder valve 652 of FIG. 6. Occluder valve 752 is configured to mate with the base unit when auxiliary unit 704 is fully seated. As described in relation to FIG. 6, the base unit may include a controller, an actuator, and a gas path valve seal. The controller operates the actuator to adjust a position of the gas path valve seal in order to control breathing gas flow from the base unit into auxiliary unit 704. The gas path valve seal may be raised against the liquid path valve seal 757 in order to simultaneously control liquid flowrate through occluder valve 752, because liquid path valve seal 757 is a rigid piece joined to flexible portion which may be flexed to raise liquid path valve seal 757 to narrow a gap between liquid path valve seal 757 and valve liquid inlet 758. The actuator, gas path valve seal, and liquid path valve seal may have, but are not limited to, three positions which are summarized in Table 3. The controller may operate the actuator to switch between each of these positions based on system inputs or received signals, as described in relation to FIG. 6. Breathing gas may be directed by valve 752 into gas path 712 which conveys breathing gas into first cap 764 of VTC 716. Liquid may be directed by valve 752 into internal tube 715 which conveys liquid into internal reservoir 732.

Delivery tube 706 is connected to delivery connector 768 at auxiliary unit outlet 772 for receiving breathing gas and liquid flows. Delivery tube 706 includes an inner gas conduit 707 configured to receive breathing gas from second cap 765 of VTC 716 through connector gas outlet 769 of delivery connector 768. Delivery tube 706 includes a first jacket channel 774 configured to receive liquid from pump outlet 764 through connector liquid outlet 770 of delivery connector 768. Liquid flows through first jacket channel 774 and a second jacket channel 775, for example, to heat or insulate the breathing gas in inner gas conduit 707. In some implementations, first jacket channel 774 conveys liquid in a first direction away from auxiliary unit outlet 772 and towards patient connector 708. First and second jacket channels 774 and 775 each have distal ends disposed within patient connector 708 which may allow for fluid communication between first and second jacket channels 774 and 775. For example, the liquid flows in the first direction through first jacket channel 774, and, upon reaching patient connector 708, flows out of the distal end of first jacket channel 774 and into the distal end of second jacket channel 775. Liquid may then flow through second jacket channel 775 in a second direction which may be substantially opposite of the first direction. Second jacket channel 775 conveys the liquid back into delivery connector 768 through connector liquid inlet 771. In some implementations, first jacket channel 774 covers a first portion of inner gas conduit 707, the first portion having an approximately semi-circular cross-sectional shape. Second jacket channel 775 may cover a second portion on the opposite side of inner gas conduit 707, the second portion having an approximately semi-circular cross-sectional shape. Auxiliary outlet 772 in this implementation has a rounded (filleted) shape which may prevent kinking of delivery tube 706 and occlusion of inner gas conduit 707, first jacket channel 774, and second jacket channel 775 when delivery tube 706 is bended or flexed.

In some implementations, one or more gas temperature sensors (not shown) are positioned at the inlet to the delivery tube 706 (near delivery connector 768), at the distal end of delivery tube 706, and/or at patient connector 708. For example, a temperature humidity sensor may be placed at the end of delivery tube 706. The gas temperature sensor(s) may have a wireless link to a controller in the base unit for transmission of temperature data without an electrical conductor running along the delivery tube 706. Temperature of the breathing gas at these points can be transmitted to a controller for controlling VTC 716 or the blower for feedback control of the breathing gas temperature. In some implementations, one or more liquid temperature sensors (not shown) are positioned along the delivery tube 706 for monitoring of the liquid temperature in the jacket. An ambient temperature sensor or ambient humidity sensor (*e.g.,* at the air intake of the base unit) may be employed to enable environmentally-conditioned control of the breathing gas along its flow path.

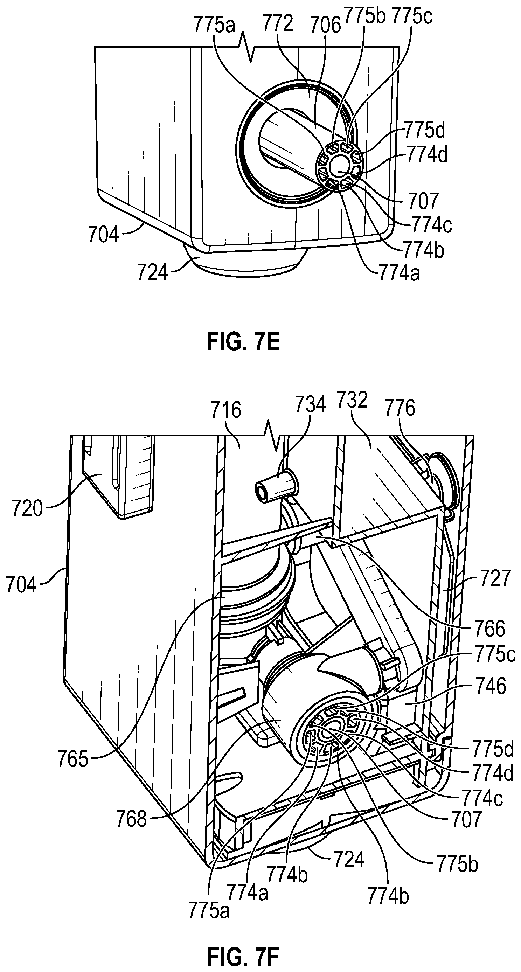

FIGs. 7E and 7F show different cross-sections of a lower portion of auxiliary unit 704, according to an illustrative implementation. In FIG. 7E, delivery tube 706 is connected to auxiliary unit 704 at auxiliary outlet 772, and a cross-section of delivery tube 706 is shown. First jacket channel 774 includes four sub-channels 774a, 774b, 774c, and 774d. Second jacket channel 775 includes four sub-channels 775a, 775b, 775c, and 775d. The plurality of sub-channels surround inner gas conduit 707.

First jacket channel 774 and second jacket channel 775 are divided into sub-channels 774a-d and 775a-d by a plurality of radial ribs. Each radial rib extends from an inner wall defining inner gas conduit 707 to an outer wall of delivery tube 706, wherein the annular space between the inner wall and outer wall defines the jacket. Each pair of radial ribs defines one sub-channel of the jacket through which liquid flows. The radial ribs allow bending of delivery tube 706 in any direction without kinking by preventing collapse of inner gas conduit 707 when delivery tube 706 is bent. In some implementations, under flexure of delivery tube 706, one or more of the sub-channels 774a-d and 775a-d may collapse or become occluded, but at least some of the sub-channels 774a-d and 775a-d and inner gas conduit 707 remain open to allow for liquid and breathing gas flow, respectively. The jacket having radial ribs may mimic a thick-walled tube to prevent collapse of inner gas conduit 707. In some implementations, the inner wall defining inner gas conduit 707 has a thickness proportional to an internal diameter of inner gas conduit 707. In some implementations, the outer wall, defining the jacket, has a thickness proportional to the overall diameter of delivery tube 706 minus the internal diameter of inner gas conduit 707. In some implementations, each radial rib has a width proportional to the inner wall thickness.

In FIG. 7F, a cross-section of auxiliary unit 704 shows delivery tube 706 disposed in delivery connector 768. Sub-channels 774a, 774b, 774c, and 774d of first jacket channel 774 of delivery tube 706 are in fluid communication with connector liquid outlet 770 of delivery connector 768. Sub-channels 775a, 775b, 775c, and 775d of second jacket channel 775 of delivery tube 706 are in fluid communication with connector liquid inlet 771 of delivery connector 768. A tab 776 disposed at the top of heating plate 727.

As described above, first jacket channel 774 is in fluid communication with connector liquid outlet 770 in order to receive liquid from pump outlet 746. Liquid is flowed or pumped through the sub-channels 774a-d and then through sub-channels 775a-d of second jacket channel 775. Inner gas conduit 707 is in fluid communication with connector gas outlet 769 to receive breathing gas from VTC second cap 765.

Tab 776 is disposed at the top of heating plate 727, near internal reservoir 732. In some implementations, tab 776 is disposed at the top of heating plate 727 such that, when liquid in internal reservoir 732 reaches a low enough level, tab 776 is exposed while the rest of heating plate 727 remains immersed in liquid. When auxiliary unit 704 is fully seated in the base unit, a temperature sensor (such as temperature sensor 542 of FIG. 5) may be positioned such that it aligns with tab 776 and can measure the temperature of tab 776. When tab 776 is not immersed in liquid, it will heat up quickly if the heating plate and heating element are in operation. The temperature of the tab may be monitored by sending one or more signals indicating the tab temperature to the controller.. When the tab heats up quickly during a low liquid level state of auxiliary unit 704, the temperature sensor can detect the rapid rise in temperature and alert the controller. In response to detecting a rapid rise in the tab temperature indicative of a low liquid level, the controller may be configured to turn off the heat actuator (e.g., an induction coil). In response to detecting a rapid rise in the tab temperature indicative of a low liquid level, the controller may be configured to increase or open the liquid flow from the external reservoir by, for example, actuating occluder valve 752 from the extended position to the middle position to allow liquid flow to refill internal reservoir 732. In response to detecting a rapid rise in the tab temperature indicative of a low liquid level, the controller may be configured to completely shut off the respiratory therapy system. In some implementations, the base unit includes a spring element that may be actuated by the controller to auto-eject auxiliary unit 704 under certain conditions, for example, when a rapid temperature rise is indicated.

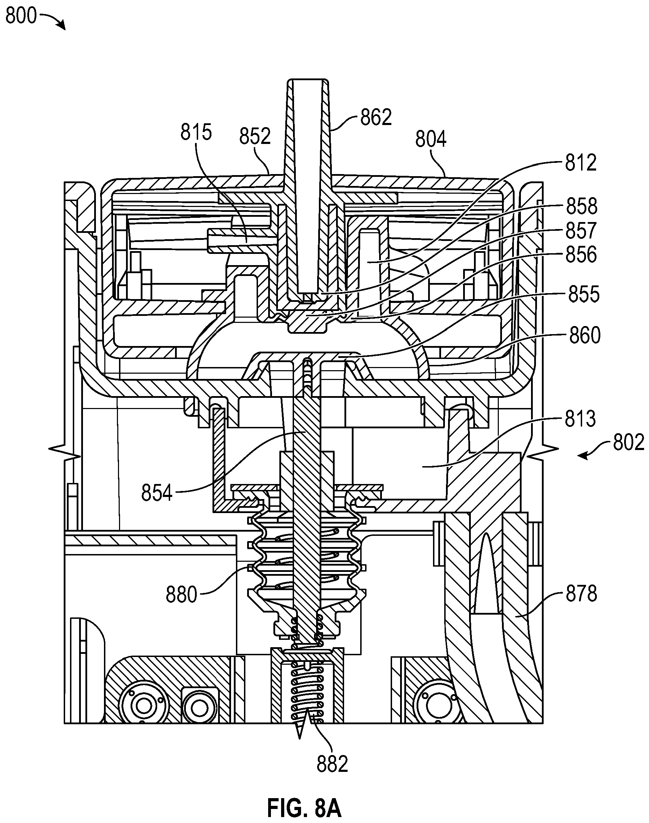

In FIG. 7D, valve 752 is shown separate from the corresponding components in the base unit. Actuation of valve 752 is performed by the controller of the base unit. Accordingly, FIGs. 8A and 8B depict system 800 in which valve 852, which may be analogous to valve 752 of FIG. 7D, is in a coupled state when auxiliary unit 804 is seated in recess 818 of base unit 802.

FIG. 8A depicts a cross-section of system 800 which includes an auxiliary unit 804 seated in base unit 802, according to an illustrative implementation. Valve 852 allows simultaneous control of breathing gas from gas path 813 of base unit 802 to gas path 812 of auxiliary unit 804 and of liquid from tube connector 862 to internal tube 815. Breathing gas is provided to gas path 813 from a blower (not shown) via blower tube 878. Valve 852 includes a rod 854 having a variable position controlled by actuator 882. Valve 852 also includes a gas path valve seal 855 and a liquid path valve seal 857 affixed to flexible portion 856. A gas seal 860 defines an annular space around gas path valve seal 855. A rod 854 extends through a bellows 880. Liquid flowing from tube connector 862 flows into valve 852 from valve liquid inlet 858.

Actuator 882 is coupled to a controller (not shown) in base unit 802. Actuator 882 may be configured to move rod 854 in a linear direction, for example, by pushing up against a lower end of rod 854. In some implementations, rod 854 is not affixed to actuator 882. For example, rod 854 may be affixed to an inner surface of bellows 880, and actuator 854 is configured to push against an outer surface of bellows 880, which is flexible, to move rod 854. The controller may send one or more signals to actuator 882 in order to adjust the position of rod 854. Gas path valve seal 855 is coupled to an end of rod 854, such that linear movement of rod 854 would also move gas path valve seal 855 in a linear direction, allowing gas path valve seal 855 to be positioned in a plurality of positions along the linear direction. Gas path valve seal 855 as shown in FIG. 8 is in a retracted position which blocks breathing gas from flowing from gas path 813 into the annular space defined by gas seal 860 in valve 852. Rod 854 is affixed to bellows 880, such that bellows 880 is expanded and contracted as rod 854 is lowered and raised, respectively, by virtue of flexibility of bellows 880.

As both the position of gas path valve seal 855 and the volume of bellows 880 may be varied, the flowrate of breathing gas from gas path 813 may be controlled by adjusting the position gas path valve seal 855 and volume of bellows 880 by actuator 854. From the retracted position of gas path valve seal 855 as shown in FIG. 8A, actuator 854 may be configured to raise gas path valve seal 855 to a middle position to open a gap underneath gas path valve seal 855 and allow breathing gas flow into valve 852. Actuator 854 may be configured to further raise gas path valve seal 855 to an extended position to enlarge the gap underneath gas path valve 855 and increase the breathing gas flowrate into valve 852.

Flexible portion 856 may similarly have a variable position by virtue of its flexibility. Flexible portion 856 and liquid path valve seal 857 may be moved in a linear direction. Liquid path valve seal 857 may be formed integrally in flexible portion 856 or disposed in flexible portion 856. Liquid path valve seal 857 may be rigid relative to flexible portion 856, for example, by having a greater thickness than portion 856 or having a reinforced structure. Gas path valve seal 855 may be raised by actuator 854 from its position shown in FIG. 8A such that gas path valve seal 855 abuts liquid path valve seal 857. Accordingly, controlling actuator 854 may allow for adjustment of the position of liquid path valve seal 857 by pushing up against it with gas path valve seal 855. Adjusting the position of liquid path valve seal 857 either narrows or enlarges a gap between liquid inlet 858 and liquid path valve seal 857, wherein liquid from an external tube connected to tube connector 862 flows through the gap when entering valve 852 on its path into auxiliary unit 804. Narrowing and enlarging the gap may decrease and increase, respectively, the flowrate of liquid out of liquid inlet 858.

Accordingly, valve 852 may provide simultaneous control of both breathing gas and liquid flowrates into auxiliary unit 804, similar to valve 652 of FIG. 6. In some implementations, linear actuation of actuator 854 allows for a non-discrete positioning of valve seals 855 and 857. In some implementations, the controller is configured to control actuator 854 between a discrete set of positions. In some implementations, the set includes a number of positions > 1, > 2, > 5, > 10, or > 20. In some implementations, three distinct positions of valve 852 are enumerated: (1) a retracted position (shown in FIG. 8A), wherein the gas path valve seal 855 is fully lowered, and liquid path valve seal 857 is not abutted, such that gas path 813 is fully occluded, and liquid inlet 858 is fully open to allow liquid flow to internal tube 815; (2) a middle position, wherein gas path valve seal 855 is partially raised to allow breathing gas flow from gas path 813, and liquid path valve 857 is not abutted to allow for liquid flow out of liquid inlet 858 to internal tube 815; and (3) an extended position, wherein gas path valve seal 855 is fully extended to allow breathing gas flow from gas path 813 and to abut liquid path valve 857 such that it occludes liquid inlet 858, blocking liquid flow. These three positions are summarized in Table 3 above.

The three positions described above may be used for certain purposes. For example, the middle position is used for refilling an internal reservoir of auxiliary unit 804 while breathing gas is delivered. The reservoir may be internal reservoirs 532 and 732 of FIGs. 5, 7C, 7D, and 7F. The extended position may be used for isolating the internal reservoir to prevent overfilling or when system 800 is in a standby or low-power mode, as described herein. For example, standby mode may involve only providing breathing gas without vaporizing liquid into the breathing gas through a VTC. Standby mode may be initiated by the controller when a main battery of base unit 802 is removed and system 800 operates on power from a reserve battery in base unit 802. The retracted position may be used when auxiliary unit 804 is removed or detached from base unit 802. In this circumstance, the retracted position may be advantageous in order to protect the gas path 813 of base unit 802 from ingress (e.g., of liquid, dust, particles, contaminants), such that the blower and blower tube 878 are not contaminated.

The controller may be configured to operate actuator 854 to alternate between the valve positions based on received signals. In some implementations, auxiliary unit 804 further comprises an internal reservoir (such as internal reservoirs 532 and 732 of FIGs. 5, 7C, 7D, and 7F) for holding the liquid and a level sensor (such as level sensor 538 of FIG. 5) configured to measure the liquid level in the internal reservoir. The level sensor may transmit a signal to the controller indicating that the liquid level is substantially low, and the controller may respond to the signal by actuating valve 852 from the extended position to the middle position, allowing liquid flow into internal tube 815. A low liquid level may also be indicated to the controller by a temperature sensor (such as temperature sensor 542 of FIG. 5) that measures temperature of a heating plate immersed in liquid in auxiliary unit 804, such that a low liquid level is indicated by a rapid increase in the heating temperature when at least a portion of the heating plate is no longer immersed in liquid, for example, when tab 776 of heating plate 727 of FIG. 7 is exposed. In response to one or more signals from the temperature sensor, the controller may actuate valve 852 from the extended position to the middle position to allow liquid flow into internal tube 815 to replenish the internal reservoir and re-immerse the heating plate. In some implementations, auxiliary unit 804 comprises an alignment marker (such as alignment marker 548 of FIG. 5), and base unit 802 includes an alignment sensor (such as alignment sensor 549 of FIG. 5) configured to detect the presence of the alignment marker when auxiliary unit 604 is fully seated in base unit 802. The alignment sensor may transmit a first signal to the controller indicating that it has detected the presence of the alignment marker, indicating auxiliary unit 804 is fully seated, and the controller may respond to the first signal by actuating valve 852 from the retracted position to the middle position, allowing breathing gas and liquid flow into gas path 812 and internal tube 815, respectively, of auxiliary unit 804. The controller may alternatively respond to the first signal by actuating valve 852 from the retracted position to the extended position, for example, if the internal reservoir is full of liquid. The alignment sensor may transmit a second signal to the controller indicating that it no longer detects the presence of the alignment marker, indicating auxiliary unit 804 has been unseated or dislodged, and the controller may respond to the second signal by actuating valve 852 from the extended or middle positions to the retracted position.

Flexible portion 856 separates the liquid flow path from the breathing gas flow path, and its flexibility allows for control of the liquid flowrate from the breathing gas side of flexible portion 856. Flexible portion 856 may be constructed from a material that is substantially flexible as to allow upward force from gas path valve seal 855 to vary the position of flexible portion 856 and liquid path valve seal 857. Suitable materials may include ethylene vinyl acetate, polyethylene, polyethylene based polyolefin elastomers, polypropylene, polyurethane, styrene butadiene copolymer, thermoplastic polyester elastomer, polypropylene based elastomers, thermoplastic polyurethane elastomer, polyvinylidene fluoride, fluorinated ethylene propylene, nylon, nylon blends, polystyrene, polyvinyl chloride, polytetrafluorethylene, and thermoplastic vulcanizate. In some implementations, gas seal 860 is formed of a flexible material, such that it may be compressed when auxiliary unit 804 is seated in base unit 802 to form a sealed annular space between gas paths 813 and 812, as shown in FIG. 8A where gas seal 860 forms an annular space around gas path valve seal 855. Gas seal 860 may be formed of the same material as flexible portion 856 or any of the materials listed above.

FIG. 8B shows another angle of system 800, depicting a cross-section of auxiliary unit 704 which includes a VTC 816 and an internal reservoir 832, according to an illustrative implementation. Auxiliary unit 804 is fully seated in recess 818 of base unit 802. VTC 816 includes a cap 864 which directs breathing gas from gas path 812 to a plurality of fibers 884.

Internal reservoir 832 stores liquid in auxiliary unit 804. Internal tube 815 directs liquid from valve 852 to internal reservoir 832. Liquid is conveyed into VTC 816 in which the liquid may be vaporized into the breathing gas directed through cap 864. For example, the liquid may be water which is vaporized to create a humidified breathing gas. VTC 816 contains the plurality of fibers 884 which allow vapor to pass from the liquid flow into the breathing gas flow. The fibers may be permeable or semi-permeable as to only allow vapor to pass through and to keep liquid separate from the breathing gas. Liquid that is not vaporized in VTC 816 may flow out of a VTC liquid outlet which returns the liquid to internal reservoir 832.

The liquid in auxiliary unit 804 conveyed into VTC 816 must be heated to at or near a boiling point temperature of the liquid in order for at least a portion of the liquid to be vaporized. FIG. 9 shows a heating section of an auxiliary unit 904, the heating section including a heating plate 927 configured to be immersed in liquid and heat the surrounding liquid, according to an illustrative implementation. Liquid is stored in internal reservoir 932, to which liquid is supplied from an external reservoir via reservoir inlet 934. Heating plate 927 includes a protruding tab 976.

In accordance with the implementations previously described, auxiliary unit 904 may be seated in a base unit to form a respiratory therapy system. Heating plate 927 may be configured to couple to a heating element in the base unit when auxiliary unit 904 is seated. In some implementations, the heating element produces a current in heating plate 927 via induction. Induction heating allows the targeted heating of heating plate 927 without contact between the heating element and heating plate 927. The heating element may be a coil. Alternating electrical current, supplied from a power supply of the base unit, may be passed through the coil, creating a variable magnetic field. The coil is formed of a conductive material, such as copper or silver. In this implementation, heating plate 927 is formed of a conductive material. When auxiliary 904 is seated, heating plate 927 is positioned in the magnetic field which induces eddy currents in heating plate 927. The induced eddy currents flow against an electrical resistivity of heating plate 927, generating localized heat in heating plate 927 without any contact between heating plate 927 and the coil. Additional heat may be produced in heating plate 927 in implementations where heating plate 927 is formed of a magnetic material. The additional heat is produced via hysteresis which involves internal friction created when magnetic heating plate 927 is within the magnetic field of the coil, the internal friction producing the additional heat. Suitable materials for heating plate 927 include, but are not limited to, steel, iron, copper, aluminum, lead, silver, tin, and alloys of the preceding.

Tab 976 is disposed at the top of heating plate 927, near internal reservoir 932. In some implementations, tab 976 is disposed at the top of heating plate 927 such that, when liquid in internal reservoir 932 reaches a low enough level, tab 976 is exposed while the rest of heating plate 927 remains immersed in liquid. When auxiliary unit 904 is fully seated in the base unit, a temperature sensor (such as temperature sensor 542 of FIG. 5) may be positioned such that it aligns with tab 976 and can measure the temperature of tab 976. When tab 976 is not immersed in liquid, it will heat up quickly if the heating plate and heating element are in operation. The temperature of the tab may be monitored by sending one or more signals indicating the tab temperature to the controller.. When the tab heats up quickly during a low liquid level state of auxiliary unit 904, the temperature sensor can detect the rapid rise in temperature and alert the controller. In response to detecting a rapid rise in the tab temperature indicative of a low liquid level, the controller may be configured to turn off the heat actuator (*e.g.,* an induction coil). In response to detecting a rapid rise in the tab temperature indicative of a low liquid level, the controller may be configured to increase or open the liquid flow from the external reservoir by, for example, actuating occluder valve 952 from the extended position to the middle position to allow liquid flow to refill internal reservoir 932. In response to detecting a rapid rise in the tab temperature indicative of a low liquid level, the controller may be configured to completely shut off the respiratory therapy system. In some implementations, the base unit includes a spring element that may be actuated by the controller to auto-eject auxiliary unit 904 under certain conditions, for example, when a rapid temperature rise is indicated.

Breathing Gas Measurement and Supplementation

It can be advantageous to measure, monitor, and manipulate breathing gas flow in a base unit described herein. FIG. 10 shows a measurement device 1086 of a base unit 1002, according to an illustrative implementation. Measurement device 1086 is in fluid communication with a gas inlet 1088 and with a blower 1003 of base unit 1002. Measurement device 1086 includes a conduit 1090, a first flow sensor 1092, and first segment 1093, a second flow sensor 1094, and a second flow segment 1095.

Measurement device 1086 may be implemented in any of the base units described herein, including base units 402, 502, 602, and 802 of FIGs. 4, 5, 6, 8A, and 8B. Gas inlet 1088 allows breathing gas to enter base unit 1002 from an external source. In some implementations, gas inlet 1088 draws ambient air from the surroundings. In some implementations, gas inlet 1088 is configured to be coupled to a wall air outlet. Gas inlet 1088 is in fluid communication with blower 1003 via conduit 1090. In some implementations, blower 1003 generates a suction pressure that draws gas into gas inlet 1088 and through conduit 1090.

Gas inlet 1088 directs breathing gas into conduit 1090. First flow sensor 1092 and second flow sensor 1094 are disposed along conduit 1090 and are configured to measure a flowrate of the breathing gas travelling through conduit 1090. First flow sensor 1092 and second flow sensor 1094 may be configured to output one or more signals to a controller in base unit 1002, the signals being indicative of the measured flowrates. The controller is operatively coupled to blower 1003 and may be configured to adjust one or more parameters of blower 1003 based on the received signals indicative of measured flowrates. Appropriate parameters to be adjusted include suction pressure, output pressure, and output flowrate.

Blower 1003 may output breathing gas at a high temperature, so a heat exchanger may be disposed at the output of blower 1003. The heat exchanger may be fitted around a tube such that heat disperses from the breathing gas radially outwards into the heat exchanger from the breathing gas. The heat exchanger may be configured to lower the breathing gas temperature to a target temperature, for example, a maximum temperature that prevents malfunction of certain components that come into contact with the breathing gas. For example, a VTC may contain fibers that would be damaged by excessively hot breathing gas. Temperature sensors may be positioned along the flow path before and after the heat exchanger in order to ensure is not too hot before entering other components such as the VTC. Measurements from the sensors may be used to adjust operation of blower 1003 (*e.g.,* by limiting RPM of blower 1003 or shutting it down) or adjust operation of the heat exchanger (*e.g.*, adjusting cooling fluid flow rate through the heat exchanger or temperature of the cooling fluid).

In some implementations, the heat exchanger is an air-to-air heat exchanger with a metal finned extrusion having an interior pathway, for breathing gas flow, and an exterior pathway, for cooling air flow. The resulting exchange of thermal energy between the hot breathing gas and the cooling air flow results in a cooled breathing gas flow. In some implementations, the heat exchanger comprises a countercurrent portion and a concurrent portion. In the countercurrent portion, hot breathing gas flows through the interior pathway in a direction opposite a direction that cooling air flows through the exterior pathway. In the concurrent portion, cooler breathing gas flows through the interior pathway in a direction parallel to a direction that cooling air flows through the exterior pathway, further cooling the breathing gas. The cooling air may be conveyed through the exterior pathway by a heat exchanger fan. This configuration results in a more compact footprint of the heat exchanger, where both the input and output of breathing gas are on one side. The heat exchanger may have an efficiency between 80 and 90%. For example, the heat exchanger efficiency is 88%.

In some implementations, the system comprises a gas temperature sensor for monitoring the temperature of the breathing gas at some point in system. For example, if the system 100 comprises a humidifier as discussed above, then a gas temperature sensor may be positioned at the inlet of the humidifier, such that the temperature of gas entering the humidifier can be monitored. As a fan is used to move cooling air in the heat exchanger, the fan can be controlled in order to adjust the rate of thermal energy transfer and regulate the temperature of the breathing gas output to the humidifier. When the temperature of the breathing gas is known at the humidifier inlet, the fan can be lower or even turned off when the temperature is low enough to not damage the fiber. Similarly, the blower fan can be adjusted. Minimizing fan usage is advantageous in that it decreases the sound generation of the system.

In some implementations, first flow sensor 1092 and second flow sensor 1094 are mass flow sensors configured to output one or more signals indicative of measured mass flowrates of the breathing gas. Conduit 1090 further includes first segment 1093 and second segment 1095. First segment 1093 is disposed adjacent to first flow sensor 1092, and second segment 1095 is disposed adjacent to second flow sensor 1094. These components are positioned such that breathing gas flowing through conduit 1090 flows through, in order from first to last, first segment 1093, first flow sensor 1092, second segment 1095, and second flow sensor 1094. In some implementations, first segment 1093 and second segment 1095 may be configured to be approximately straight. By straightening first and second segments 1093 and 1095, the breathing gas flows entering first flow sensor 1092 and second flow sensor 1094 are minimized of disturbances in the flow, such that the flow profiles are approximately uniform. For example, first and second flow sensors 1092 and 1094 may provide the most accurate or precise measurements of breathing gas flowrate when the flow profiles of breathing gas entering each sensor are approximately uniform. In some implementations, conduit 1090 also includes straight segments directly after first and second flow sensors 1092 and 1094 to minimize downstream effects of the flow on the measurements. However, it should be understood that segments 1093 and 1095 are optional and not required for accurate flow measurement.

In some implementations, first flow sensor 1092 and second flow sensor 1094 are configured to each be calibrated relative to the other. When no supplementary gas is being introduced into the gas flow, the flow rate through the first and second flow sensors is identical. The first and second sensors may indicate different flow rates, however due to manufacturing variability, drift over time, or other factors that introduce error to flow sensors. By using first sensor as a reference point and offsetting the indicated flow of the second sensor, the difference between sensors may be reduced to zero. When a supplemental gas is introduced, the flow rate of supplemental gas is calculated as the difference between the flow indicated on the first and second flow sensors. The error in the calculation is thereby reduced to the error of the second sensor.

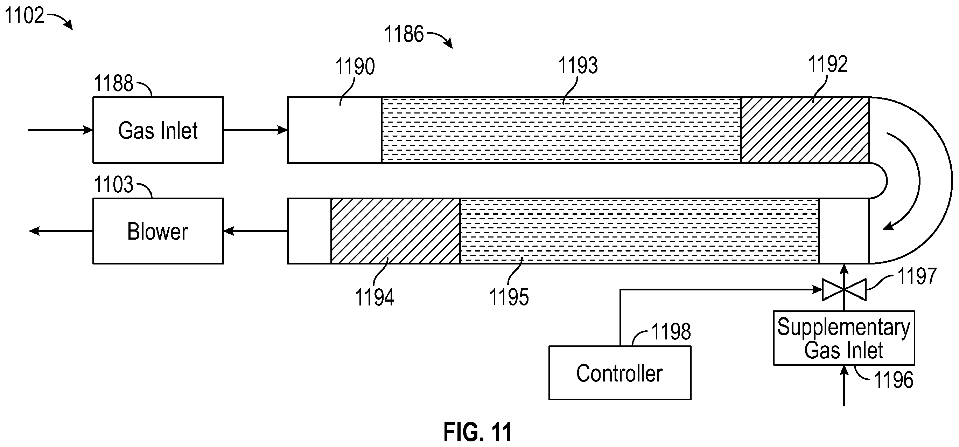

FIG. 11 shows a measurement device 1186 of a base unit 1102, according to an illustrative implementation. Measurement device 1186 includes a conduit 1190 coupled to a gas inlet 1188 and a blower 1103. A first flow sensor 1192 and a second flow sensor 1194 are disposed along conduit 1190. Conduit 1190 includes first segment 1193 and second segment 1195. A supplementary gas inlet 1196 is in fluid communication with conduit 1190 via a supplementary valve 1197. Base unit 1102 includes a controller 1198 operatively coupled to valve 1197.

Base unit 1102 may use the same components of base unit 1002 of FIG. 10 but further includes functionality for introducing a supplementary gas into conduit 1190. Appropriate supplementary gases include, but are not limited to, oxygen, oxygen-concentrated breathing gas, helium, nitric oxide, heliox, anesthetic gas, and gas including aerosolized medicament. Supplementary gas inlet 1196 provides the supplementary gas from an external source, such as, a wall gas outlet, a gas concentrator, or a gas tank. Controller 1198 is configured to actuate supplementary valve 1197 to control the flowrate of supplementary gas entering conduit 1190. Supplementary valve 1197 may be, for example, a solenoid valve, a globe valve, or a diaphragm valve.

Supplementary gas inlet 1196 may provide oxygen or another breathable gas. As illustrated in FIG. 11, the supplementary gas may be inserted into conduit 1190 and mixed with the breathing gas from gas inlet 1188 before the mixed gases pass through blower 1103. Because some oxygen sources, such as oxygen concentrators, have a limited pressure output and are designed to deliver at a low flow rate, such sources may not be able to overcome the back pressure of a high velocity flow if supplementary gas inlet 1196 is positioned after blower 1103 in the system. Supplementary valve 1197 may be a valve with a low pressure drop or minimum pressure loss (*e.g.,* dual disc check valve, butterfly valve, ball valve, diaphragm valve), such that the limited pressure output of the oxygen/supplementary gas source is conserved.

Blower 1103 may generate a high pressure which accelerates the flow of mixed gases together when oxygen is inserted into the flow before blower 1103. Blower 1103 may be oxygen rated, including appropriate cleaning of surfaces to ensure there is no oil, anodized parts, use of silicone based oils, and oxygen cleaning of parts. Alternatively, in some implementations, supplementary gas inlet 1196 and second flow sensor 1194 are positioned after the breathing gas enters blower 1193, so that oxygen or other supplementary gas does not enter blower 1193.

Upon entering conduit 1190, the supplementary gas may mix with the breathing gas in conduit 1190 originating from gas inlet 1188. In some implementations, measurement device 1086 is configured such that the breathing gas and supplementary gas are fully or uniformly mixed prior to flowing into second flow sensor 1194. Second segment 1195 may be a sufficient length to allow uniform mixing of the breathing and supplementary gases.

Supplementary gas inlet 1196 and supplementary valve 1197 are positioned to introduce supplementary gas into conduit 1190 at a point between first flow sensor 1192 and second segment 1195. In some implementations, first flow sensor 1192 measures the flowrate of breathing gas, and second flow sensor 1194 measures the flowrate of a mixed flow of breathing gas and supplementary gas. Both first flow sensor 1192 and second flow sensor 1194 are configured to output to controller 1198 signals including a first measurement and a second measurement indicative of the measurements of the breathing gas and the mixed flow, respectively. Upon receipt of the signals, controller 1198 may be configured to calculate a difference between the first measurement and the second measurement. The calculated difference is indicative of the flowrate of supplementary gas introduced via supplementary valve 1197. Controller 1198 may be configured to calculate one or more concentrations of one or more components gas in the mixed flow based on the second measurement and the calculated difference. For example, the supplementary gas may be pure oxygen, and the controller may calculate the concentration of oxygen in the mixed flow based on the second measurement and the calculated difference, the calculated difference indicating the amount of oxygen added to the breathing gas over time.

As mentioned earlier, controller 1198 is configured to actuate supplementary valve 1197 to control the flowrate of supplementary gas entering conduit 1190. Controller 1198 may be configured to adjust the flowrate of supplementary gas by actuating supplementary valve 1197 based on the calculated flow difference and/or the calculated one or more concentrations. For example, controller 1198 may compare a calculated concentration to a target concentration, the target concentration being stored in a memory (not shown) and/or received as an input. The target concentration may be inputted by a user via an interface, such as, a computer link or a display like display 430 of FIG. 4. In this example, controller 1198 may, upon determining the calculated concentration is greater than or less than the target concentration, actuate supplementary valve 1197 to decrease or increase, respectively, the flowrate of supplementary gas, or vice versa.

First flow sensor 1192 and second flow sensor 1194 may be calibrated relative to each other, allowing for the controller to detect small differences in flowrate between first flow sensor 1192 and second flow sensor 1194. For example, the calculated flow difference may be less than about 5% of the measurement from first flow sensor 1192. The calculated flow difference may be less than about 1% of the measurement from first flow sensor 1192. Calibrating first flow sensor 1192 and second flow sensor 1194 relative to each other may reduce an error associated with the flow difference calculation to an error associated with the measurement by second flow sensor 1184. Controller 1198 may be configured to pause or cease flow of supplementary gas through supplementary valve 1197, for example, to calibrate first flow sensor 1192 and second flow sensor 1194 relative to each other while there is no supplementary gas flow.

In some implementations, conduit 1190 is coupled to one or more additional supplementary gas inlets (not shown) configured to introduce one or more additional supplementary gases. There may be one additional flow sensor (not shown) for each additional supplementary gas inlet. These additional flow sensors and additional supplementary gas inlets may alternate in order along conduit 1190, such that controller 1198 may calculate the flowrate of each additional supplementary gas added to the breathing gas stream through conduit 1190, based on the previous methods.

Supplementary gas inlet 1196 may include a pressure sensor configured to measure pressure of the incoming supplementary gas. The measured pressure value may be transmitted to controller 1190. Using the measured pressure value, controller 1190 may determine what type of external source is supplying the supplementary gas. For example, oxygen is supplied at a high pressure, and controller 1190 determines it is from a wall outlet, whereas a relatively low pressure may indicate oxygen supplied from an oxygen concentrator. Each type of source may supply oxygen at different levels (*e.g.,* 100% oxygen from a wall outlet or about 93% oxygen from an oxygen concentrator), so the calculation of flow rate and oxygen concentration in the breathing gas can take into account the incoming oxygen concentration determined from the source.

Controller 1190 may include software, hardware, and/or logic to enable closed-loop control of one or more of the breathing gas flowrate, the mixed flow flowrate, a concentration of a component of the mixed flow, temperature, and/or humidity based on data received by controller 1190 from one or more sensors coupled to controller 1190. In addition or alternatively, the closed-loop control may be based on data received from one or more external devices, for example, a pulse oximeter or a transcutaneous carbon dioxide sensor.

External Monitoring

In another aspect of the present disclosure, one or more external monitoring devices may be coupled to a base unit via corresponding one or more interfaces or ports. For example, a pulse oximeter and/or a transcutaneous carbon dioxide sensor may be coupled to a base unit to provide real-time feedback of patient oxygen and carbon dioxide, respectively. The use of external monitoring devices in combination with the controller to provide adaptive control or closed-loop feedback to the system is also described in U.S. Application No. 16/008,508 (now U.S. Patent No. 10,514,662), filed June 8, 2018, and entitled "OXYGEN MIXING AND DELIVERY"; and U.S. Patent Application No. 14/602,392 (now U.S. Patent No. 10,007,238), filed January 22, 2015, and entitled "OXYGEN MIXING AND DELIVERY".

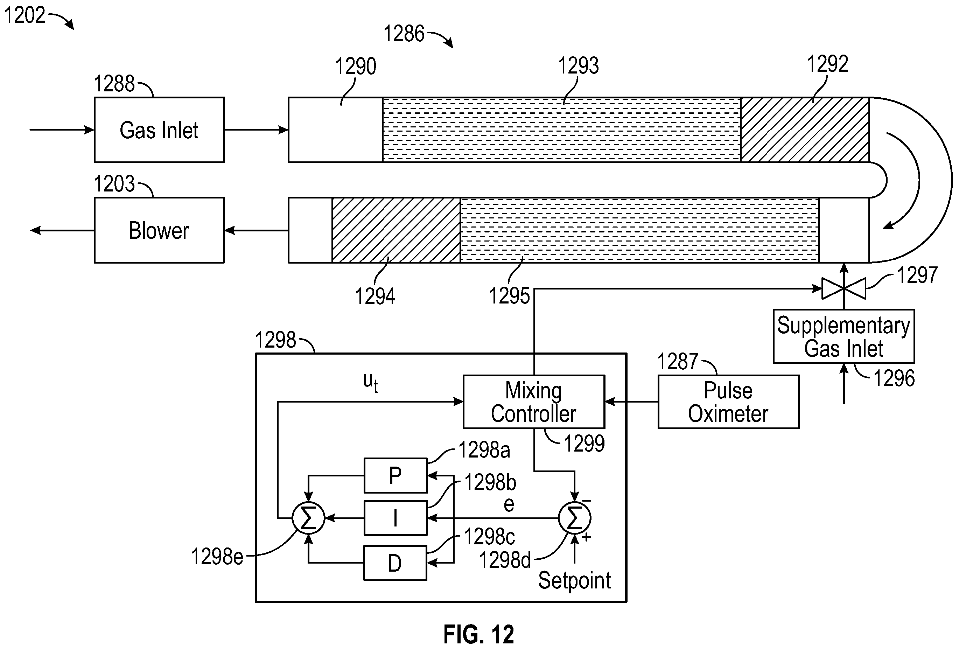

FIG. 12 depicts a measurement device 1286 configured to receive breathing gas from gas inlet 1288 and supplementary gas from supplementary gas inlet 1296 via supplementary valve 1297, according to an illustrative implementation. Measurement device 1286 includes a conduit 1290 coupled to gas inlet 1288 and a blower 1203. A first flow sensor 1292 and a second flow sensor 1294 are disposed along conduit 1290. Conduit 1290 includes first segment 1293 and second segment 1295. Supplementary gas inlet 1296 is in fluid communication with conduit 1290 via supplementary valve 1297. Base unit 1202 includes a controller 1298 operatively coupled to valve 1297. Controller 1298 includes a mixing controller 1299, a proportional module 1298a, an integral module 1298b, a derivative module 1298c, an error module 1298d, and an output module 1298e. Mixing controller 1299 is coupled to supplementary valve 1297 and a pulse oximeter 1287.

Gas inlet 1288, conduit 1290, first flow sensor 1292, second flow sensor 1294, first segment 1293, second segment 1295, and blower 1203 may be the same as the corresponding components in FIGs. 10 and 11. Like measurement device 1186 of FIG. 11, measurement device 1286 allows for introduction of a supplementary gas from supplementary gas inlet 1296 via supplementary valve 1297 which is operatively coupled to controller 1298. In this implementation, pulse oximeter 1287 is configured to output to controller 1298 data indicative of a percentage of oxygen saturation ($SpO_2$) of a patient's blood. "$SpO_2$" is an acronym for "saturation of peripheral oxygen," and within the related technology, the term $SpO_2$ is often casually referred to as "blood oxygen", "blood oxygen saturation", and other similar terms. Some implementations herein use $SpO_2$ as an estimation of blood oxygen concentration and is usually measured with a pulse oximeter. A pulse oximeter is generally a photoelectric device that measures the amount of saturated hemoglobin in tissue capillaries by transmitting beams of light through the tissue to a light receiver. A pulse oximeter is generally configured so as to clip onto a fingertip or earlobe. The amount of saturated hemoglobin affects the wavelength and reflection or transmission of the light transmitted through the tissue. By analyzing the received light, $SpO_2$ may be deduced. Pulse oximeters may also allow for measurement of a pulse and generating various alarm condition signals. While photoelectric pulse oximeters have been described here, it is to be understood that other types of pulse oximeters may be substituted for pulse oximeter 1287.

In some implementations, controller 1298 is configured to actuate valve 1297 via mixing controller 1299 based on data from pulse oximeter 1287. Controller 1298 may also receive a target $SpO_2$ as an input, for example, via a computer interface or a display such as a display 430 of FIG. 4 which allows for user interaction. A target $SpO_2$ may also be stored in a memory (not shown). Controller 1298 may be configured to compare a measured $SpO_2$ from pulse oximeter 1287 with the target $SpO_2$ and determine an appropriate change in supplementary gas flowrate. For example, the comparison may reveal that the measured $SpO_2$ is below the target $SpO_2$, and the controller may calculate a target supplementary gas flowrate that would raise the patient's $SpO_2$ to the target $SpO_2$. The target supplementary gas flowrate may be implemented by actuating valve 1297 and calculating a difference in flowrate between a first flow measurement from first flow sensor 1292 and a second flow measurement from second flow sensor 1294, because the supplementary gas is introduced between first and second flow sensors 1292 and 1294. Controller 1298 may then further adjust valve 1297 if the calculated flowrate difference is different from the target supplementary gas flowrate.

Controller 1298 may be configured to adjust the flowrate of supplementary gas through valve 1297 in order to set an oxygen concentration in the mixed flow to a minimum oxygen concentration that is determined to have a therapeutic effect on the patient based on data received from pulse oximeter 1287. This capability may be especially advantageous when supplementary gas is supplied from an external tank that has a limited capacity. Controller 1298 may minimize the amount of supplementary gas needed in order to maximize the lifetime of the external tank, providing effective respiratory therapy to a patient for as long as possible given the constraint. Controller 1298 may generate for display one or more graphics indicative of the $SpO_2$, $PaO_2$, or $FiO_2$.

Controller 1298 includes various components allowing for proportional-integral-derivative (PID) control of the breathing gas and supplementary gas. PID controller 1298 allows for feedback-based control of various system parameters, such as the breathing gas flowrate, supplementary gas flowrate, and pump flowrate. Similar configurations are described in U.S. Application No. 16/008,508 (now U.S. Patent No. 10,514,662), filed June 8, 2018, and entitled "OXYGEN MIXING AND DELIVERY"; and U.S. Patent Application No. 14/602,392 (now U.S. Patent No. 10,007,238), filed January 22, 2015, and entitled "OXYGEN MIXING AND DELIVERY". A PID controller is a control loop feedback controller. A PID controller calculates an error value as the difference between a measured process variable and a desired value of the process variable (or set point). The controller operates to minimize the difference between the measured value and the set point. A PID controller accomplishes this by use of an algorithm that uses three separate parameters - proportional (P), integral (I), and derivative (D) values interpreted at discrete increments of time where P depends on the current error, I depends of the accumulation of past errors, and D predicts future errors. The weighted sum of these three actions is used to adjust a process-in this case the proportion of oxygen represented by $FiO_2$. Mathematically, these values are generally represented by the following equations:

$$P = K_p e(t) \qquad\qquad \text{Eq. 1}$$

$$I = K_i \int_0^t e(\tau)d\tau \qquad\qquad \text{Eq. 2}$$

$$D = K_d \frac{de(t)}{dt} \qquad\qquad \text{Eq. 3}$$

where a mixing control signal is derived from the PID controller output $u_t$:

$$u_t = P + I + D = K_p e(t) + K_i \int_0^t e(\tau)d\tau + K_d \frac{de(t)}{dt} \qquad \text{Eq. 4}$$

and where:

$K_p$ : proportional gain coefficient;
$K_t$ : integral gain coefficient;
$K_d$ : derivative gain coefficient;
$e$ : error, difference between measurement and target;
$t$: time; and
$\tau$ : integration variable, takes on values from time $t = 0$ to present time $t$.

For purposes of this document, a controller may be referenced as a PID controller for convenience and by way of example, but in practice the controller may in fact not use all three of the elements of proportional, integral, and derivative control. Use of only one or two of the PID control functions is common and use of other feedback control mechanisms is also within the scope of the present teachings. For example, in other example implementations, a PI controller (proportional-integral) or other suitable feedback controller could alternatively be used.

In this aspect, controller 1298 includes modules for each PID control parameter. Module 1298a uses Eq. 1 for proportional control, module 1298b uses Eq. 2 for integral control, and module 1298c uses Eq. 3 for derivative control. The current value of $PaO_2$ (representing the measured $SpO_2$ from pulse oximeter 1287) is received by mixing controller 1299 and subtracted from the setpoint (*i.e.,* the target value) at error module 1298d to produce the error signal e at the output of error module 1298d. This error signal e is then processed by the P, I, and D modules 1298a, 1298b, and 1298c, respectively, according to equations 1-3 above. The outputs of modules 1298a, 1298b, and 1298c are summed at output module 1298e to produce output $u_t$ which is provided to mixing controller 1299. Mixing controller 1299 processes output $u_t$ into an appropriate format to effect control of supplementary valve 1297 to establish an appropriate blend of breathing gas and supplementary gas dictated by PID controller 1298.

An example PID controller equation that may be used for this application is:

$$PaO_2 = (KL * FiO_2) + K2 \qquad \text{Eq. 5}$$

where *KL* is the lung function gain coefficient relating to the lung's ability to efficiently transfer oxygen and carbon dioxide. K2 is the offset relating to level of overall respiratory capability of a patient. Controller 1298 may use a relatively long sample period, for example, about 10 seconds, which serves as a type of low-pass filter to ensure accuracy of $SpO_2$ calculated from pulse oximeter 1287. Another type of low-pass filter can be provided by using, for example, 90% of old data (from a prior sample period) and adding in, for example, 10% of the new data (from a new sample period). Both filters enhance controller performance so that it is more responsive but not overly responsive. Controller 1298 may also use an "initial" value of $O_2$ upon initiation of adaptive control. The initial value may be set by a user or operator and is used by controller 1298 to ensure the system starts in a relatively steady-state condition, for example, to avoid having PID control adjusting the patient's $O_2$ level up and down upon startup.

To ensure the system starts operation in a steady-state condition, the system may be initialized using an initial *KL* calculated by:

$$KL_i = (PaO_2)_i / (FiO_2)_i \qquad \text{Eq. 6}$$

where $KL_i$, $(PaO_2)_i$, and $(FiO_2)_i$ are all initial values. Furthermore, within each sample period, controller 1298 may use an adaptation algorithm to form new PID gain coefficients. An example algorithm is:

$$KL_{new} = 0.9 * KL_{old} + 0.1 * (PaO_2 / FiO_2) \qquad \text{Eq. 7}$$

where $KL_{new}$ may be used for calculating new PID gain coefficients.

Pulse oximeter 1287 measures $SpO_2$; however, controller 1298 may use $PaO_2$ for its calculations. The term "$PaO_2$" means partial pressure of arterial oxygen. Although, in other implementations, control signals may be more directly derived from $SpO_2$ or other measures of a patient's blood oxygen concentration without limitation. Conversion of $SpO_2$ to $PaO_2$ may be effected in a number of ways. In one example, measured $SpO_2$ is converted into the correlating partial pressure of arterial oxygen $PaO_2$ by using an oxyhemoglobin dissociation curve based on standard conditions of temperature equal to 37 °C, pH equal to 7.4, and the Bohr effect is not present. The dissociation curve plots $PaO_2$ against $SpO_2$, allowing one to read the value of $PaO_2$ off the graph based on a known value of $SpO_2$. Oxyhemoglobin dissociation curves may be shifted to the left due to conditions causing high $O_2$ affinity and to the right due to conditions causing low $O_2$ affinity. The curve generally approximates a sigmoidal shape and various equations can be devised to closely model the shape of the curve using various curve fitting techniques. Using such equations, the value of $PaO_2$ may be calculated directly. Alternatively, data points from the dissociation curve can be cataloged into a lookup table stored in a storage or memory (not shown) which can be used by mixing controller 1299 to convert the value of $SpO_2$ to a value of $PaO_2$. If the exact value of $SpO_2$ is not on the table, then a linear or non-linear interpolation (any suitable interpolation such as polynomial, piecewise, spline, bilinear, extrapolation, etc.) can be performed to approximate the corresponding value of $PaO_2$. The more data points provided in the lookup table, the more accurate the interpolation, if necessary, will be.

An example workflow for adaptive control is as follows. Mixing controller 1298 receives pulse oximeter data from pulse oximeter 1287. The pulse oximeter data includes $SpO_2$ data and alarm condition signals. From the $SpO_2$ data, mixing controller 1299 determines $PaO_2$ data for calculation of an appropriate oxygen concentration of the mixed flow output to the patient. The $PaO_2$ data may be determined by referencing a stored lookup table, using interpolation if necessary, where the lookup table may be derived from an oxyhemoglobin dissociation curve. Controller 1298 and its components 1298a-e effect adaptive feedback control of the gas based on the $SpO_2$ data by mixing controller 1298 interfacing with valve 1297. Adaptive feedback control is provided by the PID control scheme. Controller 1298 may receive flowrate data or a flowrate signal indicating that the breathing gas delivered out of conduit 1290 has been manually changed. This flowrate data may be received from a user interface, such as a display like display 430 of FIG. 4, or from first or second flow sensors 1292 and 1294. Upon receiving the flowrate data or flowrate signal, controller 1298 may enter a manual override mode and halt adaptive feedback control (*i.e.* halt sending signals to valve 1297). Controller 1298 may be configured to compare measured SpO$_2$ data with alarm limits and initiate an alarm condition if the measured SpO$_2$ data is outside the alarm limits. An alarm condition may involve halting operation of base unit 1202 and/or generating for display an alert for the user *(e.g.,* on a display like display 430 of FIG. 4).

Another example workflow involves mixing controller 1299 receiving pulse oximeter data from pulse oximeter 1287. The pulse oximeter data includes one or more signals indicative of a current blood oxygen level of the patient. Controller 1298 receives from first flow sensor 1292 and second flow sensor 1294 gas data indicative of measured breathing gas flowrates. The measured breathing gas flowrates may be subtracted to determine an amount of supplementary gas added between first and second flow sensors 1292 and 1294. Controller 1298 may calculate a concentration of oxygen (percentage oxygen) in the mixed flow (breathing gas and supplementary gas) based on the calculated added amount and the second measured breathing gas flowrate. Controller 1298 compares the one or more current blood oxygen level signals to a target blood oxygen level. The target may be stored in a memory in base unit 1202 or inputted by a user via a user interface. Controller 1298 calculates an appropriate change to the mixed flow to achieve a change in the oxygen concentration in the mixed flow. Mixing controller 1299 actuates valve 1297 to alter the oxygen concentration in the mixed flow *(e.g.,* by increasing or decreasing supplementary gas flowrate, the supplementary gas possibly containing oxygen). Mixing controller 1299 may receive new signals from pulse oximeter 1287 indicative of a current blood oxygen level of the patient.

In some implementations, a transcutaneous carbon dioxide sensor is coupled to controller 1298 of base unit 1202, and the transcutaneous carbon dioxide sensor is configured to output to controller 1298 one or more signals indicative of PaCO$_2$. Transcutaneous carbon dioxide sensors generally measure the skin-surface partial pressure of carbon dioxide (PtcCO$_2$) to provide an estimate of the partial pressure of arterial carbon dioxide (PaCO$_2$). In some cases, both the sensor also measures partial pressure of oxygen (PtcO2) to estimate the partial pressure of arterial oxygen (PaO2). Controller 1298 may be configured for closed-loop control of patient carbon dioxide based on the received signals, using the methods previously described for closed-loop oxygen control.

Using a sensor that measures both PtcCO$_2$ and PtcO$_2$, or using both a pulse oximeter and a transcutaneous carbon dioxide sensor, controller 1298 may be configured to receive both oxygen and carbon dioxide data and determine an appropriate therapy for the patient based on the received data. For example, controller 1298 may compare the measured oxygen data and the measured carbon dioxide data to a stored reference table which includes at least one reference oxygen value and at least one reference carbon dioxide value. Controller 1298 may compute differences between a current blood oxygen level and a reference oxygen level and between a current blood carbon dioxide level and a reference carbon dioxide level. Controller 1298 may be configured to determine to provide the patient with a high oxygen therapy *(e.g.,* to increase blood oxygen levels) or a high flush therapy *(e.g.,* to decrease blood carbon dioxide levels). For example, if the blood oxygen level difference is greater than the carbon dioxide level difference, controller 1298 may select the high oxygen therapy, or if the blood carbon dioxide level difference is greater than the oxygen level difference, controller 1298 may select the high flush therapy. Upon selecting high oxygen therapy, controller 1298 may direct mixing controller 1299 to actuate valve 1297 to increase the flowrate of supplementary gas into conduit 1290, for example, in cases where the supplementary gas contains a higher oxygen concentration than the breathing gas from gas inlet 1288. Upon selecting high flush therapy, controller 1298 may increase the breathing gas flowrate through gas inlet 1288 by, for example, increasing a suction pressure of blower 1203. Providing high flush therapy may involve increasing an output pressure or output flowrate of blower 1203.

Use with Aerosolized Medications

The systems described herein, including systems 100, 400, 500, 600, and 800 of FIGs. 1, 4-6, 8A, and 8B, may comprise or may be configured to connect to a device configured to produce aerosolized medication. For example, these systems include a nebulizer or aerosolizer. Liquid medicaments that are to be administered via inhalation may be aerosolized and introduced into the breathing gas supplied by these systems before being delivered to a patient. This configuration allows for treatment of some respiratory diseases by delivering breathing gas with entrained aerosolized medicament which is breathed in for direct transport to the lungs.

A nebulizer (pneumatic, mechanical, or electrical) may be connected at certain points in the system. For example, a delivery tube of the system, such as delivery tubes 406 and 706 of FIGs. 4 and 6, includes a port configured to attach a nebulizer, from which aerosolized medicament enters the delivery tube and becomes entrained in breathing gas flow. As another example, a patient connector or nasal cannula configured to the end of the delivery tube includes a port configured to attach the nebulizer. In other implementations, an auxiliary unit, such as auxiliary units 404, 504, 604, 704, 804, and 904 of FIGs. 4-9, includes a port configured to attach the nebulizer, from which aerosolized medicament enters the auxiliary unit and becomes entrained in breathing gas flow. In some implementations, the aerosol is introduced into the breathing gas before the breathing gas enters a vapor transfer unit for humidification. In other implementations, the aerosol is introduced into the breathing gas after the breathing gas is humidified by the vapor transfer unit. The system may be configured to adjust the vaporization rate in the vapor transfer unit based on the amount of aerosol entrained in the breathing gas, by monitoring the aerosol flow rate via a flow sensor or level sensor in the nebulizer or a flow sensor in the port or auxiliary unit.

An RFID tag or other tag, chip, or sensor in the auxiliary unit may store information about the type of nebulizer or medication to be introduced via the nebulizer. In some implementations, high viscosity fluids (*i.e.,* > 6 cP) are used in the nebulizer to be aerosolized and subsequently entrained. The RFID tag may be read, and the stored information is transmitted to a controller in the base unit. Based on the information, the controller may adjust the vaporization rate or heating rate in the auxiliary unit. The controller may also be operatively coupled to the nebulizer and use the information to adjust operation of the nebulizer. For example, if it is indicated that a high viscosity fluid will be nebulized, the controller adjusts the vibration frequency, pressure, force, or other parameter of the nebulizer to account for the high viscosity. The controller may be operatively coupled to the nebulizer so as to effect feedback control of the nebulizer. For example, nebulization rate may be adjusted by the controller in response to a measured patient parameter (*e.g.,* breath rate, blood oxygen concentration).

Methods of Treatment

Provided herein are systems and methods for respiratory therapy. In some aspects, these systems and methods are used to treat certain respiratory diseases or conditions. Operation of the systems provided herein may be attuned for the specific respiratory disease or condition being treated. For example, certain supplementary gases or aerosolized medicament is used for treatment of certain conditions.

In some implementations, the systems described herein are used for the treatment of coronavirus disease 2019 (COVID-19), caused by severe acute respiratory syndrome coronavirus 2 (SARS-CoV-2). These systems configured with a high flow nasal cannula may be used for mechanical ventilation of patients with COVID-19 related respiratory failure. In some implementations, this form of therapy, using a high flow nasal cannula and the system parameters described in Table 1 or Table 2, can be performed without intubation. In some implementations, this method of treatment further utilizes a face mask to reduce the spread of aerosol particles or contaminants exhaled by the patient. Treatment of COVID-19 may involve introducing supplementary oxygen into the breathing gas via a supplementary gas inlet (*e.g.,* supplementary gas inlets 1196 and 1296 of FIGs. 11 and 12). A pulse oximeter may be attached to the patient during treatment, such that $SpO_2$ is monitored and the flowrate of supplementary oxygen can be adjusted to provide enough oxygen to reach a therapeutic level of oxygen in the patient.

Mobile Use

The systems and methods described herein may be used in a mobile configuration. Systems 100, 400, 500, 600, and 800 of FIGs. 1, 4-6, 8A, and 8B can be used at home, in a vehicle, on a mobile platform or cart, or in a backpack. The systems and methods, using a blower, are configured to receive and pressurize ambient air, so they can operate without need of an external gas source like wall air or pressurized tanks. Furthermore, the systems and methods are configured to operate on battery power, so they do not require an external power source like an electrical outlet or generator. The battery has enough capacity to provide heated and humidified respiratory therapy for hours, *e.g.,* > 8 hours, > 6 hours, > 4 hours, > 2 hours, > 1 hour. For example, the respiratory therapy systems are configured on a rolling cart allowing a patient to move throughout their home or a hospital while receiving respiratory therapy from the system moving with them.

In another example, a patient in a vehicle *(e.g.,* a personal vehicle, an ambulance, an airplane, or a helicopter) receives respiratory therapy via a specific auxiliary unit (or a patient circuit including the specific auxiliary unit, delivery tube, and patient connector or nasal cannula) configured to a first base unit. Upon arriving at a medical facility, the specific auxiliary unit is removed from the first base unit. As discussed in the foregoing, the base unit may be configured to automatically stop operation when the auxiliary unit is removed. The first base unit remains in the vehicle, while the auxiliary unit is transported with the patient into the facility. The specific auxiliary unit may then be re-docked into a second base unit in the medical facility, resuming respiratory therapy via the second base unit. The second base unit must be set up again with the correct therapy and started up, consuming valuable time.

To hasten startup time of the second base unit, the auxiliary unit may comprise an RFID tag, as discussed above, that stores the previous operating conditions from when the auxiliary unit was used in the vehicle with the first base unit. The RFID tag may have data written to it by the first base unit. The data may indicate that the auxiliary unit was actively treating the patient and what therapy was being given. Upon re-docking in the second base unit, a reader in the second base unit reads the RFID tag, sending the data or information indicative of the previous therapy or operating conditions to the controller of the second base unit. The controller then sets parameters to match the previous operating conditions, allowing the patient to resume the same respiratory therapy. The same auxiliary unit is used for the patient, allowing the patient to be transferred from transport to the facility without replacing the auxiliary unit.

The data stored on the RFID tag may indicate: that the auxiliary unit was providing therapy, the time at which therapy was interrupted, the settings of the therapy (*e.g.*, breathing gas flow rate, temperature, oxygen concentration, humidity, pump power, heating rate, valve settings), the state of the patient circuit (*e.g.,* the actual temperature of the breathing gas as compared to a set point temperature), and patient data (*e.g.,* age, height, weight, disease type, disease status, concomitant disease, concomitant therapy). Data may be stored continuously, such that ejecting the auxiliary unit from the base unit results in the latest settings being stored. In some implementations, the RFID tag has limited data storage or write operations, so the past data is overwritten with current data. An operator may initiate the write operation prior to stopping therapy, or the controller may be configured to automatically write the data upon receiving a user command to stop operation. The controller may be configured to include a switch that stores the data and prompts the user to remove the device. In some implementations, data is stored whenever setting are changed, storing the last known settings. Upon re-docking of the auxiliary unit, the controller of the second base unit may be configured to generate for display a prompt to the user to resume therapy if desired. In some implementations, the therapy has lapsed for an extended time or the actual temperature was lower than the set point, so the second base unit operates the heating elements to warm up the auxiliary unit before resuming therapy.

The first base unit used in transport may have limited functions, for example, without some or all of the following features: liquid heating, liquid circulation, full range of flow rates, full range of oxygen therapy, liquid level sensing, valve control, and user interface/display. The base unit may include features that provide the minimum therapy needed for the specific patient or specific transport operation. A blower of lower pressure or flowrate capacity may be used. The auxiliary unit may be corresponding limited to function with only the features which the first base unit is limited to. Removing some of the features from the first base unit for transport allows for extended operation on battery power or low-power mode and for a more compact unit. When re-docking the auxiliary unit in the second base unit, if the first base unit is lacking certain features, the user may be prompted to adjust the settings appropriately. In some implementations, the auxiliary unit may have limited functions; for example, the delivery tube does not have a water jacket, the delivery tube has a solid insulator rather than a water jacket, and/or the auxiliary unit does not have a liquid pump. This may allow the device to be more light weight and less complex, which would be advantageous for mobile use.

Example of High Flow Therapy

FIGs. 13A and 13B show graphical representations (contour plots) of simulated amounts of carbon dioxide in a patient's airway during high flow therapy, according to an illustrative implementation. In these implementations, a blower-based respiratory therapy system is used, such as systems 100, 400, 500, 600, and 800 of FIGs. 1, 4-6, 8A, and 8B. A nasal cannula transfers breathing gas from the system into a nare of the patient. In both FIGs. 13A and 13B, the breathing gas flowrate through nasal cannula is 40 L/min, and the patient is breathing at 24 breaths per minute. The amount of $CO_2$ present in the patient's airway is captured in the contour plots when the patient is at peak expiratory flow, the maximum flowrate they reach during exhalation. Darker regions indicate a higher percentage of $CO_2$, and lighter regions indicate a lower percentage of $CO_2$.

The simulations differ in that FIG. 13A uses a large bore cannula, and FIG. 13B uses a small bore cannula. Using the large bore cannula (specifically having a 4.13 mm inner diameter), simulated in FIG. 13A, high flow therapy results in a large percentage $CO_2$ (up to 0.0486 mass fraction of $CO_2$) in the patient's upper airway. Patients typically rebreathe a third of previously expired tidal volume, where previously exhaled breath (low in oxygen and with some $CO_2$) is not fully exhaled and remains in the upper airway. As patients rebreathe, $CO_2$ in this upper airway reservoir is drawn into the lungs. Patients with acute respiratory failure rebreathe a larger percentage of gas, resulting in rebreathing larger amounts of carbon dioxide as they draw breaths from the upper airway reservoir. Accordingly, it is not desirable for the patient to have larger percentages of $CO_2$ in the reservoir, especially when higher amounts of oxygen are needed for a therapeutic effect.

FIG. 13B shows $CO_2$ percentages based on simulation of high flow therapy with a small bore cannula, specifically having a 2.64 mm inner diameter in accordance with the parameters of row 3 of Table 1 or row 3 of Table 2. Use of the small bore cannula, at otherwise the same operational parameters, leads to lower percentages of $CO_2$ in the patient's upper airway compared to the large bore cannula implementation. Continuous high flow therapy with the small bore cannula washes out the upper airway, because the smaller inner diameter of the nasal cannula prong induces a higher exit velocity of gas when operating at the same volumetric flowrate. A continuous high flow of fresh gas with this higher exit velocity, the upper airway of the patient is washed out, effectively creating a reservoir of oxygen in the upper airway (pharyngeal dead space) available for gas exchange during rebreathing. In this implementation, rebreathing of $CO_2$ is avoided, instead replacing $CO_2$ with oxygen-rich gas and improving breathing efficiency.

While the small bore cannula shows better flushing of $CO_2$ in the upper airway, the large bore cannula may be advantageous for use in oxygen delivery. Accordingly, in some implementations, a large bore cannula is used for oxygen delivery and a small bore cannula is used situations in which flushing is more important (*e.g.*, for hypercapnic therapy).

Minimum Back Pressure to Achieve High Velocity

Described herein are systems and methods for high velocity respiratory therapy. These systems and methods can utilize a nasal cannula to administer breathing gas to the nare(s) of a patient. The cannulas used herein have one or more nasal prongs with outlets that are small relative to conventional cannula designs. The small outlet accelerates the flow of breathing gas to a velocity that is considered high enough to achieve high velocity nasal insufflation, which has the effect of flushing the upper airway as described above.

The cannula may be designed such that each portion of its flowpath has a large cross-section and smooth transitions wherever possible, except at the nasal prong where the prong cross-section tapers down to a small diameter at the prong opening. Using such a design, one can assume that most or all of the pressure drop effected on the flow of breathing gas by the nasal cannula is due to the tapering of the nasal prong(s), such that all of the nasal cannula pressure drop contributes to the acceleration of the flow to the high velocity.

Described herein is a computational fluid dynamics (CFD) study performed to determine the minimum possible nasal cannula pressure drop that could be achieved for any given target velocity. The study uses a nasal cannula designed such that the pressure drop through the nasal cannula is zero until the flow is accelerated through the prong to the final exit velocity, such that the cannula pressure drop is used for accelerating the flow. Example cannula designs have prong inner diameters ranging from 1 mm to 4 mm. In the study, prong inner diameters from 1.27 mm (0.05 in) to 3.81 mm (0.15 in) were studied. Each prong size was simulated at a flowrate that would result in exit velocities of about 40, about 50, about 60, about 70, and about 80 m/s. Any of the blower-based respiratory therapy system described herein, such as systems 100, 400, 500, 600, and 800 of FIGs. 1, 4-6, 8A, and 8B, or systems using other breathing gas sources (*e.g.*, compressors) can be designed to have the flow path sizing and pressure drops needed to achieve high velocity as described in the study. The full results of the study are tabulated below in Table 4.

**Table** 4: Results of the CFD study, showing the volumetric flow rate and cannula pressure drop that results from exit velocities of about 40, about 50, about 60, about 70, and about 80 m/s, for each prong inner diameter size.

| Prong ID (in) | Prong ID (mm) | Volume Flow Rate (L/min) | Velocity (m/s) | Cannula Pressure Drop [kPa] |
|---|---|---|---|---|
| 0.05 | 1.27 | 5.8 | 38.2 | 1.04 |
| 0.05 | 1.27 | 7.3 | 47.7 | 1.59 |
| 0.05 | 1.27 | 8.7 | 57.5 | 2.30 |
| 0.05 | 1.27 | 10.2 | 67.4 | 3.14 |
| 0.05 | 1.27 | 11.8 | 77.4 | 4.10 |
| 0.06 | 1.524 | 8.4 | 38.6 | 1.04 |
| 0.06 | 1.524 | 10.5 | 48.2 | 1.61 |
| 0.06 | 1.524 | 12.7 | 57.8 | 2.31 |
| 0.06 | 1.524 | 14.8 | 67.7 | 3.17 |
| 0.06 | 1.524 | 17.0 | 77.6 | 4.15 |
| 0.07 | 1.778 | 11.6 | 39.1 | 1.06 |
| 0.07 | 1.778 | 14.6 | 48.9 | 1.65 |
| 0.07 | 1.778 | 17.5 | 58.7 | 2.38 |
| 0.07 | 1.778 | 20.5 | 68.7 | 3.26 |
| 0.07 | 1.778 | 23.6 | 79.1 | 4.39 |
| 0.08 | 2.032 | 15.2 | 39.1 | 1.06 |
| 0.08 | 2.032 | 19.0 | 48.8 | 1.65 |
| 0.08 | 2.032 | 22.8 | 58.6 | 2.38 |
| 0.08 | 2.032 | 26.8 | 68.8 | 3.31 |
| 0.08 | 2.032 | 30.7 | 79.0 | 4.35 |
| 0.09 | 2.286 | 19.5 | 39.7 | 1.08 |
| 0.09 | 2.286 | 24.4 | 49.6 | 1.68 |

(continued)

| Prong ID (in) | Prong ID (mm) | Volume Flow Rate (L/min) | Velocity (m/s) | Cannula Pressure Drop [kPa] |
|---|---|---|---|---|
| 0.09 | 2.286 | 29.3 | 59.5 | 2.42 |
| 0.09 | 2.286 | 34.4 | 69.8 | 3.37 |
| 0.09 | 2.286 | 39.5 | 80.2 | 4.43 |
| 0.1 | 2.54 | 24.2 | 39.7 | 1.07 |
| 0.1 | 2.54 | 30.2 | 49.6 | 1.67 |
| 0.1 | 2.54 | 36.3 | 59.8 | 2.44 |
| 0.1 | 2.54 | 42.5 | 70.0 | 3.33 |
| 0.1 | 2.54 | 48.9 | 80.4 | 4.39 |
| 0.11 | 2.794 | 29.4 | 40.0 | 1.07 |
| 0.11 | 2.794 | 36.7 | 49.9 | 1.67 |
| 0.11 | 2.794 | 44.1 | 60.0 | 2.44 |
| 0.11 | 2.794 | 51.6 | 70.2 | 3.33 |
| 0.11 | 2.794 | 59.2 | 80.5 | 4.37 |
| 0.12 | 3.048 | 35.4 | 40.4 | 1.08 |
| 0.12 | 3.048 | 44.2 | 50.5 | 1.70 |
| 0.12 | 3.048 | 53.2 | 60.7 | 2.47 |
| 0.12 | 3.048 | 62.2 | 71.0 | 3.40 |
| 0.12 | 3.048 | 71.5 | 81.6 | 4.51 |
| 0.13 | 3.302 | 41.3 | 40.2 | 1.08 |
| 0.13 | 3.302 | 51.8 | 50.4 | 1.70 |
| 0.13 | 3.302 | 62.2 | 60.6 | 2.46 |
| 0.13 | 3.302 | 72.8 | 70.9 | 3.39 |
| 0.13 | 3.302 | 83.7 | 81.5 | 4.49 |
| 0.14 | 3.556 | 48.2 | 40.5 | 1.11 |
| 0.14 | 3.556 | 60.5 | 50.7 | 1.74 |
| 0.14 | 3.556 | 72.9 | 61.2 | 2.53 |
| 0.14 | 3.556 | 85.4 | 71.6 | 3.47 |
| 0.14 | 3.556 | 98.1 | 82.3 | 4.62 |
| 0.15 | 3.81 | 55.5 | 40.6 | 1.11 |
| 0.15 | 3.81 | 69.7 | 51.0 | 1.74 |
| 0.15 | 3.81 | 83.6 | 61.1 | 2.53 |
| 0.15 | 3.81 | 98.4 | 71.9 | 3.48 |

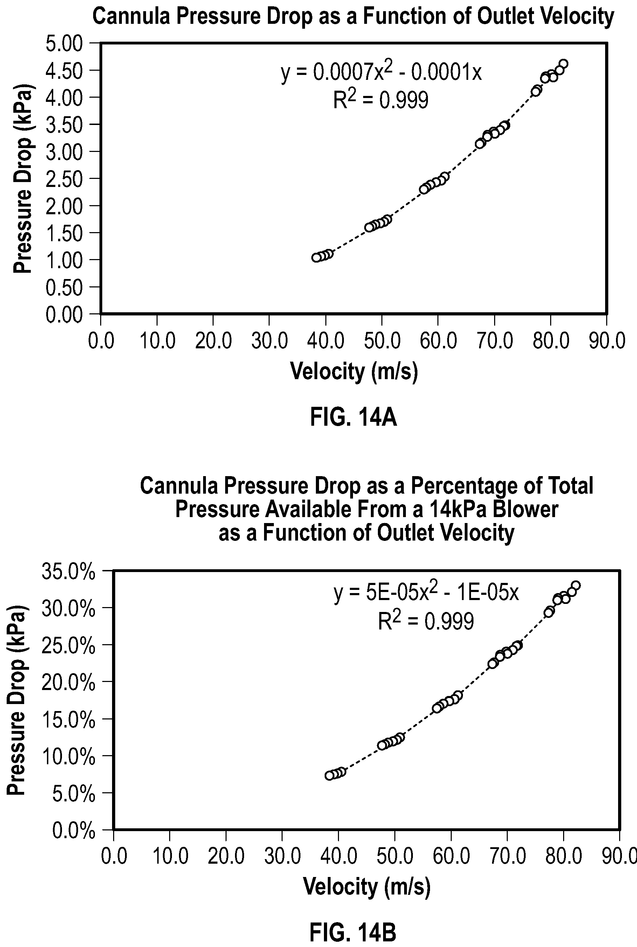

FIG. 14A shows a plot of the cannula pressure drop as a function of the outlet velocity for each size and flowrate studied. For each prong size studied, the flowrate needed to achieve a particular velocity is different. For example, to achieve a velocity of approximately 40 m/s, a 1.27 mm (0.05 in) prong requires a flowrate of 5.8 L/min, while a 2.54 mm (0.10 in) prong requires 24.2 L/min. In both cases, however, the pressure required to reach that velocity is about 1 kPa, and this pattern is consistent throughout the studied prong sizes and flowrates for each velocity. This pattern shows that, in general, the minimum cannula pressure drop to achieve a particular velocity is constant, regardless of prong diameter size. FIG. 14A reflects this pattern, as the combinations of prong size and flowrate for each velocity are grouped at approximately the same cannula pressure drop.

When using a blower to generate air flow, the blower is typically limited to relatively low pressure, as compared to compressors. Blowers that are sized for use in the delivery of medical gasses generally do not exceed about 14 kPa (2 psi). Blowers that can achieve these pressure are typically capable of very high flowrates that exceed the requirements of medical gas delivery and are operated at a point in the characteristic curve of the blower very close to the maximum available pressure. Operating close to the maximum available pressure corresponds to a relatively low flowrate when compared to what the blower can deliver. Blowers typically have a very flat characteristic curve at flowrates close to their maximum pressure, and this means that for flowrates from 0 to 60 L/min, a blower can be assumed to have relatively constant maximum pressure. Using this assumption, a combination of the maximum blower output pressure and the minimum pressure drop that is required to achieve a particular velocity, the pressure required to deliver high velocity nasal insufflation can be expressed as a percentage of the maximum pressure available (assumed herein to be the output pressure of the breathing gas source).

Accordingly, FIG. 14B shows a plot of cannula pressure drop as a percentage of total pressure available from a 14 kPa blower as a function of outlet velocity. There are few blowers sized for a respiratory therapy device that can delivery greater than 14 kPa, and the curve shown in FIG. 14B represents an ideal cannula where all pressure drop in the cannula is contributing to acceleration of the flow of breathing gas. Therefore, for any given velocity, it is unlikely that a cannula could be designed that requires a lower percentage of the total available pressure than is shown in the plot of FIG. 14B.

In practice, the gas passage used to convey the breathing gas from the blower to the nasal cannula cannot be of an unlimited size. Larger tubing is difficult to manage and could be uncomfortable for the patient. The additional surface area of larger tubing can exacerbate rainout from humidified gas. Using a subset of the prong sizes and velocities studied in FIGs. 14A and 14B, another simulation was run to study the impact of gas passage size on the pressure drop of the system. The cross-sectional area of the gas passage, expressed as multiples of the prong cross-section, varied from 1.1 to 5 times the prong cross-section. A 40.6 cm (16 in) gas passage length is assumed. The pressure drop in the gas passage is compared to the minimum cannula pressure drop for each particular velocity and prong size.

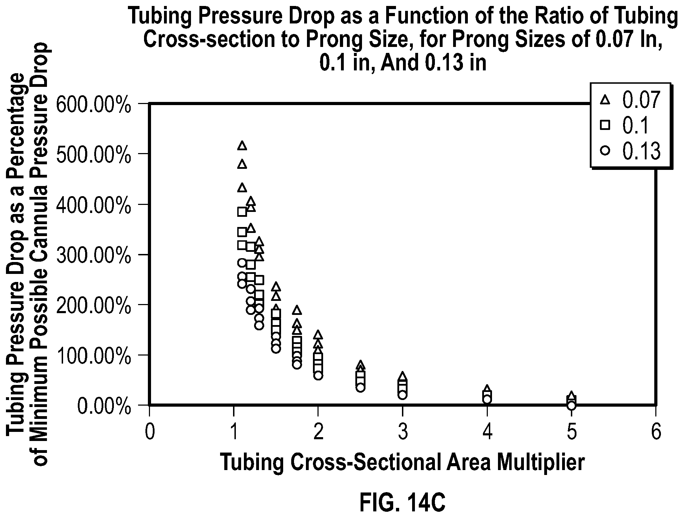

The results are shown in the plot of FIG. 14C. In the plot, when the gas passage cross-sectional area is 1.1 times the prong cross-sectional area, the pressure drop in the gas passage is 200 to 500% of the minimum cannula pressure drop. Increasing the gas passage cross-sectional area to 2.5 to 3 times the prong cross-sectional area reduces the pressure drop to approximately 50% of the minimum cannula pressure drop. Further increasing the gas passage size delivers diminishing returns. In the systems described herein, a gas passage may be 2.5 times the prong cross-sectional area when using a cannula sized for adult patients, whereas a gas passage may be 3 times the prong cross-sectional area when using a cannula sized for pediatric patients. Due to the pressure drop, the breathing gas is delivered from the at least one nasal prong at a prong output pressure equal to the output pressure of the breathing gas source (e.g., about 14kPa) minus a cumulative pressure drop which includes the pressure drop caused by the nasal cannula discussed above. The cumulative pressure drop may include the nasal cannula pressure drop and a gas passage pressure drop. In some implementations, the cumulative pressure drop also includes a pressure drop caused by frictional losses.

Humidification Measurement

Described herein is a method for calculating water content in a humidified gas stream, given a respiratory therapy system (or humidifier) in which the gas stream is fully saturated. The method relies on a relationship between gas flow rate and a measure of energy supplied into heating the water used for humidification. The method may be used on any of the systems described herein, for example those described in relation to FIGs. 1, 2, 4-9, and other humidifiers that rely on supplied electrical power for vaporization of water.

Water vapor content in the gas stream delivered to a patient is an important measure of performance of medical respiratory humidifiers. For example, compliance standards provide a minimum level of water vapor content in mg/L of gas flow to be classified as a medical humidifier. Existing systems use gravimetric methods or a chilled mirror hygrometer in order to directly measure humidity of delivered gas; however, these are manual methods that require an operator to interact with the systems, and collecting humidification data from large sets of samples (e.g., during research and development) can be time-consuming.

The method described herein provides for accurate humidification measurements that can be performed in a fully automated fashion, e.g., by a controller in the system. One particular use of the method involves collecting humidification data throughout the period of an extended life test of the humidifier without considerable use of manual labor and risk of data collection errors.

In the respiratory therapy system, breathing gas may be supplied over a range of flow rates (e.g., between 5 and 45 L/min). The humidifier (in the system or external to the system and receiving the breathing gas from the system) receives electric power from, for example, a power supply in the system. A heater in the humidifier, such as the induction heater described in the foregoing, uses the electric power to heat water in a reservoir of the humidifier. When the system is operating at a steady state (e.g., the amount of water and the water temperature in the humidifier is about constant), it can be approximated that all of the supplied power (or a known fraction of the supplied power, based on an efficiency of the heater) is directed towards vaporization of the water, where the energy of vaporization of water determines the energy required to generate water vapor into the gas stream.

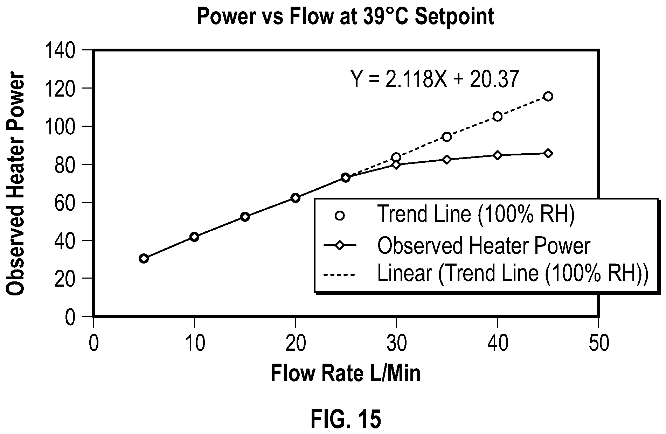

Plotting the energy supplied to the heater over the range of flow rates gives a curve having a linear region. FIG. 15 shows the plot for power vs. flow at a breathing gas temperature setpoint of 39 °C, using a respiratory therapy system similar to those described herein. The linear region indicates where the gas stream is fully saturated with water, for example, at 44 mg/L at 37 °C gas. The slope and y-intercept of the linear region provides a characteristic of the specific system in which the humidity measurement is being performed. A trendline can be calculated using the data in the linear region and extrapolated across the full range of flow rates. The trendline provides the expected power contribution if the gas stream were to remain fully saturated. The ratio of the observed power (indicated by diamond symbols in FIG. 15) to the expected power according to the trendline (indicated by the dotted line in FIG. 15) can be used to determine the water content in the gas stream by multiplying the full saturation state humidification level (*e.g.,* 44 mg water per L gas at 37 °C gas) by the ratio.

In the example shown in FIG. 15, the measurements were taken on a sample system similar to those described herein over the range of flowrates using breathing gas composed of room air having 21% $FiO_2$. The gas temperature setpoint was 37 °C, and ambient conditions were 22 °C and 29% RH. At 45 L/min breathing gas flow rate, the water content in the delivered gas stream was calculated to be 30.3 mg/L using the method described here.

A controller in the system or an external computer can programmatically set the gas flow rate and then receive and record the heater power data in order to perform this humidity level calculation. The gas temperature may also be programmatically set by the controller/computer, which may contain a memory storing tables or formulas for determining the full saturation level for a given gas temperature.

Humidifier reservoirs may be periodically refilled, and the power input to the heater is temporarily high while the temperature of newly introduced water is raised to meet the setpoint water temperature (assuming the incoming water comes from a source with a lower temperature, such as room temperature). The system may be programmed to only determine humidity using this method when the reservoir is at a steady state, which may be approximated as starting at a predetermined period of time after refilling has ceased. Alternatively, the amount of water used for refilling may be measured by a level sensor in the reservoir (such as the capacitive sensor described herein) in order to determine a period of time until the refilled reservoir has reached the steady state.

The humidification calculation may need to take into account ambient humidity in order to assess how much water is already in the breathing gas at the system air intake. For example, the systems described herein can deliver a mixture of room air and pure dry oxygen in order to deliver elevated oxygen levels to the patient. When the system is set to deliver 100% oxygen, then the humidification due to the system alone is measured.

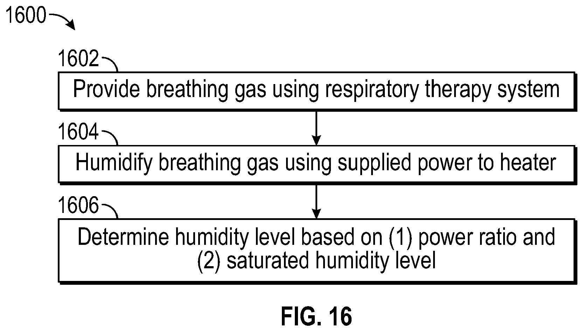

FIG. 16 shows a flowchart describing a humidification measurement method 1600, according to an example. Method 1600 includes steps 1602, 1604, and 1606. Step 1602 involves providing breathing gas to a patient at a gas flow rate and a gas temperature using a respiratory therapy system. The system comprises a controller, a humidifier having a reservoir containing water and a heater configured to heat the water in the reservoir, and a power supply coupled to the heater and configured to supply power to the heater. Step 1604 involves humidifying the breathing gas by supplying power to the heater and heating the water in the reservoir of the humidifier. Step 1606 involves determining a humidity level of the breathing gas based on (1) a ratio of the supplied power and an expected power and (2) a saturated humidity level for the gas temperature.

In some implementations, the expected power is determined based on a linear relationship between the gas flow rate and the supplied power over a range of flow rate values. For example, the range of flow rate values is between 5 L/min and 25 L/min. In particular, the range of values may be a subset in which the breathing gas is fully saturated due to the humidification.

The power supply may transmit data indicative of the supplied power to the controller in order for the data to be used in the humidification calculation. The controller may perform the determining step. The controller may include a memory that stores a table of saturated humidity level values for a range of gas temperature values or one or more formulas for determining saturated humidity level values given a specific gas temperature value. The determining step may include retrieving the saturated humidity level from the memory. The gas temperature may be stored in the memory as a setpoint temperature. The controller may automatically perform the determining step when the respiratory therapy system is in a steady state (*e.g.*, when the water in the reservoir is at a steady level or a steady temperature). The reservoir may be periodically refilled (manually or automatically via the controller), at which point the heat demand will be temporarily high for a period of time to bring the newly added water to the setpoint temperature, so the humidification measurement may be performed after the temporarily high heat demand period (*e.g.,* a predetermined period of time or a period calculated based on how much water is added during refilling).

Suitable humidifiers for this method include any humidifier that uses supplied electrical power to vaporize water. For example, wick humidifiers, vapor transfer cartridges, hotpot humidifiers, and other suitable humidifiers may be used. In some implementations the humidifier includes a heating plate disposed in the reservoir and an inductive element configured to receive the supplied power and generate a magnetic induction current in the heating plate to transfer heat to the water in the reservoir. The inductive element may be physically separate from the heating plate (*e.g.,* the inductive element is outside of the reservoir and not in contact with the water).

The foregoing is merely illustrative of the principles of the disclosure, and the apparatuses can be practiced by other than the described implementations, which are presented for purposes of illustration and not of limitation. It is to be understood that the apparatuses disclosed herein, while shown for use in high flow therapy systems, may be applied to systems to be used in other ventilation circuits.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

Examples of changes, substitutions, and alterations are ascertainable by one skilled in the art and could be made without departing from the scope of the information disclosed herein.

**Claims**

1.   A system (100) for providing breathing gas, the system comprising:

   a blower (102) configured to operate at a pressure less than 270 hPa and to:

      receive breathing gas from ambient air, a tank, or a wall outlet; and
      output pressurized breathing gas (104);

   a humidifier in fluid communication with the blower, wherein the humidifier is arranged to receive the pressurized breathing gas from the blower, and to heat and humidify the pressurized gas, the humidifier comprising:

      a reservoir configured to contain water;
      an induction heating plate disposed in the reservoir, configured to generate heat due to a resistance against a current in the plate induced by a magnetic coil; and

   a non-sealing nasal cannula (108) having at least one nasal prong (110), the nasal cannula being in fluid communication with a conduit (106) and configured to receive the humidified breathing gas from the humidifier through the conduit , the at least one nasal prong having a distal opening and being configured to provide the humidified breathing gas through the distal opening;
   wherein the nasal prong is configured to provide the humidified breathing gas at an exit velocity of at least 32.0 m/s and less than 90.0 m/s and at a flow rate of at least 4.0 L/min.

2.   The system according to claim 1, wherein:

   the at least one nasal prong (110) has an inner diameter greater than or equal to 1.1 mm and less than 2.2 mm, and the system has a maximum flow setpoint greater than or equal to 7.2 L/min and less than 33.2 L/min; or
   the at least one nasal prong has an inner diameter greater than or equal to 1.4 mm and less than 2.3 mm, and the system has a maximum flow setpoint greater than or equal to 10.4 L/min and less than 37.2 L/min; or
   the at least one nasal prong has an inner diameter greater than or equal to 1.5 mm and less than 3.6 mm, and the system (100) has a maximum flow setpoint greater than or equal to 16.8 L/min and less than 72.0 L/min; or
   the at least one nasal prong has an inner diameter greater than or equal to 2.4 mm and less than 4.8 mm, and the system has a maximum flow setpoint greater than or equal to 27.2 L/min and less than 96.0 L/min; or
   the at least one nasal prong has an inner diameter greater than or equal to 0.9 mm and less than 2.3 mm, and the nasal cannula (108) has a pressure drop less than 96 hPa when operating at a maximum flow setpoint of 8 L/min $\pm$ 20%; or
   the at least one nasal prong has an inner diameter greater than or equal to 1.2 mm and less than 2.4 mm, and the nasal cannula has a pressure drop less than 120 hPa when operating at a maximum flow setpoint of 20 L/min $\pm$ 20%; or
   the at least one nasal prong has an inner diameter greater than or equal to 1.5 mm and less than 4.2 mm, and the nasal cannula has a pressure drop less than 96 hPa when operating at a maximum flow setpoint of 40 L/min $\pm$ 20%.

3.   The system (100) according to claim 1 or 2, further comprising a controller (210) and a processor (206) configured to:

receive first data indicative of one or more dimensions of the nasal cannula (108);
receive second data indicative of a flowrate of breathing gas (104); and
calculate the exit velocity based on the first data and the second data.

4. The system (100) according to claim 3, wherein at least one of:

the system further comprises a display, and the processor (206) is further configured to generate for display at least one of: the flowrate, the exit velocity, a maximum flow setpoint, and a pressure drop;
the blower (102), controller (210), and processor (206) are housed in a base unit, the system further comprising an attachable unit configured to reversibly connect to the base unit;
the system further comprises at least one sensor configured to measure the flowrate of breathing gas and send the second data to the processor.

5. The system (100) according to claim 3 or 4, wherein:

the blower (102), controller (210), and processor (206) are housed in a base unit, the system further comprising an attachable unit configured to reversibly connect to the base unit;
the conduit (106) is configured to reversibly connect to the attachable unit;
the nasal cannula (108) is configured to reversibly connect to the conduit; and
the processor is configured to receive the first data when the attachable unit is connected to the base unit.

6. The system (100) according to claim 5, wherein the processor (206) is configured to receive the first data from an RFID tag within the attachable unit.

7. The system (100) according to claim 3, wherein:

the first data comprises an inner diameter of the at least one nasal prong (110); or
the processor (206) is configured to identify the nasal cannula based on the first data.

8. The system (100) according to any of claims 4-7, wherein:
the processor (206) is further configured to receive a user input to :

change the flowrate of breathing gas (104) to a modified flowrate of breathing gas; and/or
change the exit velocity to a modified velocity.

9. The system (100) according to claim 8, wherein:

the controller (210) is configured to change the flowrate of breathing gas to the modified flowrate based on the user input; and/or
the processor (206) is further configured to calculate a modified velocity based on the modified flowrate and the first data.

10. The system according to any of the preceding claims, further comprising:
a level sensor configured to measure a level of the water in the reservoir, the level sensor comprising a capacitive sensing circuit positioned alongside the reservoir and configured to measure a change in capacitance of the water as the level changes.

11. A method for providing breathing gas (104), the method comprising:

receiving, at a blower (102), breathing gas from ambient air, a tank, or a wall outlet, wherein the blower operates at a pressure of less than 270 hPa;
outputting, from the blower, a flow of pressurized breathing gas;
heating and humidifying the pressurized breathing gas in a humidifier that is in fluid communication with the blower, the humidifier comprising a reservoir configured to contain water with an induction heating plate disposed in the reservoir and configured to generate heat due to a resistance against a current in the plate induced by a magnetic coil;
outputting the heated and humidified breathing gas from the humidifier, through a conduit (106), into a non-sealing nasal cannula (108); and

providing the humidified breathing gas from a distal opening of at least one nasal prong (110) of the nasal cannula; wherein the at least one nasal prong is configured to provide the humidified breathing gas from the distal opening of the at least one nasal prong at an exit velocity of at least 32 m/s and less than 90 m/s and at a flowrate of at least 4 L/min.

12. The method according to claim 11, wherein:

the at least one nasal prong (110) has an inner diameter greater than or equal to 1.1 mm and less than 2.2 mm, and the blower has a maximum flow setpoint greater than or equal to 7.2 L/min and less than 33.6 L/min; or
the at least one nasal prong has an inner diameter greater than or equal to 1.4 mm and less than 2.3 mm, and the blower has a maximum flow setpoint greater than or equal to 10.4 L/min and less than 37.2 L/min; or
the at least one nasal prong has an inner diameter greater than or equal to 1.5 mm and less than 3.6 mm, and the blower (102) has a maximum flow setpoint greater than or equal to 16.8 L/min and less than 72.0 L/min; or
the at least one nasal prong has an inner diameter greater than or equal to 2.6 mm and less than 4.8 mm, and the blower has a maximum flow setpoint greater than or equal to 27.2 L/min and less than 96 L/min; or
the at least one nasal prong has an inner diameter greater than or equal to 0.9 mm and less than 1.9 mm, and the nasal cannula (108) has a pressure drop less than 64 hPa when the blower operates at a maximum flow setpoint of 8 L/min $\pm$ 20%; or
the at least one nasal prong has an inner diameter greater than or equal to 1.2 mm and less than 2.4 mm, and the nasal cannula has a pressure drop less than 120 hPa when the blower operates at a maximum flow setpoint of 20 L/min $\pm$ 20%; or
the at least one nasal prong has an inner diameter greater than or equal to 1.5 mm and less than 4.2 mm, and the nasal cannula has a pressure drop less than 96 hPa when the blower operates at a maximum flow setpoint of 40 L/min $\pm$ 20%.

13. The method according to claim 11 or 12, further comprising:

receiving first data indicative of one or more dimensions of the nasal cannula (108);
receiving second data indicative of a flowrate of the breathing gas (104); and
calculating the exit velocity based on the first data and the second data.

14. The method according to claim 13, further comprising at least one of:

generating for display at least one selected from the group of: the flowrate, the exit velocity, a maximum flow setpoint, and a pressure drop;
receiving a user input to increase or decrease the flowrate of the breathing gas, changing the flowrate to a modified flowrate of the breathing gas, calculating a modified velocity based on the modified flowrate and the first data, and generating for display at least one selected from the group of: the modified flowrate and the modified velocity;
using a capacitive sensing circuit positioned alongside the reservoir to sense a level of the water in the reservoir based on a change in capacitance of the water as the level changes.

15. The system of any of claims 1-9 or the method according to any of claims 11-14, wherein the exit velocity is:

at least 32 m/s and less than 84 m/s; or
at least 32 m/s and less than 78 m/s; or
at least 32 m/s and less than 72 m/s or
the exit velocity is at least 32 m/s and less than 66 m/s; or
the exit velocity is at least 32 m/s and less than 60 m/s; or
at least 32 m/s and less than 54 m/s; or
40 m/s $\pm$ 20%.

**Patentansprüche**

1. System (100) zum Bereitstellen von Atemgas, wobei das System Folgendes umfasst:
ein Gebläse (102), das dazu konfiguriert ist, bei einem Druck von weniger als 270 hPa zu arbeiten und:

Atemgas aus der Umgebungsluft, einem Tank oder einer Wandsteckdose zu beziehen; und
unter Druck stehendes Atemgas auszugeben (104);
einen Befeuchter in Fluidverbindung mit dem Gebläse, wobei der Befeuchter dazu eingerichtet ist, das unter Druck stehende Atemgas vom Gebläse aufzunehmen und das unter Druck stehende Gas zu erwärmen und zu befeuchten, wobei der Befeuchter Folgendes umfasst:

ein Reservoir, das dazu konfiguriert ist, Wasser aufzunehmen;
eine Induktionsheizplatte, die im Behälter angeordnet ist und dazu konfiguriert ist, Wärme aufgrund eines Widerstands gegen einen durch eine Magnetspule induzierten Strom in der Platte zu erzeugen; und
eine nicht abdichtende Nasenkanüle (108) mit mindestens einem Nasenstecker (110), wobei die Nasenkanüle in Fluidverbindung mit einer Leitung (106) steht und dazu konfiguriert ist, das befeuchtete Atemgas vom Befeuchter durch die Leitung aufzunehmen, wobei der mindestens eine Nasenstecker eine distale Öffnung aufweist und dazu konfiguriert ist, das befeuchtete Atemgas durch die distale Öffnung bereitzustellen;
wobei der Nasenstecker dazu konfiguriert ist, das befeuchtete Atemgas mit einer Austrittsgeschwindigkeit von mindestens 32,0 m/s und weniger als 90,0 m/s und einer Durchflussrate von mindestens 4,0 l/min bereitzustellen.

2. System nach Anspruch 1, wobei:

der mindestens eine Nasenstecker (110) einen Innendurchmesser von größer oder gleich 1,1 mm und kleiner als 2,2 mm aufweist und das System einen maximalen Durchflusssollwert von größer oder gleich 7,2 l/min und kleiner als 33,2 l/min hat; oder
der mindestens eine Nasenstecker einen Innendurchmesser von größer oder gleich 1,4 mm und kleiner als 2,3 mm aufweist und das System einen maximalen Durchflusssollwert von größer oder gleich 10,4 l/min und kleiner als 37,2 l/min hat; oder
der mindestens eine Nasenstecker einen Innendurchmesser von größer oder gleich 1,5 mm und kleiner als 3,6 mm aufweist und das System (100) einen maximalen Durchflusssollwert von größer oder gleich 16,8 l/min und kleiner als 72,0 l/min aufweist; oder
der mindestens eine Nasenstecker einen Innendurchmesser von größer oder gleich 2,4 mm und kleiner als 4,8 mm aufweist und das System einen maximalen Durchflusssollwert von größer oder gleich 27,2 l/min und kleiner als 96,0 l/min hat; oder
der mindestens eine Nasenstecker einen Innendurchmesser von größer oder gleich 0,9 mm und kleiner als 2,3 mm aufweist und die Nasenkanüle (108) einen Druckabfall von weniger als 96 hPa aufweist, wenn sie mit einem maximalen Durchflusssollwert von 8 l/min ± 20 % betrieben wird; oder
der Innendurchmesser des mindestens einen Nasensteckers ist größer oder gleich 1,2 mm und kleiner als 2,4 mm, und die Nasenkanüle weist einen Druckabfall von weniger als 120 hPa auf, wenn sie mit einem maximalen Durchflusssollwert von 20 l/min ± 20 % betrieben wird; oder
der mindestens eine Nasenstecker hat einen Innendurchmesser von größer oder gleich 1,5 mm und kleiner als 4,2 mm, und die Nasenkanüle weist einen Druckabfall von weniger als 96 hPa auf, wenn sie mit einem maximalen Durchflusssollwert von 40 l/min ± 20 % betrieben wird.

3. System (100) nach Anspruch 1 oder 2, das ferner eine Steuerung (210) und einen Prozessor (206) umfasst, die dazu konfiguriert sind:

erste Daten zu empfangen, die eine oder mehrere Abmessungen der Nasenkanüle (108) angeben;
zweite Daten zu empfangen, die eine Durchflussrate des Atemgases (104) anzeigen; und
die Austrittsgeschwindigkeit basierend auf den ersten und den zweiten Daten zu berechnen.

4. System (100) nach Anspruch 3, wobei mindestens eines der folgenden:

das System ferner eine Anzeige umfasst und der Prozessor (206) ferner so konfiguriert ist, dass er zur Anzeige mindestens eines der Durchflussrate, der Austrittsgeschwindigkeit, eines maximalen Durchflusssollwerts und eines Druckabfalls erzeugt;
das Gebläse (102), die Steuerung (210) und der Prozessor (206) in einer Basiseinheit untergebracht sind, wobei das System ferner eine anbringbare Einheit umfasst, die dazu konfiguriert ist, reversibel mit der Basiseinheit zu verbinden;
das System ferner mindestens einen Sensor umfasst, der dazu konfiguriert ist, die Durchflussrate des Atemgases zu messen und die zweiten Daten an den Prozessor zu senden.

5. System (100) nach Anspruch 3 oder 4, wobei:

das Gebläse (102), die Steuerung (210) und der Prozessor (206) sind in einer Basiseinheit untergebracht, wobei das System ferner eine anbringbare Einheit umfasst, die dazu konfiguriert ist, reversibel mit der Basiseinheit zu verbinden;
die Leitung (106) dazu konfiguriert ist, reversibel mit der anbringbaren Einheit zu verbinden;
die Nasenkanüle (108) dazu konfiguriert ist, reversibel mit der Leitung zu verbinden; und
der Prozessor dazu konfiguriert ist, die ersten Daten zu empfangen, wenn die anschließbare Einheit mit der Basiseinheit verbunden wird.

6. System (100) nach Anspruch 5, wobei der Prozessor (206) dazu konfiguriert ist, die ersten Daten von einem RFID-Tag innerhalb der anbringbaren Einheit zu empfangen.

7. System (100) nach Anspruch 3, wobei:

die ersten Daten einen Innendurchmesser des mindestens einen Nasensteckers (110) umfassen; oder
der Prozessor (206) dazu konfiguriert ist, die Nasenkanüle auf Grundlage der ersten Daten zu identifizieren.

8. System (100) nach einem der Ansprüche 4 bis 7, wobei:
der Prozessor (206) ferner dazu konfiguriert ist, eine Benutzereingabe zu empfangen, um:

die Flussrate des Atemgases (104) auf eine modifizierte Flussrate des Atemgases zu ändern; und/oder
die Austrittsgeschwindigkeit auf eine modifizierte Geschwindigkeit zu ändern.

9. System (100) nach Anspruch 8, wobei:

die Steuerung (210) dazu konfiguriert ist, die Durchflussrate des Atemgases basierend auf der Benutzereingabe auf die modifizierte Durchflussrate zu ändern; und/oder
der Prozessor (206) ferner dazu konfiguriert ist, eine modifizierte Geschwindigkeit basierend auf der modifizierten Durchflussrate und den ersten Daten zu berechnen.

10. System nach einem der vorhergehenden Ansprüche, ferner umfassend:
einen Füllstandssensor, der dazu konfiguriert ist, den Wasserstand im Reservoir zu messen, wobei der Füllstandssensor eine kapazitive Erfassungsschaltung umfasst, die neben dem Reservoir positioniert ist und dazu konfiguriert ist, eine Änderung der Kapazität des Wassers zu messen, wenn sich der Füllstand ändert.

11. Verfahren zum Bereitstellen von Atemgas (104), wobei das Verfahren Folgendes umfasst:

Empfangen von Atemgas aus der Umgebungsluft, einem Tank oder einer Wandsteckdose an einem Gebläse (102), wobei das Gebläse bei einem Druck von weniger als 270 hPa arbeitet;
Ausgeben eines unter Druck stehenden Atemgasstroms aus dem Gebläse;
Erhitzen und Befeuchten des unter Druck stehenden Atemgases in einem Befeuchter, der in Fluidverbindung mit dem Gebläse steht, wobei der Luftbefeuchter einen Behälter umfasst, der so konfiguriert ist, dass er Wasser enthält, wobei in dem Behälter eine Induktionsheizplatte angeordnet ist und dazu konfiguriert ist, um aufgrund eines Widerstands gegen einen durch eine Magnetspule induzierten Strom in der Platte Wärme zu erzeugen;
Ausgeben des erwärmten und befeuchteten Atemgases aus dem Befeuchter durch eine Leitung (106) in eine nicht abdichtende Nasenkanüle (108); und
Bereitstellen des befeuchteten Atemgases aus einer distalen Öffnung mindestens eines Nasensteckers (110) der Nasenkanüle;
wobei der mindestens eine Nasenstecker dazu konfiguriert ist, das befeuchtete Atemgas aus der distalen Öffnung des mindestens einen Nasensteckers mit einer Austrittsgeschwindigkeit von mindestens 32 m/s und weniger als 90 m/s und mit einer Strömungsrate von mindestens 4 l/min bereitzustellen.

12. Verfahren nach Anspruch 11, wobei:

der mindestens eine Nasenstecker (110) einen Innendurchmesser von größer oder gleich 1,1 mm und kleiner als 2,2 mm aufweist und das Gebläse einen maximalen Durchflusssollwert von größer oder gleich 7,2 l/min und kleiner als 33,6 l/min hat; oder

der mindestens eine Nasenstecker einen Innendurchmesser von größer oder gleich 1,4 mm und kleiner als 2,3 mm aufweist und das Gebläse einen maximalen Durchflusssollwert von größer oder gleich 10,4 l/min und kleiner als 37,2 l/min hat; oder

der mindestens eine Nasenstecker einen Innendurchmesser von größer oder gleich 1,5 mm und kleiner als 3,6 mm aufweist und das Gebläse (102) einen maximalen Durchflusssollwert von größer oder gleich 16,8 l/min und kleiner als 72,0 l/min aufweist; oder

der mindestens eine Nasenstecker einen Innendurchmesser von größer oder gleich 2,6 mm und kleiner als 4,8 mm aufweist und das Gebläse einen maximalen Durchflusssollwert von größer oder gleich 27,2 l/min und kleiner als 96 l/min hat; oder

der mindestens eine Nasenstecker einen Innendurchmesser von größer oder gleich 0,9 mm und kleiner als 1,9 mm aufweist und die Nasenkanüle (108) einen Druckabfall von weniger als 64 hPa aufweist, wenn das Gebläse mit einem maximalen Durchflusssollwert von 8 l/min ± 20 % arbeitet; oder

der mindestens eine Nasenstecker einen Innendurchmesser von größer oder gleich 1,2 mm und kleiner als 2,4 mm aufweist und die Nasenkanüle einen Druckabfall von weniger als 120 hPa aufweist, wenn das Gebläse mit einem maximalen Durchflusssollwert von 20 l/min ± 20 % arbeitet; oder

der mindestens eine Nasenstecker einen Innendurchmesser von größer oder gleich 1,5 mm und kleiner als 4,2 mm aufweist und die Nasenkanüle einen Druckabfall von weniger als 96 hPa aufweist, wenn das Gebläse mit einem maximalen Durchflusssollwert von 40 l/min ± 20 % arbeitet.

13. Verfahren nach Anspruch 11 oder 12, ferner umfassend:

Empfangen erster Daten, die eine oder mehrere Abmessungen der Nasenkanüle (108) angeben;
Empfangen von zweiten Daten, die eine Strömungsrate des Atemgases angeben (104); und
Berechnen der Austrittsgeschwindigkeit basierend auf den ersten und den zweiten Daten.

14. Verfahren nach Anspruch 13, ferner umfassend mindestens eines der folgenden:

Generieren von mindestens einem Wert aus der folgenden Gruppe zur Anzeige: Durchflussrate, Austrittsgeschwindigkeit, maximaler Durchflusssollwert und Druckabfall;
Empfangen einer Benutzereingabe zum Erhöhen oder Verringern der Flussrate des Atemgases, Ändern der Flussrate in eine modifizierte Flussrate des Atemgases, Berechnen einer modifizierten Geschwindigkeit basierend auf der modifizierten Flussrate und den ersten Daten und Generieren zur Anzeige mindestens eines ausgewählten Werts aus der Gruppe: der modifizierten Flussrate und der modifizierten Geschwindigkeit;
Verwenden einer kapazitiven Sensorschaltung, die neben dem Reservoir positioniert ist, um den Wasserstand im Reservoir anhand einer Kapazitätsänderung des Wassers bei einer Änderung des Wasserstands zu erfassen.

15. System nach einem der Ansprüche 1 bis 9 oder das Verfahren nach einem der Ansprüche 11 bis 14, wobei die Austrittsgeschwindigkeit Folgendes beträgt:

mindestens 32 m/s und weniger als 84 m/s; oder
mindestens 32 m/s und weniger als 78 m/s; oder
mindestens 32 m/s und weniger als 72 m/s oder
die Austrittsgeschwindigkeit mindestens 32 m/s und weniger als 66 m/s beträgt; oder
die Austrittsgeschwindigkeit mindestens 32 m/s und weniger als 60 m/s beträgt; oder
mindestens 32 m/s und weniger als 54 m/s; oder
40 m/s ± 20 %.

## Revendications

1. Système (100) de fourniture de gaz respiratoire, le système comprenant :

un ventilateur (102) configuré pour fonctionner à une pression inférieure à 270 hPa et pour : recevoir du gaz respiratoire à partir de l'air ambiant, d'un réservoir, ou d'une prise murale ; et produire du gaz respiratoire sous pression (104) ;
un humidificateur en communication fluidique avec le ventilateur, dans lequel l'humidificateur est agencé pour recevoir le gaz respiratoire sous pression à partir du ventilateur, et pour chauffer et humidifier le gaz sous pression, l'humidificateur comprenant :

un réservoir configuré pour contenir de l'eau ;
une plaque chauffante à induction disposée dans le réservoir, configurée pour générer de la chaleur en raison d'une résistance contre un courant dans la plaque induit par une bobine magnétique ; et
une canule nasale non étanche (108) présentant au moins une broche nasale (110), la canule nasale étant en communication fluidique avec un conduit (106) et configurée pour recevoir le gaz respiratoire humidifié à partir de l'humidificateur à travers le conduit, l'au moins une broche nasale présentant une ouverture distale et étant configurée pour fournir le gaz respiratoire humidifié à travers l'ouverture distale ;
dans lequel la broche nasale est configurée pour fournir le gaz respiratoire humidifié à une vitesse de sortie d'au moins 32,0 m/s et inférieure à 90,0 m/s et à un débit d'au moins 4,0 L/min.

2. Système selon la revendication 1, dans lequel :

l'au moins une broche nasale (110) présente un diamètre intérieur supérieur ou égal à 1,1 mm et inférieur à 2,2 mm, et le système présente une consigne de débit maximal supérieure ou égale à 7,2 L/min et inférieure à 33,2 L/min ; ou
l'au moins une broche nasale présente un diamètre intérieur supérieur ou égal à 1,4 mm et inférieur à 2,3 mm, et le système présente une consigne de débit maximal supérieure ou égale à 10,4 L/min et inférieur à 37,2 L/min ; ou
l'au moins une broche nasale présente un diamètre intérieur supérieur ou égal à 1,5 mm et inférieur à 3,6 mm, et le système (100) présente une consigne de débit maximal supérieure ou égale à 16,8 L/min et inférieure à 72,0 L/min ; ou
l'au moins une broche nasale présente un diamètre intérieur supérieur ou égal à 2,4 mm et inférieur à 4,8 mm, et le système présente une consigne de débit maximal supérieure ou égale à 27,2 L/min et inférieur à 96,0 L/min ; ou
l'au moins une broche nasale présente un diamètre intérieur supérieur ou égal à 0,9 mm et inférieur à 2,3 mm, et la canule nasale (108) présente une chute de pression inférieure à 96 hPa lorsqu'elle fonctionne à une consigne de débit maximal de 8 L/min ± 20 % ; ou
l'au moins une broche nasale présente un diamètre intérieur supérieur ou égal à 1,2 mm et inférieur à 2,4 mm, et la canule nasale présente une chute de pression inférieure à 120 hPa lorsqu'elle fonctionne à une consigne de débit maximal de 20 L/min ± 20 % ; ou
l'au moins une broche nasale présente un diamètre intérieur supérieur ou égal à 1,5 mm et inférieur à 4,2 mm, et la canule nasale présente une chute de pression inférieure à 96 hPa lorsqu'elle fonctionne à une consigne de débit maximal de 40 L/min ± 20 %.

3. Système (100) selon la revendication 1 ou 2, comprenant également un dispositif de commande (210) et un processeur (206) configurés pour :

recevoir des premières données indicatives d'une ou de plusieurs dimensions de la canule nasale (108) ;
recevoir des secondes données indicatives d'un débit de gaz respiratoire (104) ; et
calculer la vitesse de sortie sur la base des premières données et des secondes données.

4. Système (100) selon la revendication 3, dans lequel au moins l'un des éléments suivants :
le système comprend également un affichage, et le processeur (206) est également configuré pour générer pour l'affichage au moins l'un des éléments suivants :

le débit, la vitesse de sortie, une consigne de débit maximal, et une chute de pression ;
le ventilateur (102), le dispositif de commande (210), et le processeur (206) sont logés dans une unité de base, le système comprenant également une unité attachable configurée pour se connecter de manière réversible à l'unité de base ;
le système comprend également au moins un capteur configuré pour mesurer le débit de gaz respiratoire et envoyer les secondes données au processeur.

5. Système (100) selon la revendication 3 ou 4, dans lequel :

le ventilateur (102), le dispositif de commande (210), et le processeur (206) sont logés dans une unité de base, le système comprenant également une unité attachable configurée pour se connecter de manière réversible à l'unité de base ;

le conduit (106) est configuré pour se connecter de manière réversible à l'unité attachable ;

la canule nasale (108) est configurée pour se connecter de manière réversible au conduit ; et

le processeur est configuré pour recevoir les premières données lorsque l'unité attachable est connectée à l'unité de base.

6. Système (100) selon la revendication 5, dans lequel le processeur (206) est configuré pour recevoir les premières données à partir d'une étiquette RFID à l'intérieur de l'unité attachable.

7. Système (100) selon la revendication 3, dans lequel :

les premières données comprennent un diamètre intérieur de l'au moins une broche nasale (110) ; ou

le processeur (206) est configuré pour identifier la canule nasale sur la base des premières données.

8. Système (100) selon l'une quelconque des revendications 4 à 7, dans lequel :

le processeur (206) est également configuré pour recevoir une entrée utilisateur pour :

modifier le débit du gaz respiratoire (104) en un débit de gaz respiratoire modifié ; et/ou

changer la vitesse de sortie en une vitesse modifiée.

9. Système (100) selon la revendication 8, dans lequel :

le dispositif de commande (210) est configuré pour modifier le débit de gaz respiratoire au débit modifié sur la base de l'entrée l'utilisateur ; et/ou

le processeur (206) est également configuré pour calculer une vitesse modifiée sur la base du débit modifié et des premières données.

10. Système selon l'une quelconque des revendications précédentes, comprenant également :

un capteur de niveau configuré pour mesurer un niveau de l'eau dans le réservoir, le capteur de niveau comprenant un circuit de détection capacitif positionné le long du réservoir et configuré pour mesurer un changement de capacité de l'eau lorsque le niveau change.

11. Procédé de fourniture de gaz respiratoire (104), le procédé comprenant :

la réception, au niveau d'un ventilateur (102), de gaz respiratoire à partir de l'air ambiant, d'un réservoir, ou d'une prise murale, dans lequel le ventilateur fonctionne à une pression inférieure à 270 hPa ;

la production, à partir du ventilateur, d'un flux de gaz respiratoire sous pression ;

le chauffage et l'humidification du gaz respiratoire sous pression dans un humidificateur qui est en communication fluidique avec le ventilateur, l'humidificateur comprenant un réservoir configuré pour contenir de l'eau avec une plaque chauffante à induction disposée dans le réservoir et configurée pour générer de la chaleur en raison d'une résistance contre un courant dans la plaque induite par une bobine magnétique ;

la production du gaz respiratoire chauffé et humidifié à partir de l'humidificateur, à travers un conduit (106), dans une canule nasale non étanche (108) ; et

la fourniture du gaz respiratoire humidifié à partir d'une ouverture distale d'au moins une broche nasale (110) de la canule nasale ;

dans lequel l'au moins une broche nasale est configurée pour fournir le gaz respiratoire humidifié à partir de l'ouverture distale de l'au moins une broche nasale à une vitesse de sortie d'au moins 32 m/s et inférieure à 90 m/s et à un débit d'au moins 4 L/min.

12. Procédé selon la revendication 11, dans lequel :

l'au moins une broche nasale (110) présente un diamètre intérieur supérieur ou égal à 1,1 mm et inférieur à 2,2 mm, et le ventilateur présente une consigne de débit maximal supérieure ou égale à 7,2 L/min et inférieure à 33,6 L/min ; ou

l'au moins une broche nasale présente un diamètre intérieur supérieur ou égal à 1,4 mm et inférieur à 2,3 mm, et le ventilateur présente une consigne de débit maximal supérieure ou égale à 10,4 L/min et inférieure à 37,2 L/min ; ou

l'au moins une broche nasale présente un diamètre intérieur supérieur ou égal à 1,5 mm et inférieur à 3,6 mm, et le ventilateur (102) présente une consigne de débit maximal supérieure ou égale à 16,8 L/min et inférieure à 72,0 L/min ; ou

l'au moins une broche nasale présente un diamètre intérieur supérieur ou égal à 2,6 mm et inférieur à 4,8 mm, et le ventilateur présente une consigne de débit maximal supérieure ou égale à 27,2 L/min et inférieure à 96 L/min ; ou

l'au moins une broche nasale présente un diamètre intérieur supérieur ou égal à 0,9 mm et inférieur à 1,9 mm, et la canule nasale (108) présente une chute de pression inférieure à 64 hPa lorsque le ventilateur fonctionne à une consigne de débit maximal de 8 L/min ± 20 % ; ou

l'au moins une broche nasale présente un diamètre intérieur supérieur ou égal à 1,2 mm et inférieur à 2,4 mm, et la canule nasale présente une chute de pression inférieure à 120 hPa lorsque le ventilateur fonctionne à une consigne de débit maximal de 20 L/min ± 20 % ; ou

l'au moins une broche nasale présente un diamètre intérieur supérieur ou égal à 1,5 mm et inférieur à 4,2 mm, et la canule nasale présente une chute de pression inférieure à 96 hPa lorsque le ventilateur fonctionne à une consigne de débit maximal de 40 L/min ± 20 %.

13. Procédé selon la revendication 11 ou 12, comprenant également :

la réception de premières données indicatives d'une ou de plusieurs dimensions de la canule nasale (108) ;
la réception de secondes données indicatives d'un débit du gaz respiratoire (104) ; et
le calcul de la vitesse de sortie sur la base des premières données et des secondes données.

14. Procédé selon la revendication 13, comprenant également au moins l'un des éléments suivants :

la génération pour l'affichage d'au moins un élément sélectionné dans le groupe comprenant : le débit, la vitesse de sortie, une consigne de débit maximal, et une chute de pression ;
la réception d'une entrée utilisateur pour augmenter ou diminuer le débit du gaz respiratoire, changer le débit en un débit modifié du gaz respiratoire, calculer une vitesse modifiée sur la base du débit modifié et des premières données, et générer pour l'affichage au moins un élément sélectionné dans le groupe comprenant : le débit modifié et la vitesse modifiée ;
l'utilisation d'un circuit de détection capacitif positionné le long du réservoir pour détecter un niveau de l'eau dans le réservoir sur la base d'un changement de capacité de l'eau lorsque le niveau change.

15. Système selon l'une quelconque des revendications 1 à 9 ou procédé selon l'une quelconque des revendications 11 à 14, dans lequel la vitesse de sortie est :

d'au moins 32 m/s et inférieure à 84 m/s ; ou
d'au moins 32 m/s et inférieure à 78 m/s ; ou
d'au moins 32 m/s et inférieure à 72 m/s ou
la vitesse de sortie est d'au moins 32 m/s et inférieure à 66 m/s ; ou
la vitesse de sortie est d'au moins 32 m/s et inférieure à 60 m/s ; ou
d'au moins 32 m/s et inférieure à 54 m/s ; ou
40 m/s ± 20 %.

FIG. 1

**FIG. 2**

EP 4 117 756 B1

300

302
Receive first data indicative of nasal cannula dimensions

304
Receive second data indicative of breathing gas flowrate

306
Calculate exit velocity based on first and second data

308
Display the calculated exit velocity

**FIG. 3**

FIG. 4

**FIG. 5**

600

658
657
656
660

614

604

615

612

655

652

613

654

602

**FIG. 6**

**FIG. 7A**

**FIG. 7B**

**FIG. 7C**

**FIG. 7D**

**FIG. 7E**

**FIG. 7F**

FIG. 8A

FIG. 8B

**FIG. 9**

FIG. 10

**FIG. 11**

EP 4 117 756 B1

**FIG. 12**

EP 4 117 756 B1

0.0486
0.0432
0.0378
0.0324
0.0270
0.0216
0.0161
0.0107
0.0053
-8.0364e-05

**Mass Fraction of Carbon Dioxide []**

**Cut Plot 3: Contours**

**FIG. 13A**

0.0486
0.0432
0.0378
0.0324
0.0270
0.0216
0.0161
0.0107
0.0053
-8.0364e-05

**Mass Fraction of Carbon Dioxide []**

Cut Plot 2: Contours
**Cut Plot 1: Contours**

**FIG. 13B**

**Cannula Pressure Drop as a Function of Outlet Velocity**

$y = 0.0007x^2 - 0.0001x$
$R^2 = 0.999$

**FIG. 14A**

**Cannula Pressure Drop as a Percentage of Total Pressure Available From a 14kPa Blower as a Function of Outlet Velocity**

$y = 5E\text{-}05x^2 - 1E\text{-}05x$
$R^2 = 0.999$

**FIG. 14B**

Tubing Pressure Drop as a Function of the Ratio of Tubing Cross-section to Prong Size, for Prong Sizes of 0.07 In, 0.1 in, And 0.13 in

FIG. 14C

Power vs Flow at 39°C Setpoint

$Y = 2.118X + 20.37$

Legend:
- o Trend Line (100% RH)
- ◇ Observed Heater Power
- ----- Linear (Trend Line (100% RH))

Y-axis: Observed Heater Power
X-axis: Flow Rate L/Min

FIG. 15

1600

1602

Provide breathing gas using respiratory therapy system

1604

Humidify breathing gas using supplied power to heater

1606

Determine humidity level based on (1) power ratio and (2) saturated humidity level

FIG. 16

**REFERENCES CITED IN THE DESCRIPTION**

**Patent documents cited in the description**

- WO 2018071812 A1
- WO 2015033288 A1
- US 10300236 B
- US 20180104436
- US 10596345 B

- US 008508
- US 10514662 B
- US 602392
- US 10007238 B